# EUROPEAN PATENT APPLICATION

(11) **EP 4 653 421 A1**
(43) Date of publication of application: **26.11.2025**
(21) Application number: 24763252.4
(22) Date of filing: 01.03.2024
(51) Int. Cl.: C07C 323/62, C07D 333/20, A61K 31/00, A61P 35/00

(54) **AROMATIC-RING COMPOUND AND USE THEREOF**

(30) Priority: 01.03.2023 CN 202310183442; 30.08.2023 CN 202311106221
(71) Applicant: Hitgen Inc., Chengdu, Sichuan 610200 (CN)
(72) Inventor: LI, Jin, Chengdu, Sichuan 610200 (CN); WANG, Qingyang, Chengdu, Sichuan 610200 (CN); WANG, Xiaotao, Chengdu, Sichuan 610200 (CN); LI, Xiaomei, Chengdu, Sichuan 610200 (CN); DOU, Dengfeng, Chengdu, Sichuan 610200 (CN); MENG, Xiaoyun, Chengdu, Sichuan 610200 (CN); LI, Xianyang, Chengdu, Sichuan 610200 (CN); CHEN, Qiuxia, Chengdu, Sichuan 610200 (CN); GUO, Anping, Chengdu, Sichuan 610200 (CN); ZHENG, Nuo, Chengdu, Sichuan 610200 (CN); HU, Hong, Chengdu, Sichuan 610200 (CN); HE, Wei, Chengdu, Sichuan 610200 (CN)
(74) Representative: Vitina, Maruta
(86) International application number: PCT/CN2024/079575
(87) International publication number: WO 2024/179572

(57) **Abstract**

A compound of the following formula 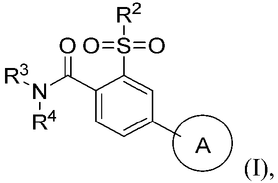 capable of binding to a tripartite motif (TRIM) protein E3 ubiquitin ligase (TRIM21). This application also provides a method for treating a disease associated with abnormal cell proliferation with the compound of formula (I). This application further provides a method for preparing a drug for targeted protein degradation with such compound as an intermediate.

## Description

### TECHNICAL FIELD

This application relates to pharmaceuticals, and more particularly to a ligand compound capable of binding to a tripartite motif-containing protein 21 (TRIM21) (a member of the E3 ubiquitin ligase family) and a proteolysis targeting chimera (PROTAC) bi-function compound containing the same.

### BACKGROUND

Protein degradation is a highly-regulatable process essential for maintaining the cellular homeostasis. The selective recognition and removal of damaged, misfolded, or over-expressed proteins are achieved through a ubiquitin-proteasome pathway (UPP). The UPP is responsible for clearing defective proteins, and is characterized by its ATP-dependency, high efficiency and high selectivity. The catalytic component involved in the UPP is an E3 ubiquitin ligase, and it is required to recruit the target protein before the catalytic degradation. PROTAC (proteolysis targeting chimera) technology is designed based on the UPP, in which the target protein-binding ligand is linked to the E3 ligase ligand with appropriate chemical bonds, enabling the recognition of the target protein and enhancing the binding affinity between the E3 ligase and the target protein. This results in targeted ubiquitination and degradation of the target protein, with excellent catalytic activity, high efficiency and high selectivity.

Multiple ubiquitin molecules can label the target proteins through the covalent linkage between the terminal lysine residues and the E3 ubiquitin ligase for proteasomal degradation. In this process, the proteins are digested into small peptides and eventually into the amino acids, which are used for the synthesis of new proteins. The degradation of defective proteasomes is associated with a variety of clinical diseases, including Alzheimer's disease, Parkinson's disease, Huntington's disease, muscular dystrophy, cardiovascular diseases, and cancer.

The TRIM E3 ubiquitin ligase family, to which the TRIM21 belongs, includes multiple members for the ubiquitin-dependent proteolysis involved in the cellular NF-κB signaling pathway. It has been demonstrated that the TRIM21 is associated with autoimmune diseases, and the TRIM21 has also been found to play a role in the development and prognosis of various tumors. As evidenced by the protein expression database-based analysis, the TRIM21 is highly expressed in various tumor cells compared to normal tissues. Therefore, the development of TRIM21-based ligand molecules and corresponding PROTACs is considered as promising tools for targeted degradation of pathogenic proteins in tumors with high TRIM21 expression.

The present application discloses a novel class of compounds that can serve as effective TRIM21 ligands and can be applied to the synthesis of corresponding PROTAC bi-function compounds suitable for the treatment of various diseases, especially abnormal cell proliferation.

### SUMMARY

This application provides a compound of formula (I), or a stereoisomer, a deuterated compound or a pharmaceutically acceptable salt thereof: 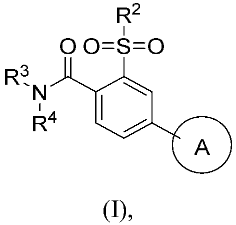 wherein:
A ring is selected from the group consisting of 6 to 10-membered aromatic ring, 5 to 10-membered heteroaromatic ring, 3 to 10-membered cycloalkyl and 3 to 10-membered heterocyclic ring, wherein aromatic ring, heteroaromatic ring, cycloalkyl and heterocyclic ring are independently unsubstituted or substituted with one, two, three or four R¹;
each R¹ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, hydroxy-substituted - C₁₋₆ alkyl, -C₀₋₄ alkylene-OR¹², -C₀₋₄ alkylene-OC(O)R¹², -C₀₋₄ alkylene-SR¹², -C₀₋₄ alkylene-S(O)₂R¹², -C₀₋₄ alkylene-S(O)R¹², -C₀₋₄ alkylene-S(O)₂NR¹²R¹³, -C₀₋₄ alkyleneS(O)NR¹²R¹³, -C₀₋₄ alkylene-C(O)R¹², -C₀₋₄ alkylene-C(O)OR¹², -C₀₋₄ alkylene-C(O)NR¹²R¹³, -C₀₋₄ alkylene-NR¹²R¹³, -C₀₋₄ alkylene-NR¹²C(O)R¹³, -C₀₋₄ alkylene-NR¹²S(O)₂R¹³, -C₀₋₄ alkylene-NR¹²S(O)R¹³, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring);
R¹² and R¹³ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R¹⁴;
each R¹⁴ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl;
each R² is independently selected from the group consisting of hydrogen, halogen, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR²², -C₀₋₄ alkylene-OC(O)R²², -C₀₋₄ alkylene-SR²², -C₀₋₄ alkylene-S(O)₂R²², -C₀₋₄ alkylene-S(O)R²², -C₀₋₄ alkyleneS(O)₂NR²²R²³, -C₀₋₄ alkylene-S(O)NR²²R²³, -C₀₋₄ alkylene-C(O)R²², -C₀₋₄ alkylene-C(O)OR²², -C₀₋₄ alkylene-C(O)NR²²R²³, -C₀₋₄ alkylene-NR²²R²³, -C₀₋₄ alkylene-NR²²C(O)R²³, -C₀₋₄ alkylene-NR²²S(O)₂R²³, -C₀₋₄ alkylene-NR²²S(O)R²³, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring);
R²² and R²³ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl,-C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R²⁴;
each R²⁴ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl;
R³ and R⁴ are each independently selected from the group consisting of hydrogen, halogen, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR³⁴, -C₀₋₄ alkylene-OC(O)R³⁴, -C₀₋₄ alkylene-SR³⁴, -C₀₋₄ alkylene-S(O)₂R³⁴, -C₀₋₄ alkylene-S(O)R³⁴, - C₀₋₄ alkylene-S(O)₂NR³⁴R³⁵, -C₀₋₄ alkylene-S(O)NR³⁴R³⁵, -C₀₋₄ alkylene-C(O)R³⁴, -C₀₋₄ alkylene-C(O)OR³⁴, -C₀₋₄ alkylene-C(O)NR³⁴R³⁵, -C₀₋₄ alkylene-NR³⁴R³⁵, -C₀₋₄ alkylene-NR³⁴C(O)R³⁵, -C₀₋₄ alkylene-NR³⁴S(O)₂R³⁵, -C₀₋₄ alkylene-NR³⁴S(O)R³⁵, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R³⁶;
each R³⁶ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR³⁴, -C₀₋₄ alkylene-OC(O)R³⁴, -C₀₋₄ alkylene-SR³⁴, -C₀₋₄ alkylene-S(O)₂R³⁴, -C₀₋₄ alkyleneS(O)R³⁴, -C₀₋₄ alkylene-S(O)₂NR³⁴R³⁵, -C₀₋₄ alkylene-S(O)NR³⁴R³⁵, -C₀₋₄ alkylene-C(O)R³⁴ -C₀₋₄ alkylene-C(O)OR³⁴, -C₀₋₄ alkylene-C(O)NR³⁴R³⁵, -C₀₋₄ alkylene-NR³⁴R³⁵, -C₀₋₄ alkylene-NR³⁴C(O)R³⁵, -C₀₋₄ alkylene-NR³⁴S(O)₂R³⁵, -C₀₋₄ alkylene-NR³⁴S(O)R³⁵, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R³⁷;
R³⁴ and R³⁵ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl and halogen-substituted -C₂₋₆ alkynyl; and
each R³⁷ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl.

In an embodiment, the A ring is selected from the group consisting of 6-membered aromatic ring, 6-membered cycloalkyl, 9-membered heterocycloalkyl, 6-membered heteroaromatic ring and 9-membered heteroaromatic ring, wherein aromatic ring, cycloalkyl, heterocycloalkyl and heteroaromatic ring are independently unsubstituted or substituted with one, two, three or four R¹.

In an embodiment, the A ring is benzene ring, wherein the benzene ring is independently unsubstituted or substituted with one, two, three or four R¹; preferably, the benzene ring is substituted with one R¹.

In an embodiment, the compound of formula (I) is represented by formula (IIA): 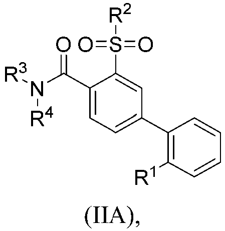 wherein R¹, R², R³ and R⁴ are defined as above.

In an embodiment, each R¹ is independently selected from the group consisting of hydrogen, halogen, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, hydroxy-substituted -C₁₋₆ alkyl, -C₀₋₄ alkylene-OR¹², -C₀₋₄ alkylene-OC(O)R¹², -C₀₋₄ alkylene-SR¹², -C₀₋₄ alkylene- NR¹²R¹³, -C₀₋₄ alkylene-(3 to 6-membered cycloalkyl), -C₀₋₄ alkylene-(3 to 6-membered heterocycloalkyl), -C₀₋₄ alkylene-(6 to 10-membered aromatic ring) and -C₀₋₄ alkylene-(5 to 10-membered heteroaromatic ring); and
R¹² and R¹³ are each independently selected from the group consisting of hydrogen, - C₁₋₆ alkyl, -C₂₋₆ alkenyl, -C₂₋₆ alkynyl and halogen-substituted -C₁₋₆ alkyl.

In an embodiment, each R¹ is independently selected from the group consisting of hydrogen, methylthio, halogen, methyl, ethyl, propyl, isopropyl, methoxy, trifluoromethyl, 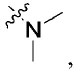 cyclopropyl and

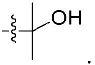

In the following embodiments of the present disclosure, each R¹ is independently selected from the group consisting of hydrogen, fluoro, chloro, cyano group, methyl, ethyl, propyl, isopropyl, n-butyl, isobutyl, tert-butyl, ethenyl, ethynyl, fluoromethyl, difluoromethyl, trifluoromethyl, hydroxymethyl, methoxy, ethoxy, n-propoxy, isopropoxy, n-butoxy, isobutoxy, tert-butoxy, ethyleneoxy, ethynyloxy, methylthio, ethylthio, n-propylthio, isopropylthio, n-butylthio, isobutylthio, tert-butylthio, methylamino, ethylamino, dimethylamino, diethylamino, cyclopropyl, cyclobutyl, cyclopentyl or

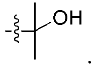

In an embodiment, the A ring is selected from the group consisting of

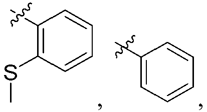

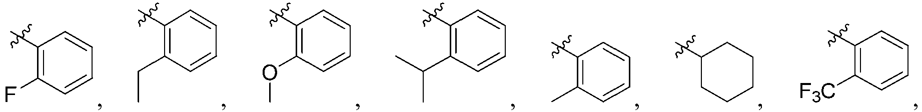

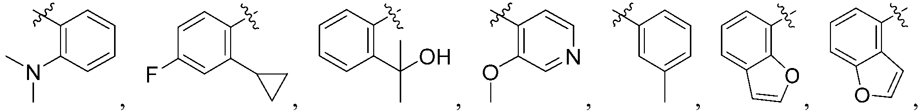

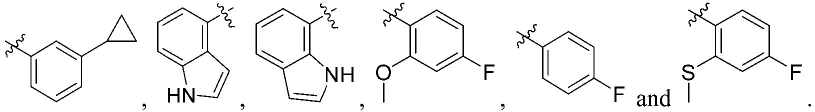

In an embodiment, R² is -C₁₋₃ alkyl; preferably, R² is methyl.

In an embodiment, R³ and R⁴ are each independently selected from the group consisting of hydrogen, -C₁₋₆ alkyl, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R³⁶;
each R³⁶ is independently selected from the group consisting of hydrogen, halogen, cyano group, =O, -C₁₋₆ alkyl, halogen-substituted -C₁₋₆ alkyl, -C₀₋₄ alkylene-OR³⁴, -C₀₋₄ alkylene-C(O)R³⁴, -C₀₋₄ alkylene-C(O)OR³⁴, -C₀₋₄ alkylene-C(O)NR³⁴R³⁵, -C₀₋₄ alkylene-NR³⁴R³⁵, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring); and
R³⁴ and R³⁵ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl and halogen-substituted -C₂₋₆ alkynyl.

In an embodiment, R³ and R⁴ are each independently selected from the group consisting of hydrogen, methyl, ethyl, propyl, butyl, amyl, hexyl, -C₀₋₂ alkylene-(3-membered cycloalkyl), -C₀₋₂ alkylene-(4-membered cycloalkyl), -C₀₋₂ alkylene-(5-membered cycloalkyl), -C₀₋₂ alkylene-(6-membered cycloalkyl), -C₀₋₂ alkylene-(4-membered heterocycloalkyl), -C₀₋₂ alkylene-(5-membered heterocycloalkyl), -C₀₋₂ alkylene-(6-membered heterocycloalkyl), - C₀₋₂ alkylene-(9-membered heterocycloalkyl), -C₀₋₂ alkylene-(6-membered aromatic ring), - C₀₋₂ alkylene-(5-membered heteroaromatic ring), -C₀₋₂ alkylene-(6-membered heteroaromatic ring) and -C₀₋₂ alkylene-(9-membered heteroaromatic ring), wherein alkylene, cycloalkyl, heterocycloalkyl, aromatic ring and heteroaromatic ring are independently unsubstituted or substituted with one, two, three or four R³⁶; wherein R³ is preferably selected from the group consisting of hydrogen, methyl, ethyl, propyl, butyl or amyl;
each R³⁶ is independently selected from the group consisting of hydrogen, halogen, =O, -C₁₋₃ alkyl, halogen-substituted -C₁₋₃ alkyl, -OR³⁴, -C(O)R³⁴ and -NR³⁴R³⁵; and
R³⁴ and R³⁵ are each independently selected from the group consisting of hydrogen and -C₁₋₃ alkyl.

In an embodiment, R³ and R⁴ are each independently selected from the group consisting of hydrogen, methyl, ethyl, propyl, butyl, amyl, hexyl, 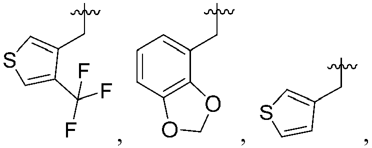 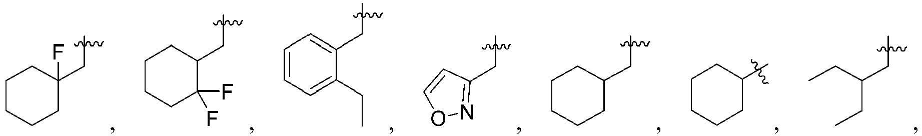 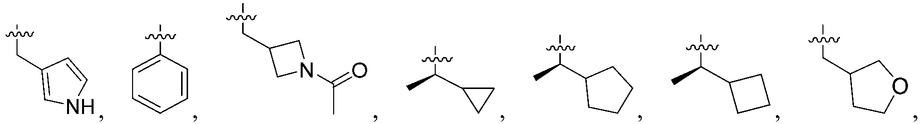 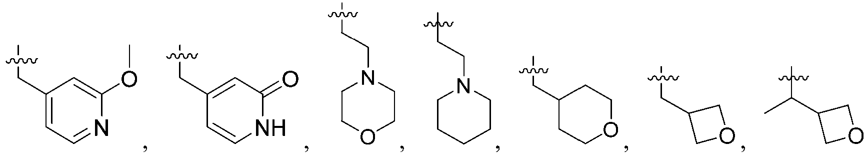 and

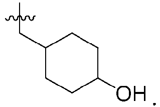

In an embodiment, R³ is selected from the group consisting of hydrogen, methyl, ethyl, propyl, butyl and amyl; preferably, R³ is selected from the group consisting of hydrogen and methyl; preferably, R³ is methyl.

In an embodiment, R⁴ is selected from the group consisting of hydrogen, methyl, ethyl, propyl, butyl, amyl, hexyl,

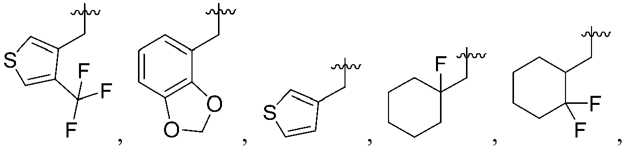

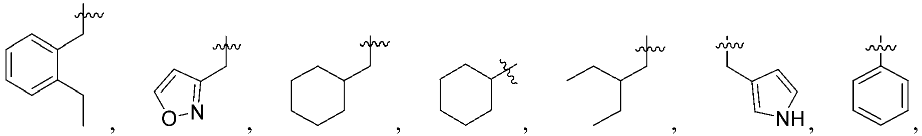

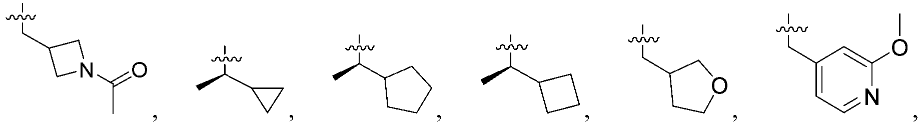

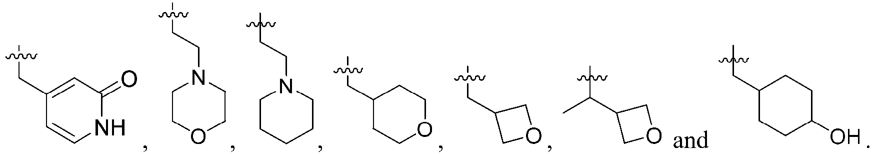

In an embodiment, the compound of formula (I) is selected from the group consisting of: 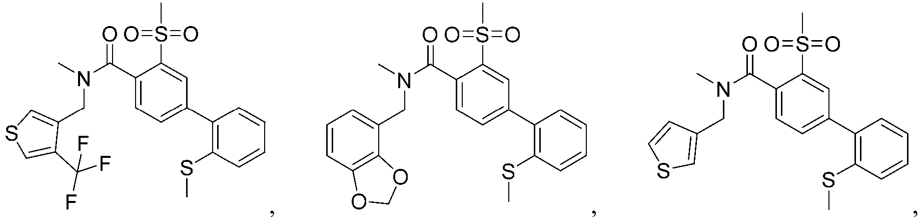 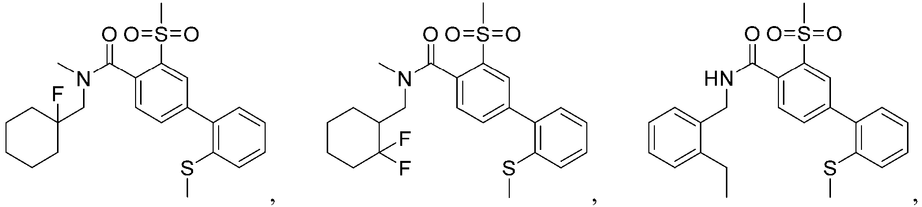 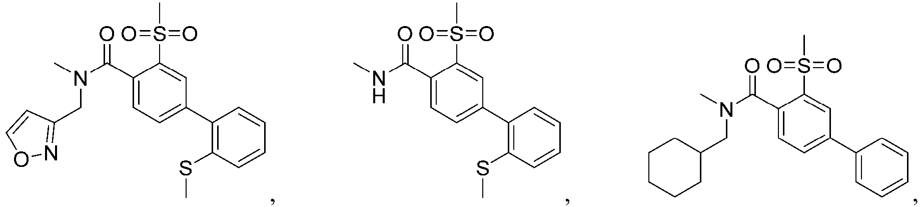 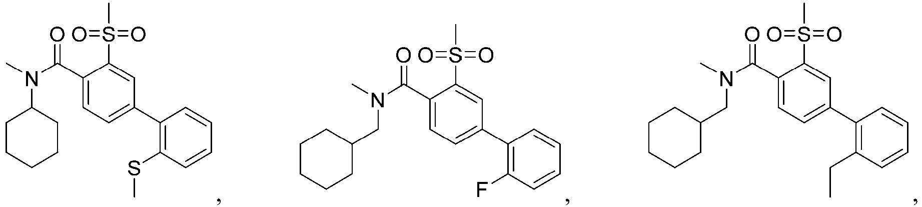 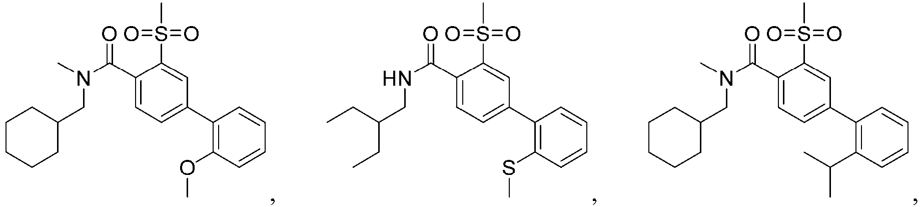 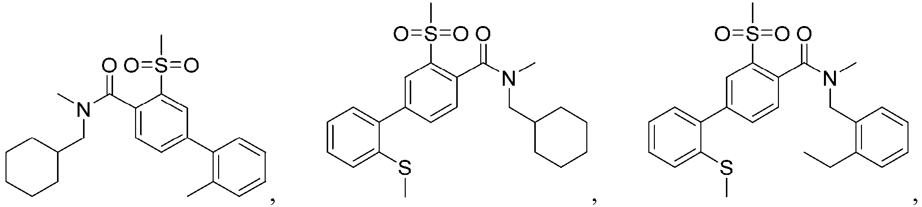 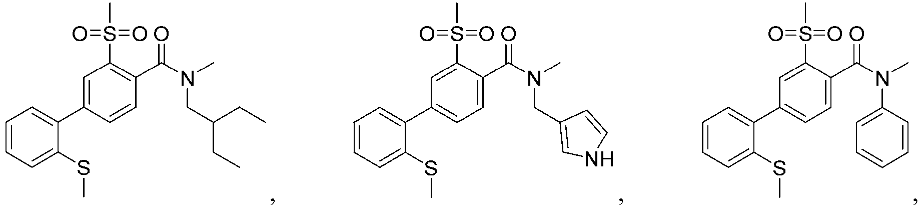 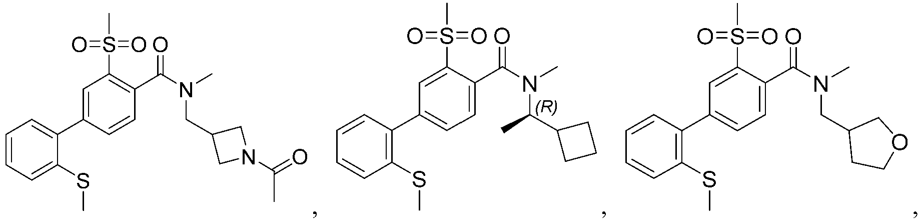 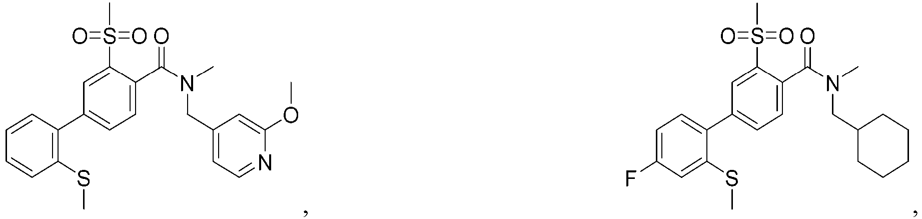 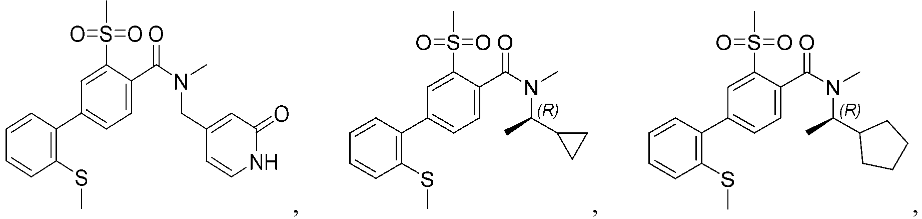 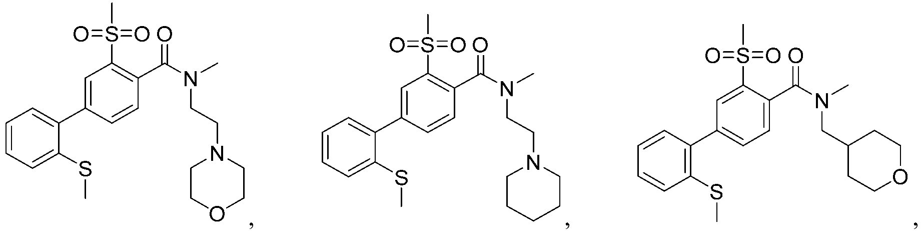 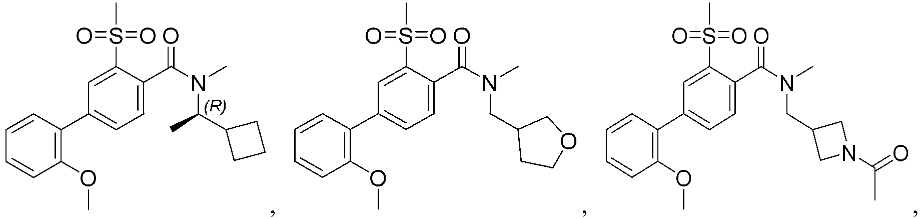 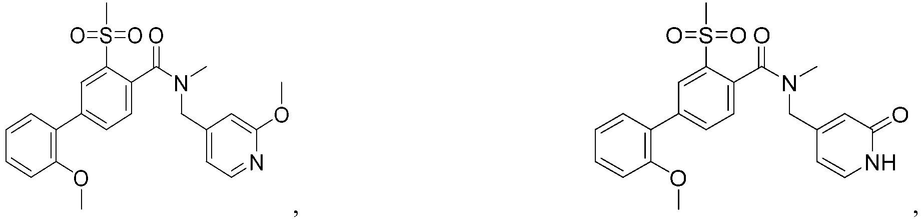 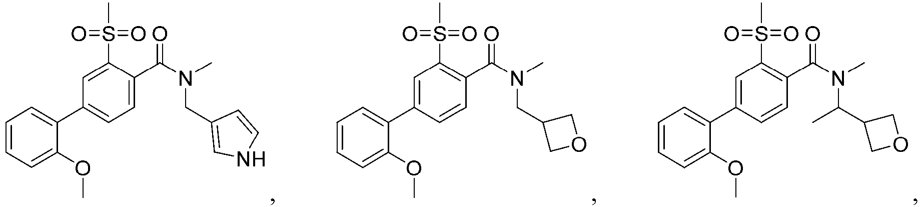 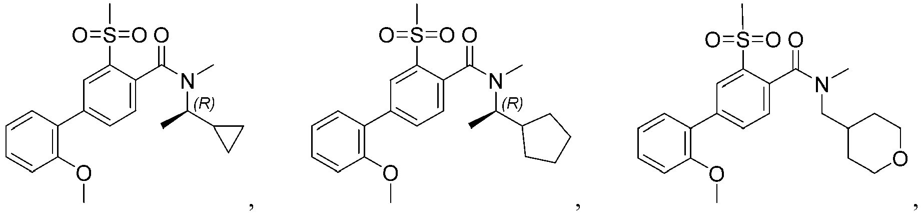 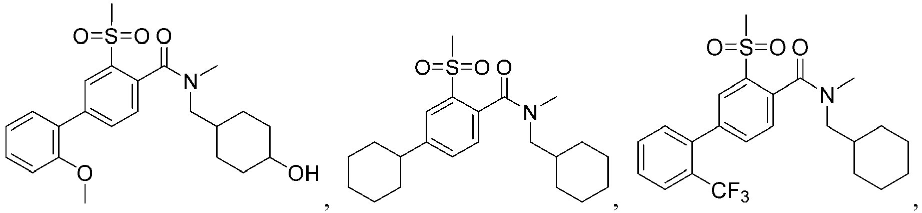 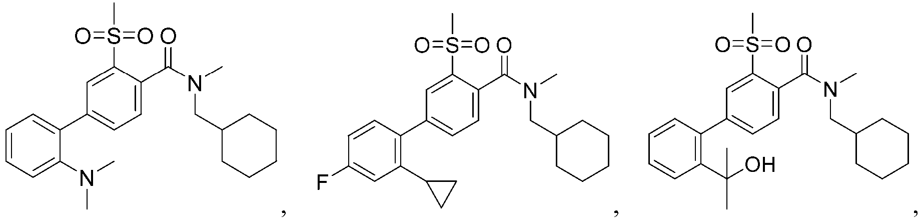 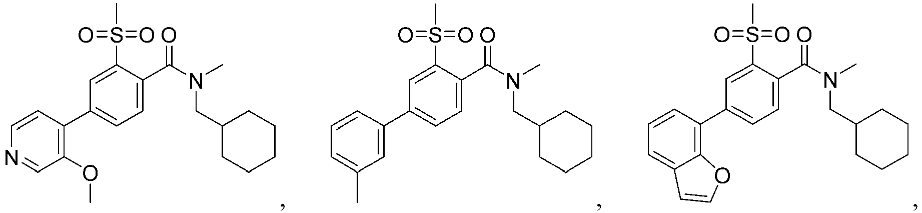 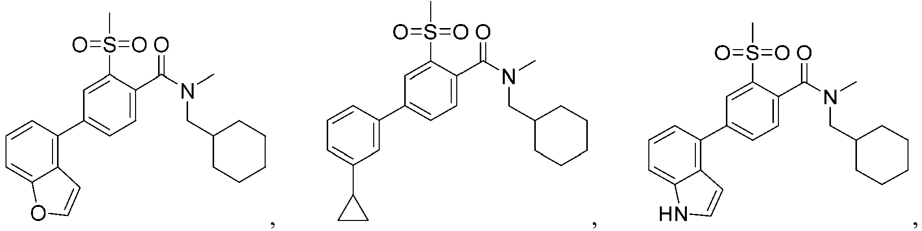 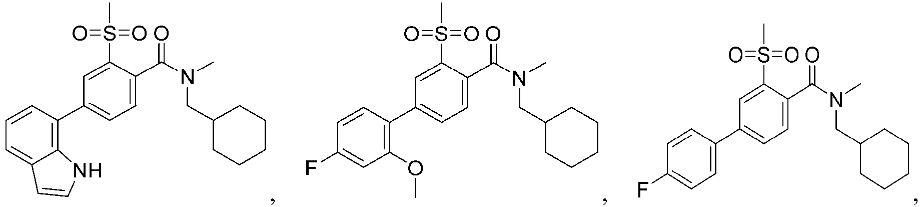 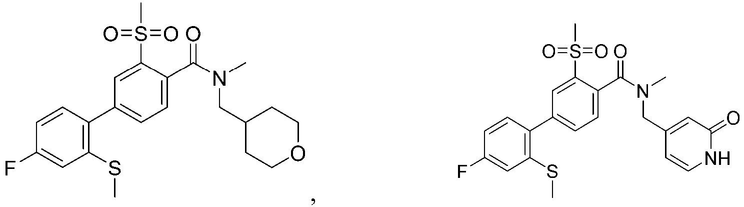 and

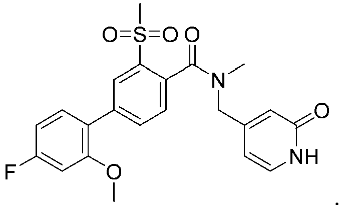

This application also provides a compound of formula (V), or a stereoisomer, a deuterated compound or a pharmaceutically acceptable salt thereof: 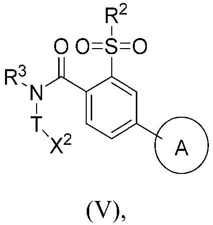 wherein:
A ring is selected from the group consisting of 6 to 10-membered aromatic ring, 5 to 10-membered heteroaromatic ring, 3 to 10-membered cycloalkyl and 3 to 10-membered heterocyclic ring, wherein aromatic ring, aromatic ring, heteroaromatic ring, cycloalkyl and heterocyclic ring are independently unsubstituted or substituted with one, two, three or four R¹;
each R¹ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR¹², - C₀₋₄ alkylene-OC(O)R¹², -C₀₋₄ alkylene-SR¹², -C₀₋₄ alkylene-S(O)₂R¹², -C₀₋₄ alkyleneS(O)R¹², -C₀₋₄ alkylene-S(O)₂NR¹²R¹³, -C₀₋₄ alkylene-S(O)NR¹²R¹³, -C₀₋₄ alkylene-C(O)R¹², -C₀₋₄ alkylene-C(O)OR¹², -C₀₋₄ alkylene-C(O)NR¹²R¹³, -C₀₋₄ alkylene-NR¹²R¹³, -C₀₋₄ alkylene-NR¹²C(O)R¹³, -C₀₋₄ alkylene-NR¹²S(O)₂R¹³, -C₀₋₄ alkylene-NR¹²S(O)R¹³, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring);
R¹² and R¹³ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R¹⁴;
each R¹⁴ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl;
each R² is independently selected from the group consisting of hydrogen, halogen, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR²², -C₀₋₄ alkylene-OC(O)R²², -C₀₋₄ alkylene-SR²², -C₀₋₄ alkylene-S(O)₂R²², -C₀₋₄ alkylene-S(O)R²², -C₀₋₄ alkyleneS(O)₂NR²²R²³, -C₀₋₄ alkylene-S(O)NR²²R²³, -C₀₋₄ alkylene-C(O)R²², -C₀₋₄ alkylene-C(O)OR²², -C₀₋₄ alkylene-C(O)NR²²R²³, -C₀₋₄ alkylene-NR²²R²³, -C₀₋₄ alkylene-NR²²C(O)R²³, -C₀₋₄ alkylene-NR²²S(O)₂R²³ -C₀₋₄ alkylene-NR²²S(O)R²³, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring);
R²² and R²³ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl,-C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R²⁴;
each R²⁴ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl;
R³ is independently selected from the group consisting of hydrogen, halogen, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR³⁴, -C₀₋₄ alkylene-OC(O)R³⁴,
-C₀₋₄ alkylene-SR³⁴, -C₀₋₄ alkylene-S(O)₂R³⁴, -C₀₋₄ alkylene-S(O)R³⁴, -C₀₋₄ alkyleneS(O)₂NR³⁴R³⁵, -C₀₋₄ alkylene-S(O)NR³⁴R³⁵, -C₀₋₄ alkylene-C(O)R³⁴, -C₀₋₄ alkylene-C(O)OR³⁴, -C₀₋₄ alkylene-C(O)NR³⁴R³⁵, -C₀₋₄ alkylene-NR³⁴R³⁵, -C₀₋₄ alkylene-NR³⁴C(O)R³⁵, -C₀₋₄ alkylene-NR³⁴S(O)₂R³⁵, -C₀₋₄ alkylene-NR³⁴S(O)R³⁵, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R³⁶;
each R³⁶ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR³⁴, -C₀₋₄ alkylene-OC(O)R³⁴, -C₀₋₄ alkylene-SR³⁴, -C₀₋₄ alkylene-S(O)₂R³⁴, -C₀₋₄ alkyleneS(O)R³⁴, -C₀₋₄ alkylene-S(O)₂NR³⁴R³⁵, -C₀₋₄ alkylene-S(O)NR³⁴R³⁵, -C₀₋₄ alkylene-C(O)R³⁴, -C₀₋₄ alkylene-C(O)OR³⁴, -C₀₋₄ alkylene-C(O)NR³⁴R³⁵, -C₀₋₄ alkylene-NR³⁴R³⁵, -C₀₋₄ alkylene-NR³⁴C(O)R³⁵, -C₀₋₄ alkylene-NR³⁴S(O)₂R³⁵, -C₀₋₄ alkylene-NR³⁴S(O)R³⁵, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R³⁷;
R³⁴ and R³⁵ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl and halogen-substituted -C₂₋₆ alkynyl;
each R³⁷ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl;
T is -(L^{T})_{q}-;
q is an integer selected from 1-50;
each L^{T} is independently selected from the group consisting of CR^{T2}R^{T3}, C(O), -C(S)-, O, S, S(O), S(O)₂, NR^{T2}, -CR^{T2}=CR^{T3}-, -C≡C-, 3 to 12-membered cycloalkyl, 3 to 12-membered heterocycloalkyl, 6 to 10-membered aromatic ring, 5 to 10-membered heteroaromatic ring, 5 to 12-membered spirocycle, 5 to 12-membered spiroheterocycle, 5 to 12-membered bridged ring, 5 to 12-membered bridged heterocycle and a combination thereof, wherein cycloalkyl, heterocycloalkyl, aromatic ring, heteroaromatic ring, spirocycle, spiroheterocycle, bridged ring and bridged heterocycle, are independently unsubstituted or substituted with one, two, three or four R^{T1};
each R^{T1} is independently selected from the group consisting of hydrogen, halogen, cyano group, nitro, =O, =S, =CR^{T2}R^{T3}, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR^{T2}, -C₀₋₄alkylene-OC(O)R^{T2}, -C₀₋₄alkylene-SR^{T2}, -C₀₋₄alkylene-S(O)₂R^{T2}, -C₀₋₄ alkylene-S(O)R^{T2}, -C₀₋₄ alkylene-S(O)₂NR^{T2}R^{T3}, -C₀₋₄ alkylene-S(O)NR^{T2}R^{T3}, -C₀₋₄ alkylene-C(O)R^{T2}, -C₀₋₄ alkylene-C(O)OR^{T2}, -C₀₋₄ alkylene-C(O)NR^{T2}R^{T3}, -C₀₋₄ alkylene-NR^{T2}R^{T3}, -C₀₋₄ alkylene-NR^{T2}C(O)R^{T3}, -C₀₋₄ alkylene-NR^{T2}S(O)₂R^{T3}, -C₀₋₄ alkylene-NR^{T2}S(O)R^{T3}, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R^{T4};
R^{T2}, R^{T3} and R^{T4} are each independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring);
X² is selected from the group consisting of NH₂, -NHR^{X21}, -OH, -SH, ethynyl, ethenyl, -C(O)H and -C(O)OH-; and
R^{X21} is selected from the group consisting of hydrogen, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl.

In an embodiment, the compound of formula (V) is represented by formula (VIa): 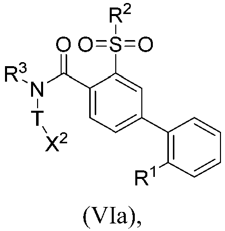 wherein R¹, R², R³, T and X² are defined as above.

In an embodiment, T is selected from the group consisting of

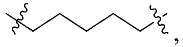

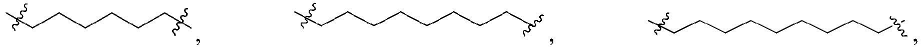

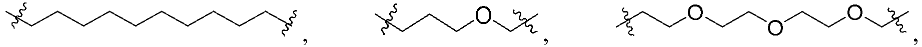

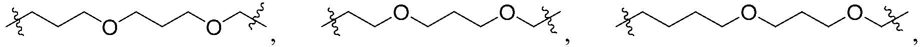

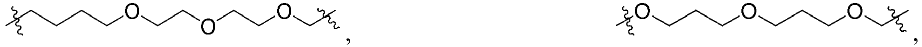

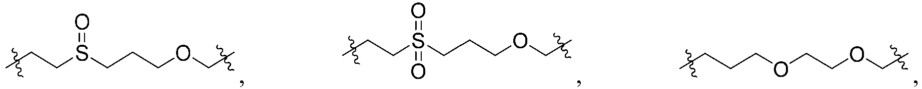

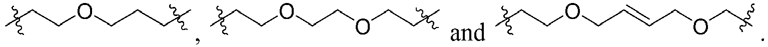

This application also provides a method for treating a disease associated with abnormal cell proliferation in a subject in need thereof, comprising:
administering to the subject a therapeutically effective amount of the above compound, or a stereoisomer, a deuterated compound or a pharmaceutically acceptable salt thereof.

In an embodiment, the disease is a cancer.

This application also provides a method for preparing a drug for targeted protein degradation, comprising:
preparing the drug from the above compound, or a stereoisomer, a deuterated compound or a pharmaceutically acceptable salt thereof.

In an embodiment, the compound, or a stereoisomer, a deuterated compound or a pharmaceutically acceptable salt thereof is used as an intermediate.

In an embodiment, the drug is a drug dependent on a tripartite motif protein 21 E3 ubiquitin ligase (TRIM21) for protein degradation.

The compounds provided herein and derivatives thereof can be named according to the nomenclature system of the International Union of Pure and Applied Chemistry (IUPAC) or Chemical Abstracts Service (CAS), Columbus, Ohio.

Unless otherwise specified, the initial definition of group or term provided herein is applicable throughout the specification. And those terms that are not specifically defined herein should be construed according to the disclosure and context.

"Substitution" means that the hydrogen atom in the molecule is replaced by other different atoms or groups; or the lone electron pair of an atom in the molecule is replaced by S atom or O atom.

The limitation "capable of being substituted" indicates that a "substitution" may occur, but is not required. The description includes instances where it does or does not occur.

A minimum and a maximum of a content of carbon atoms in a hydrocarbon group are indicated by the prefix. For example, a prefix C_{a-b} alkyl indicates any alkyl group containing a-b carbon atoms, i.e., C₁₋₄ alkyl refers to an alkyl group containing 1-4 carbon atoms.

The "alkyl" refers to a saturated hydrocarbon chain having the specified number of member atoms, i.e., C₁₋₆ alkyl refers to an alkyl group containing 1-6 carbon atoms. The alkyl group can be straight-chain or branched. Representative branched alkyl groups have one, two or three branched chains. The alkyl group may optionally be substituted with one or more substituents as defined herein. The alkyl includes methyl, ethyl, propyl (n-propyl and isopropyl), butyl (n-butyl, isobutyl and tert-butyl), pentyl (n-pentyl, isopentyl and neopentyl) and hexyl. The alkyl group may also be part of other groups, such as C₁₋₆ alkoxy. The "alkyl" refers to a saturated hydrocarbon chain having the specified number of member atoms, i.e., C₁₋₆ alkyl refers to an alkyl group containing 1-6 carbon atoms. The alkyl group can be straight-chain or branched. Representative branched alkyl groups have one, two or three branched chains. The alkyl group may optionally be substituted with one or more substituents as defined herein. The alkyl includes methyl, ethyl, propyl (n-propyl and isopropyl), butyl (n-butyl, isobutyl and tert-butyl), pentyl (n-pentyl, isopentyl and neopentyl) and hexyl. The alkyl group may also be part of other groups, such as C₁₋₆ alkoxy.

The term "alkylene" in the present disclosure means a divalent saturated aliphatic hydrocarbon group having a specified number of carbon atoms. The "C_{a-b} alkylene" refers to alkylene groups with a-b carbon atoms. The alkylene groups include both branched and straight-chain hydrocarbon groups. For example, C₁₋₆ alkylene is intended to include methylene, ethylidene, propylidene, 2-methylpropylidene, dimethylethylidene and pentylidene. For example, propylidene is 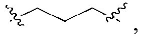 and dimethylbutylidene is 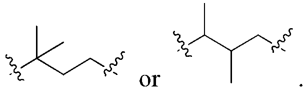 For example, the term "C₀₋₄ alkylene" is alkylene with C₀, alkylene with C₁ (such as -CH₂-), alkylene with C₂ (such as -CH₂CH₂-), alkylene with C₃ (such as -CH₂CH₂CH₂-), or alkylene with C₄ (such as -CH₂CH₂CH₂CH₂-). C₀ alkylene refers to the absence of the group here, and a connection here is chemical bonding. For example, A-C₀ alkylene-B refers to A-B, that is, A is directly connected to B through a chemical bond. The term "alkenyl" refers to a straight or branched hydrocarbon group having a specified number of carbon atoms, 2-6 carbon atoms or 2-4 carbon atoms, and having at least one vinyl unsaturated site (>C=C<). For example, C_{a-b} alkenyl is an alkenyl group with a-b carbon atoms, such as vinyl, propenyl, isopropenyl and 1,3-butadienyl.

As used herein, the term "alkenylene" refers to a hydrocarbon chain having 2-10 carbon atoms, at least one double bond and two unsaturated valences. For example, (C₃-C₆) alkenylene includes ">C=CH-CH₂-" and "-CH₂-CH=CH-CH₂-".

The term "alkynyl" is a straight-chain monovalent hydrocarbon radical or a branched monovalent hydrocarbon radical containing at least one triple bond. The term "alkynyl" includes those alkyl groups having a triple bond and a double bond. For example, C₂₋₆ alkynyl includes ethynyl and propynyl.

The term "halogen" is fluorine, chlorine, bromine or iodine.

The terms "haloalkyl" and "halogen-substituted alkyl" refer to an yl in which the hydrogen atom may be replaced with one or more halogen atoms. For example, halogen-substituted C₁₋₄ alkyl refers to an alkyl group containing 1-4 carbon atoms with the hydrogen atoms substituted by one or more halogen atoms.

The terms "-OR" and "-NRR" in the present disclosure indicate that the R group is connected to the O atom or N atom by a single bond.

The terms "-C(O)R" and "-S(O)₂R" in the present disclosure indicate that the O atom is connected to the C atom or S atom by a double bond, and the R is connected to the C or S atom by a single bond.

The term "cycloalkyl" and "cycloalkane" in the present disclosure refer to a saturated or partially saturated cyclic group having a plurality of carbon atoms without heterocyclic atom, and having a single ring or a plurality of rings (including fused, bridged, spiro, and adamantane systems). For polycyclic systems with aromatic and non-aromatic rings that do not contain heterocyclic atoms, the term "cycloalkyl" is applicable when the attachment point is located at a non-aromatic carbon atom (for example, 5,6,7,8-tetrahydronaphthalen-5-yl). The terms "cycloalkyl" includes cycloalkenyl groups, such as cyclohexenyl. The cycloalkyl includes adamantyl, cyclopropyl, cyclobutyl, cyclohexyl, cyclopentyl, cyclooctyl, cyclopentenyl and cyclohexenyl. The cycloalkyl including a polybicycloalkyl ring systems includes dicyclohexyl, dicyclopentyl and dicyclooctyl, such as 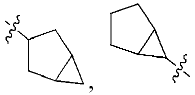 and 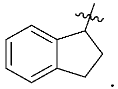 The adamantyl includes, but is not limited to, the following structure:

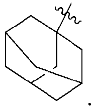

The terms "heterocycle", "heterocycloalkyl" and "heterocycloalkane" in the present disclosure refer to a saturated ring or a non-aromatic unsaturated ring containing at least one heteroatom, where the heteroatom refers to atoms such as nitrogen, oxygen and sulfur. For example, monovalent saturated or partially unsaturated monocyclic or bicyclic ring systems of a plurality of ring atoms, preferably a monovalent saturated or partially unsaturated single-ring or bicyclic ring system containing 3 to 9 ring atoms, which includes 1, 2, or 3 heteroatoms selected from the group consisting of N, O, and S, and the remaining ring atoms are carbon. Bicycles represent a chain consisting of two rings atoms in common, that is, a bridge separating the two rings is either a single bond or one or two ring atoms. The monocyclic saturated heterocyclicalkyl includes oxetanyl, azetidinyl, pyrrolidinyl, 2-oxopyrrolidin-3-yl, tetrahydrofuranyl, tetrahydrothienyl, pyrazolidinyl, imidazolidinyl, thiazolidinyl, piperidinyl, tetrahydropyranyl, tetrahydrothiopyranyl, piperazinyl, morpholinyl, 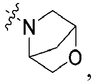 thiomorpholinyl, 1,1-dioxo-thiomorpholin-4-yl, azepanyl, diazepanyl, homopiperazinyl and oxazepanyl. The bicyclic saturated heterocycloalkyl includes 8-azabicyclo[3.2.1]octan, quinuclidinyl, 8-oxa-3-azabicyclo[3.2.1]octan, 9-azabicyclo[3.3.1]nonane and 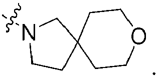 The partially unsaturated heterocycloalkyl includes dihydrofuranyl, imidazolinyl, tetrahydro-pyridyl and dihydropyranyl.

The terms "spiro-heterocyclic group" and "spiro-heterocycle" can be used interchangeably and refer to a non-aromatic saturated ring or a non-aromatic unsaturated ring system in which two monocycles share a carbon atom, consisting of carbon atoms and heteroatoms selected from the group consisting of N, O, S and P. For example, "5 to 12-membered spiro-heterocycle" refers to a spiro-heterocycle having 5 to 12 ring atoms, where 1, 2 or 3 of the ring atoms are heteroatoms.

The terms "bridged-ring" or "bridged-ring group" refer to a saturated or unsaturated cyclic group formed by two or more cyclic structures sharing two non-adjacent atoms with each other, including but not limited to

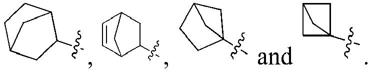

The terms "bridged-heterocyclic group" and "bridged-heterocycle" can be used interchangeably and refer to a saturated or unsaturated cyclic group formed by two or more cyclic structures sharing two non-adjacent atoms with each other, composed of carbon atoms and heteroatoms selected from the group consisting of N, O, S, and P. Specific examples include, but are not limited to

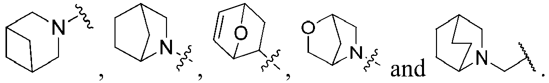

The terms "aromatic ring" and "aryl group" mentioned herein refer to an aromatic group with a plurality of carbon atoms. The aryl group is usually a monocyclic, bicyclic or tricyclic aryl having 5-20 carbon atoms. Furthermore, the term "aryl group" provided herein refers to an aromatic substituent that can be a single aromatic ring or multiple aromatic rings fused together. Non-limiting examples include phenyl, naphthyl and tetrahydronaphthyl.

The terms "heteroaromatic ring" and "heteroaryl group" mentioned herein refer to an aromatic unsaturated ring containing at least one heteroatom. The heteroatom includes nitrogen atoms, oxygen atoms and sulfur atoms. For example, aromatic monocyclic or bicyclic hydrocarbons with a plurality of ring atoms, where one or more of the ring atoms are selected from the group consisting of O, N and S. Preferably, there are 1-3 heteroatoms. The heteroaryl group includes pyridyl, indolyl, quinoxalinyl, quinolinyl, isoquinolinyl, benzothienyl, benzofuranyl, benzopyranyl, benzothiopyranyl, furanyl, pyrrolyl, thiazolyl, oxazolyl, isoxazolyl, triazolyl, tetrazolyl, pyrazolyl, imidazolyl, thienyl, oxadiazolyl, benzimidazolyl, benzothiazolyl and benzoxazolyl.

The term "stereoisomer" mentioned herein includes enantiomer and diastereomers.

The term "deuterated compound" mentioned herein refers to a substitution of one or more hydrogen atoms in a molecule or group by deuterium atoms, where the percentage of deuterium atoms is greater than an abundance of deuterium in nature.

The term "pharmaceutically acceptable" mentioned herein refers to a carrier, delivery agent, diluent, excipient, and/or salt that are generally chemically or physically compatible with other ingredients used in a pharmaceutical dosage form, and are physiologically compatible with the receptor.

The terms "salt" and "pharmaceutically acceptable salt" mentioned herein refer to salts formed by the aforementioned compounds or their stereoisomers with inorganic and/or organic acids and bases, including both acidic and/or base salts, as well as zwitterionic salts (inner salts) and quaternary ammonium salts, such as alkylammonium salts. These salts can be directly obtained during the final separation and purification of the compound. Alternatively, they can also be obtained by mixing the aforementioned compounds or their stereoisomers with an appropriate amount of acid or base (e.g., equivalent). The salts may be insoluble, and can be collected by filtration, evaporation or freeze drying. The salts provided herein include but not limited to hydrochloride, sulfate, citrate, benzene sulfonate, hydrobromide, hydrofluoride, phosphate, acetate, propionate, succinate, oxalate, malate, succinate, fumarate, maleate, tartarate and trifluoroacetate.

Apparently, according to the foregoing disclosure, various modifications, replacements or variations can be made by those skilled in the art without departing from the spirit of the present disclosure.

### DETAILED DESCRIPTION OF EMBODIMENTS

The disclosure will be further described in detail below with reference to the following examples. However, it should be understood that the scope of the present disclosure is not limited to the following exemplary embodiments. All embodiments implemented in light of the content disclosed herein shall fall within the scope of the present disclosure.

The known starting materials of the present disclosure can be synthesized according to the methods known in the prior art, or can be purchased from Energy Chemical Co., Ltd, Chengdu Kelong Chemical Co., Ltd, Accela ChemBio Co., Ltd, or J&K Scientific Co., Ltd. Unless otherwise specified, reaction was carried out under a nitrogen atmosphere, a solution is an aqueous solution, a temperature of the reaction is room temperature, which is the most suitable temperature for the reaction (20°C to 30°C), and M represents mol/L.

Compounds are structurally characterized by Nuclear Magnetic Resonance (NMR) and Mass Spectrometry (MS). The NMR shifts (δ) is expressed in a unit of 10⁻⁶ (ppm). The NMR spectra are obtained by using a Nuclear Magnetic Resonance Spectrometer (Bruker Avance III 400) and (Bruker Avance 600 spectrometer) with deuterated dimethyl sulfoxide (DMSO-*d₆*)*,* deuterated chloroform (CDCl₃), or deuterated methanol (Methol-*d₄*) as the solvent, and tetramethylsilane (TMS) as the internal standard. LC-MS is carried out using Shimadzu LC-MS 2020 (ESI). HPLC is performed using a Shimadzu High-Performance Liquid Chromatograph (Shimadzu LC-20A). MPLC (Medium-Pressure Preparative Liquid Chromatography) is performed on a Gilson GX-281 reversed phase preparative chromatograph. Yantai Huanghai HSGF254 or Qingdao GF254 silica gel plates are used for thin layer chromatography, and the product size used in the thin layer chromatography is 0.4 mm to 0.5 mm. Typically, 200-300 mesh silica gel (Yantai Huanghai silica gel) is used as the carrier in column chromatography.

Pd(dppf)cl₂: 1,1'-bis(diphenylphosphino)ferrocene palladium(ii) dichloride; DIPEA: N,N-diisopropylethylamine; HATU: 2-(7-azabenzotriazol-1-yl)-N,N,N',N'-tetramethyluronium hexafluorophosphate; DCE: dichloroethane; and DMF N,N-dimethylformamide.

### Example 1 Preparation of compound 1

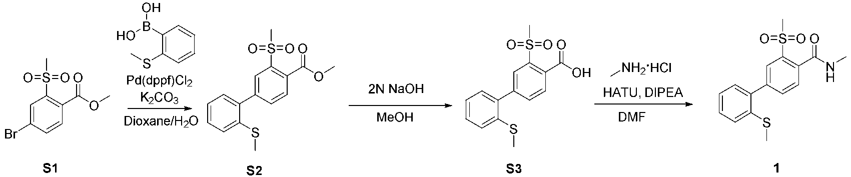

### Synthesis of compound S2

Under the protection of N₂, substrate **S1** (200 mg, 0.682 mmol), 2-(methylthio)phenylboronic acid (137.57 mg, 0.818 mmol), Pd(dppf)Cl₂ (49.51 mg, 0.068 mmol), K₂CO₃ (282.89 mg, 2.05 mmol) and 2 mL of 1,4-dioxane/H₂O (v:v=3: 1) were added into a microwave tube, and reacted under stirring at 100°C for 2 h, where the reaction was monitored through liquid chromatography-mass spectrometry (LC-MS). The reaction mixture was subjected to rotary evaporation, and extraction with ethyl acetate three times. The resultant organic phases were combined, washed with a saturated NaCl aqueous solution, dried over anhydrous sodium sulfate, and purified through medium-pressure liquid chromatography to obtain a brown solid as substrate **S2** (198 mg, 0.588 mmol, 86% yield). **LCMS** (ESI⁺) m/z: 337.0 [M+H]⁺.

### Synthesis of compound S3

The substrate **S2** (200 mg, 0.594 mmol) was added into a single-neck flask, to which 2 mL of MeOH was added. The reaction mixture was stirred for dissolution, added with a 2N NaOH solution (71.34 mg, 1.78 mmol), and reacted under stirring at 60 °C for 2 h, where the reaction was monitored by LC-MS. The reaction mixture was subjected to rotary evaporation, pH adjustment to 5-6 with a 3N HCl solution, freeze-drying, and purification through medium-pressure liquid chromatography to obtain a light reddish-brown oily liquid as substrate **S3** (180 mg, 0.558 mmol, 93.92% yield). **LCMS** (ESI⁺) m/z: 321.1 [M-H]⁻.

### Synthesis of compound 1

The substrate **S3** (30 mg, 0.093 mmol) and 3 mL DMF were added into a dried single-neck flask, followed by stirring for dissolution. The reaction mixture was added with DIPEA (120.26 mg, 0.93 mmol), cooled in an ice water bath, added with HATU (42.43 mg, 0.111 mmol), stirred at 0 °C for 5 min, added with methylamine (3.47 mg, 0.111 mmol), and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **1** (16 mg, 0.047 mmol, 50.90% yield, 99.3% purity). **LCMS** (ESI⁺) m/z: 336.1 [M+H]⁺.

**¹H NMR** (400 MHz, DMSO-d6) *δ* 8.63 (d, J = 4.7 Hz, 1H), 7.94 (d, J = 1.8 Hz, 1H), 7.77 (dd, J = 7.7, 1.8 Hz, 1H), 7.60 (d, J = 7.8 Hz, 1H), 7.50 - 7.38 (m, 2H), 7.28 (d, J = 2.3 Hz, 1H), 6.04 (s, 1H), 3.41 (s, 3H), 2.77 (d, J = 4.6 Hz, 3H), 2.42 (s, 3H).

### Example 2 Preparation of compound 2

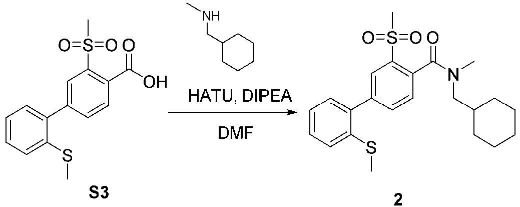

The substrate **S3** (50 mg, 0.155 mmol) and 3 mL DMF were added into a dried single-neck flask, followed by stirring for dissolution. The reaction mixture was added with DIPEA (160.35 mg, 1.24 mmol, 216.10 µL), cooled in an ice water bath, added with HATU (70.8 mg, 0.186 mmol), stirred at 0 °C for 5 min, added with N-methylcyclohexylmethanamine (23.7 mg, 0.186 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain an off-white solid as compound **2** (28.9 mg, 0.067 mmol, 43.2% yield, 99.0% purity). **LCMS** (ESI⁺) m/z: 432.1 [M+H]⁺.

**¹H NMR** (400 MHz, DMSO-d6) δ 7.94 (d, J = 1.7 Hz, 1H), 7.80 (dt, J = 7.8, 1.6 Hz, 1H), 7.58 - 7.38 (m, 3H), 7.31 (ddd, J = 7.7, 6.6, 1.6 Hz, 2H), 3.28 (d, J = 14.4 Hz, 3H), 2.89 (d, J = 75.7 Hz, 3H), 2.42 (s, 3H), 1.70 (dq, J = 48.1, 16.7, 13.4 Hz, 7H), 1.22 (q, J = 9.9, 8.2 Hz, 4H), 1.00 (s, 2H).

### Example 3 Preparation of compound 3

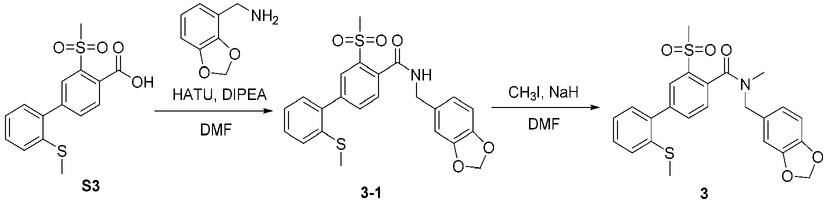

The substrate **S3** (40 mg, 0.124 µmol) and 3 mL DMF were added into a dried single-neck flask, followed by stirring for dissolution. The reaction mixture was added with DIPEA (160.35 mg, 1.24 mmol, 216.10 µL), cooled in an ice water bath, added with HATU (42.43 mg, 0.111 mmol), stirred at 0 °C for 5 min, added with 3,4-methylenedioxybenzylamine (22.51 mg, 0.149 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **3-1** (46 mg, 0.093 mmol, 74.88% yield, 92.0% purity). **LCMS** (ESI⁺) m/z: 456.0 [M+H]⁺.

A dried single-neck flask was added with NaH (5.27 mg, 0.132 mmol, 60% purity), sealed, replaced with nitrogen three times, added with 1 mL of DMF and cooled in an ice water bath. The compound **3-1** (20 mg, 0.044 mmol) was dissolved in 0.5 mL DMF, and dropwise added into the single-neck flask. The reaction mixture was stirred for 20 min, added with CH₃I (20 mg, 0.143 mmol), reacted at 0 °C for 1 h and quenched with water, followed by extraction with ethyl acetate. The resultant organic phase was washed with a saturated NaCl aqueous solution, dried over anhydrous sodium sulfate, and filtered, a filtrate was collected, concentrated and purified through medium-pressure liquid chromatography to obtain a white solid as compound 3 (13 mg, 0.026 mmol, 58.83% yield, 93.3% purity). **LCMS** (ESI⁺) m/z: 470.1 [M+H]⁺.

**¹H NMR** (400 MHz, DMSO-d6) *δ*7.96 (t, J = 2.2 Hz, 1H), 7.83 (dd, J = 7.8, 1.8 Hz, 1H), 7.59 (dd, J = 7.8, 1.6 Hz, 1H), 7.49 - 7.39 (m, 2H), 7.35 - 7.25 (m, 2H), 6.97 (dd, J = 7.1, 2.1 Hz, 1H), 6.89 - 6.84 (m, 2H), 6.07 (s, 2H), 4.86 (s, 1H), 4.50 (s, 1H), 3.33 (s, 3H), 3.31 (s, 3H), 2.42 (d, J = 4.3 Hz, 3H).

### Example 4 Preparation of compound 4

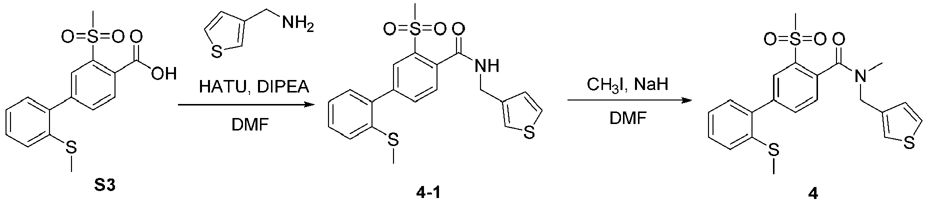

The substrate **S3** (30 mg, 0.09 mmol) and 1.5 mL DMF were added into a dried single-neck flask, followed by stirring for dissolution. The reaction mixture was added with DIPEA (117 mg, 0.9 mmol), cooled in an ice water bath, added with HATU (42.5 mg, 0.11 mmol), stirred at 0 °C for 5 min, added with 3-(aminomethyl)thiophene (12.6 mg, 0.11 mmol), and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **4-1** (15.7 mg, 0.038 mmol, 42% yield). **LCMS** (ESI⁺) m/z: 418.1 [M+H]⁺.

A dried single-neck flask was added with NaH (3.8 mg, 0.158 mmol), sealed, replaced with nitrogen three times, added with 1 mL of DMF and cooled in an ice water bath. The compound **4-1** (8 mg, 0.019 mmol) was dissolved in 0.5 mL DMF and dropwise added into the single-neck flask. The reaction mixture was stirred for 20 min, added with CH₃I (8.2 mg, 0.058 mmol), reacted at 0 °C for 1 h, and quenched with water, followed by extraction with ethyl acetate. The resultant organic phase was washed with a saturated NaCl aqueous solution, dried over anhydrous sodium sulfate, and filtered, a filtrate was collected, concentrated and purified through medium-pressure liquid chromatography to obtain a compound **4** (3.2 mg, 8.8 µmol, 46% yield). **LCMS** (ESI⁺) m/z: 432.8 [M+H]⁺.

**¹H NMR** (400 MHz, DMSO-d6) δ 7.96 (t, J = 1.8 Hz, 1H), 7.80 (ddd, J = 20.0, 7.8, 1.8 Hz, 1H), 7.64 (dd, J = 12.7, 7.8 Hz, 1H), 7.59 - 7.39 (m, 4H), 7.36 - 7.25 (m, 2H), 7.16 (ddd, J = 23.2, 4.9, 1.3 Hz, 1H), 4.83 - 4.23 (m, 2H), 2.82 (d, J = 69.3 Hz, 3H), 2.42 (d, J = 6.8 Hz, 4H), 1.19 (d, 2H).

### Example 5 Preparation of compound 5

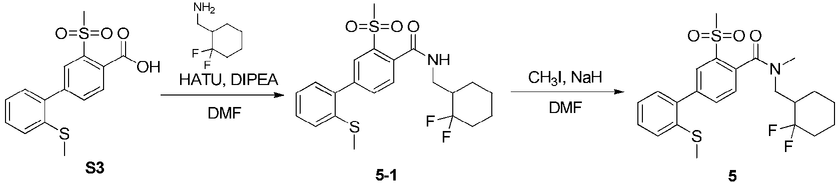

The substrate **S3** (16.1 mg, 0.05 mmol) and 1.5 mL DMF were added into a dried single-neck flask, followed by stirring for dissolution. The reaction mixture was added with DIPEA (65 mg, 0.5 mmol), cooled in an ice water bath, added with HATU (22.8 mg, 0.06 mmol), stirred at 0 °C for 5 min, added with 2,2-difluorocyclohexanemethanamine (11.1 mg, 0.06 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **5-1** (16 mg, 0.035 mmol, 71% yield). **LCMS** (ESI⁺) m/z: 454.1 [M+H]⁺.

A dried single-neck flask was added with NaH (3.4 mg, 0.086 mmol), sealed, replaced with nitrogen three times, added with 1 mL of DMF cooled in an ice water bath. The compound **5-1** (13 mg, 0.029 mmol) was dissolved in 0.5 mL DMF and dropwise added into the single-neck flask. The reaction mixture was stirred for 20 min, added with CH₃I (20 mg, 0.143 mmol), reacted at 0 °C for 1 h and quenched with water, followed by extraction with ethyl acetate. The resultant organic phase was washed with a saturated NaCl aqueous solution, dried over anhydrous sodium sulfate, and filtered, a filtrate was collected, concentrated and purified through medium-pressure liquid chromatography to obtain a compound **5** (7.4 mg, 15.8 µmol, 55% yield). **LCMS** (ESI⁺) m/z: 468.0 [M+H]⁺.

**¹H NMR** (400 MHz, DMSO-d6) δ 7.95 (d, J = 1.8 Hz, 1H), 7.82 (dd, J = 7.8, 1.8 Hz, 1H), 7.62 - 7.39 (m, 3H), 7.37 - 7.26 (m, 2H), 3.90 - 3.67 (m, 1H), 3.60 - 3.40 (m, 1H), 3.29 (s, 3H), 2.82 (s, 3H), 2.42 (s, 3H), 2.14 - 1.59 (m, 5H), 1.53 - 0.92 (m, 4H).

### Example 6 Preparation of compound 6

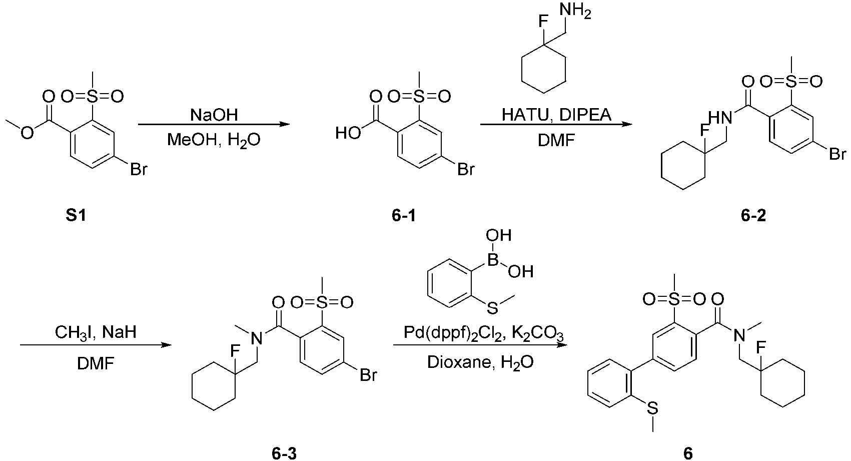

The substrate **S1** (100 mg, 0.34 mmol), 4 mL MeOH and 2 mL H₂O were added into a dried single-neck flask, followed by stirring for dissolution. The reaction mixture was added with crushed NaOH (68.5 mg, 1.71 mmol) and subjected to backflow at 50 °C for 1h, where the reaction was monitored by LC-MS. A 1M hydrochloric acid solution was added into the reaction solution until neutral, followed by extraction with ethyl acetate three times. The resultant organic phases were combined, washed with a saturated NaCl aqueous solution, dried over anhydrous sodium sulfate, and purified through medium-pressure liquid chromatography to obtain a colorless oily compound **6-1** (46 mg, 0.20 mmol, 48% yield). **LCMS** (ESI⁺) m/z: 278.9 [M+H]⁺.

A dried single-neck flask was added with 1-fluorocyclohexanemethanamine (26 mg, 0.19 mmol), 1.5 mL DMF and DIPEA (213 mg, 1.65 mmol), cooled in an ice water bath, added with HATU (69 mg, 0.18 mmol) and stirred in an ice water bath for 5 min. The compound **6-1** (46 mg, 0.20 mmol) was added into the single-neck flask and stirred in an ice water bath for 10 min, where the reaction was monitored by LC-MS. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated and purified through medium-pressure liquid chromatography to obtain a compound **6-2** (40.6 mg, 0.10 mmol, 62% yield). **LCMS** (ESI⁺) m/z: 392.2 [M+H]⁺.

A dried single-neck flask was added with NaH (4.9 mg, 0. 20mmol), sealed, replaced with nitrogen three times, added with 1 mL of DMF and cooled in an ice water bath. The compound **6-2** (40.6 mg, 0.10 mmol) was dissolved in 0.5 mL DMF and dropwise added into the single-neck flask. The reaction mixture was stirred for 20 min, added with CH₃I (29 mg, 0.20 mmol), and reacted at 0 °C for 1 h, where the reaction was monitored by LC-MS. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated and purified through medium-pressure liquid chromatography to obtain a compound **6-3** (31 mg, 76.5 µmol, 73% yield). **LCMS** (ESI⁺) m/z: 406.3 [M+H]⁺.

The compound 6-3 (15 mg, 37µmol), 2 mL 1,4-dioxane and 0.2 mL H₂O were added into a dried single-neck flask, added with 2-(methylthio)phenylboronic acid (7.5 mg, 44µmol), K₂CO₃ (15.4 mg, 111µmol) and Pd(dppf)₂Cl₂ (3 mg, 3.7µmol),sealed, replaced with nitrogen three times, and subjected to backflow at 100 °C for 2 h, where the reaction was monitored by LC-MS. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated and purified through medium-pressure liquid chromatography to obtain a green solid as compound **6** (15.5 mg, 34.5 µmol, 93% yield). **LCMS** (ESI⁺) m/z: 450.0 [M+H]⁺.

**¹H NMR** (400 MHz, DMSO-d6) δ 7.96 (d, J = 1.8 Hz, 1H), 7.82 (dd, J = 7.8, 1.8 Hz, 1H), 7.58 (d, J = 7.8 Hz, 1H), 7.50 - 7.39 (m, 2H), 7.36 - 7.26 (m, 2H), 3.29 (s, 3H), 2.88 (d, J = 1.1 Hz, 3H), 2.42 (s, 3H), 1.86 (s, 2H), 1.56 (s, 9H).

### Example 7 Preparation of compound 7

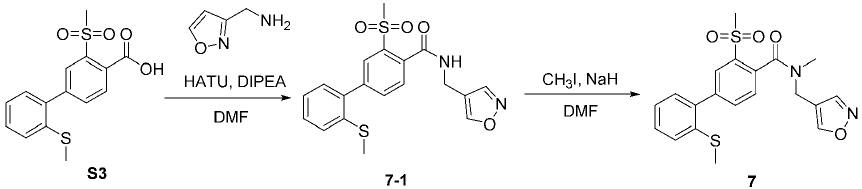

The substrate **S3** (30 mg, 93 µmol) and 1.5 mL DMF were added into a dried single-neck flask, followed by stirring for dissolution. The reaction mixture was added with DIPEA (65 mg, 0.5 mmol), cooled in an ice water bath, added with HATU (42 mg, 0.11 mmol), stirred at 0 °C for 5 min, added with 4-(aminomethyl)isoxazole (15 mg, 0.11 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **7-1** (32 mg, 80 µmol, 86% yield). **LCMS** (ESI⁺) m/z: 403.1 [M+H]⁺.

**¹H NMR** (400 MHz, DMSO-*d*₆) δ 9.36 (t, *J* = 5.9 Hz, 1H), 8.88 (d, *J* = 1.6 Hz, 1H), 7.97 (d, *J* = 1.8 Hz, 1H), 7.82 (dd, *J* = 7.8, 1.8 Hz, 1H), 7.68 (d, *J* = 7.8 Hz, 1H), 7.53 - 7.38 (m, 2H), 7.36 - 7.24 (m, 2H), 6.67 (d, *J* = 1.7 Hz, 1H), 4.56 (d, *J* = 5.9 Hz, 2H), 3.44 (s, 3H), 2.42 (s, 3H).

A dried single-neck flask was added with NaH (7 mg, 0.186 mmol), sealed, replaced with nitrogen three times, added with 1 mL of DMF and cooled in an ice water bath. The compound **7-1** (25 mg, 0.062 mmol) was dissolved in 0.5 mL DMF, dropwise added into the single-neck flask. The reaction mixture was stirred for 20 min, added with CH₃I (44 mg, 0.31 mmol) and reacted at 0 °C for 1 h. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated and purified through medium-pressure liquid chromatography to obtain a compound **7** (20 mg, 48 µmol, 77% yield). **LCMS** (ESI⁺) m/z: 417.0 [M+H]⁺.

**¹H NMR** (400 MHz, DMSO-*d*₆) δ 8.90 (d, *J* = 1.7 Hz, 1H), 7.98 (d, *J* = 2.0 Hz, 1H), 7.85 (dd, *J* = 7.8, 1.8 Hz, 1H), 7.63 (d, *J* = 7.8 Hz, 1H), 7.51 - 7.39 (m, 2H), 7.36 - 7.28 (m, 2H), 6.68 (d, *J* = 1.7 Hz, 1H), 4.44 (d, *J* = 3.2 Hz, 2H), 3.33 (s, 3H), 2.81 (s, 3H), 2.42 (s, 3H).

### Example 8 Preparation of compound 8

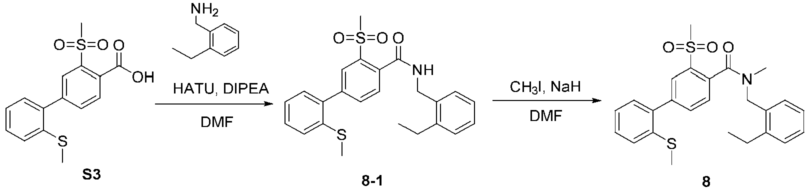

The substrate **S3** (30 mg, 0.09 mmol) and 1.5 mL DMF were added into a dried single-neck flask, followed by stirring for dissolution. The reaction mixture was added with DIPEA (117 mg, 0.9 mmol), cooled in an ice water bath, added with HATU (42.5 mg, 0.11 mmol), stirred at 0 °C for 5 min, added with (2-ethylphenyl)methanamine (15.1 mg, 0.11 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **8-1** (15 mg, 0.034 mmol, 38% yield). **LCMS** (ESI⁺) m/z: 440.6 [M+H]⁺.

A dried single-neck flask was added with NaH (5.46 mg, 0.136 mmol, 60% purity), sealed, replaced with nitrogen three times, added with 1 mL of DMF was added into the reactant, and cooled in an ice water bath. The compound **8-1** (20 mg, 0.046 µmol) was dissolved in 0.5 mL DMF, and dropwise added into the single-neck flask. The reaction mixture was stirred for 20 min, added with CH₃I (12.92 mg, 0.091 mmol) and reacted at 0 °C for 1 h. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated and purified through medium-pressure liquid chromatography to obtain a compound **8** (5 mg, 0.011 mmol, 24% yield, 99.0% purity). **LCMS** (ESI⁺) m/z: 454.2 [M+H]⁺.

**¹H NMR** (400 MHz, DMSO-d6) *δ* 7.96 (dd, J = 14.4, 1.8 Hz, 1H), 7.84 (dd, J = 7.8, 1.9 Hz, 1H), 7.68 - 7.58 (m, 1H), 7.42 (ddd, J = 16.1, 13.6, 7.3 Hz, 4H), 7.35 - 7.30 (m, 1H), 7.29 - 7.23 (m, 3H), 5.03 (d, J = 15.2 Hz, 1H), 4.48 (d, J = 15.3 Hz, 1H), 3.34 (s, 3H), 2.72 (s, 2H), 2.42 (s, 3H), 1.21 (t, J = 7.5 Hz, 3H).

### Example 9 Preparation of compound 9

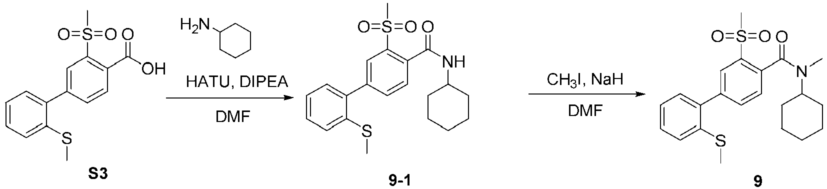

The substrate **S3** (10.0 mg, 0.031 mmol) and 3 mL DMF were added into a dried single-neck flask, followed by stirring for dissolution. The reaction mixture was added with DIPEA (65 mg, 0.5 mmol), cooled in an ice water bath, added with HATU (14.2 mg, 0.037 mmol) and stirred at 0 °C for 5 min, added with cyclohexylamine (4.0 mg, 0.040 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **9-1** (11 mg, 0.027 mmol, 88% yield). **LCMS** (ESI⁺) m/z: 404.2 [M+H]⁺.

A dried single-neck flask was added with NaH (3.2 mg, 60%wt, 0.081 mmol) under the protection of N₂, added with 2 mL of DMF, and cooled in an ice water bath. The compound **9-1** (11 mg, 0.027 mmol) was dissolved in 1 mL DMF, and dropwise added into the single-neck flask. The reaction mixture was stirred for 20 min, added with CH₃I (18.9 mg, 0.135 mmol) and reacted at 0 °C for 1 h. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated and purified through medium-pressure liquid chromatography to obtain a compound **9** (6 mg, 14.4 µmol, 53% yield). **LCMS** (ESI⁺) m/z: 418.1 [M+H]⁺.

**¹H NMR** (400 MHz, Chloroform-d) δ 8.08 (dd, J = 15.2, 1.8 Hz, 1H), 7.66 (dd, J = 7.7, 1.8 Hz, 1H), 7.36 - 7.27 (m, 2H), 7.27 - 7.22 (m, 1H), 7.19 (d, J = 2.2 Hz, 1H), 7.16 (dd, J = 6.7, 1.7 Hz, 1H), 3.22 (s, 3H), 2.68 (s, 3H), 2.33 (s, 3H), 2.03 - 1.87 (m, 1H), 1.85 - 1.59 (m, 4H), 1.57 - 1.34 (m, 4H), 1.11 - 0.91 (m, 2H).

### Example 10 Preparation of compound 10

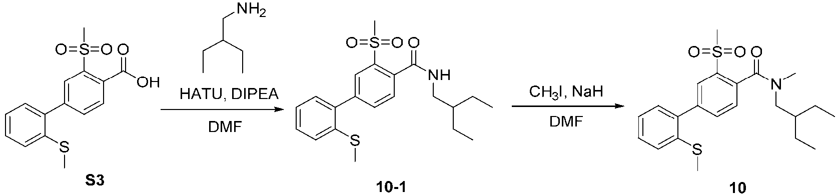

The substrate **S3** (30 mg, 0.09 mmol) and 1.5 mL DMF were added into a dried single-neck flask, followed by stirring for dissolution. The reaction mixture as added with DIPEA (117 mg, 0.9 mmol), cooled in an ice water bath, added with HATU (42.5 mg, 0.11 mmol), and stirred at 0 °C for 5 min. The reaction mixture was added with 3-aminopentane (11.4 mg, 0.11 mmol) and reacted at 0 °C for 20 min, where the reaction completion was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **10-1** (12.5 mg, 0.031 mmol, 34% yield). **LCMS** (ESI⁺) m/z: 406.6 [M+H]⁺.

A dried single-neck flask was added with NaH (2.4 mg, 0.1 mmol), sealed, replaced with nitrogen three times, added with 1 mL of DMF and cooled in an ice water bath. The compound **10-1** (20 mg, 0.046 µmol) was dissolved in 0.5 mL DMF, and dropwise added into the single-neck flask. The reaction mixture was stirred for 20 min, added with CH₃I (8.4 mg, 0.059 mmol) and reacted at 0 °C for 1 h, where the reaction mixture was quenched with water, followed by extraction with ethyl acetate. The resultant organic phase was washed with a saturated NaCl aqueous solution, dried over anhydrous sodium sulfate, and filtered, a filtrate was collected, concentrated and purified through medium-pressure liquid chromatography to obtain a compound **10** (4.4 mg, 10.5 µmol, 36% yield). **LCMS** (ESI⁺) m/z: 420.6 [M+H]⁺.

**¹H NMR** (400 MHz, DMSO-d6) δ 7.94 (t, J = 2.3 Hz, 1H), 7.86 - 7.75 (m, 1H), 7.58 - 7.39 (m, 3H), 7.37 - 7.26 (m, 2H), 3.13 (d, J = 108.1 Hz, 2H), 2.78 (s, 3H), 2.41 (d, J = 7.7 Hz, 3H), 1.81 - 1.04 (m, 7H), 0.90 (t, J = 7.4 Hz, 5H), 0.68 (q, J = 7.8 Hz, 2H).

### Example 11 Preparation of compound 11

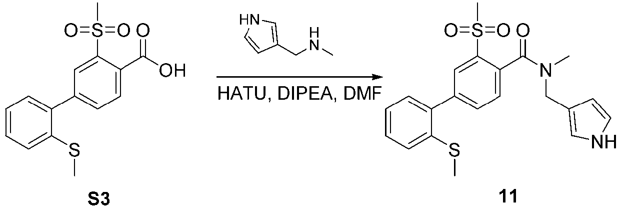

The substrate **S3** (15 mg, 0.047 mmol) and 1 mL DMF were added into a dried single-neck flask to dissolve and cooled to 0 °C. The reaction mixture was added with DIPEA (40 µmol, 0.23 mmol), reacted with 5 min, added with HATU (21.26 mg, 0.056 mmol) and reacted for 10 min. The reaction mixture was added with N-methyl-1-(1H-pyrrolidin-3-yl)methanamine (10.25 mg, 0.093 mmol) and reacted for 0.5 h, where the reaction was monitored by LC-MS. The reaction mixture was quenched with water, followed by extraction with ethyl acetate. The remnant was purified through high performance liquid chromatography (basic) to obtain a compound **11** (7.1 mg, 0.017 mmol). **LCMS** (ESI) m/z: 415.1 [M+H]⁺. HPLC method B: R_{T} = 8.44 min, purity > 99.9%.

**¹H NMR** (600 MHz, DMSO-*d*₆) δ 10.70 (d, *J* = 28.8 Hz, 1H), 7.96 - 7.95 (m, 1H), 7.81 - 7.79 (m, 1H), 7.63 - 7.50 (m, 1H), 7.48 - 7.44 (m, 1H), 7.42 - 7.40 (m, 1H), 7.34 - 7.27 (m, 2H), 6.83 - 6.74 (m, 1H), 6.73 - 6.71 (m, 1H), 6.11 - 6.06 (m, 1H), 4.67 - 4.40 (m, 1H), 4.18 - 3.02 (m, 1H), 3.33 (s, 3H), 2.88 - 2.68 (m, 3H), 2.42 (d, *J* = 5.4 Hz, 3H).

### Example 12 Preparation of compound 12

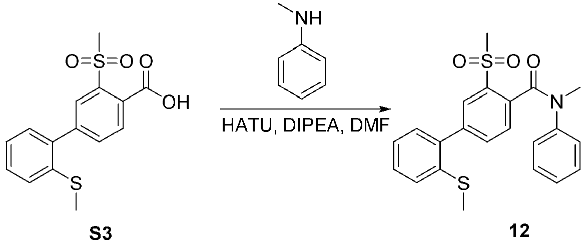

The substrate **S3** (20 mg, 0.062 mmol) and 1 mL DMF were added into a dried single-neck flask, and cooled to 0 °C. The reaction mixture was added with DIPEA (40 µL, 0.23 mmol), reacted for 5 min, added with HATU (28.3 mg, 0.074 mmol), and reacted for 10 min. The reaction mixture was added with N-methylaniline (8 mg, 0.074 mmol) and react for 0.5 h, where the reaction completion was monitored by LC-MS. The reaction liquid was subjected to decompression concentration, and the remnant was purified through high performance liquid chromatography (basic) to obtain a compound **12** (2.0 mg, 4.9 µmol, 8% yield). **LCMS** (ESI) m/z: 412.1 [M+H]⁺.

**¹H NMR** (400 MHz, DMSO-d6) δ 8.02 - 7.79 (m, 2H), 7.51 - 7.44 (m, 2H), 7.43 - 7.32 (m, 4H), 7.25 (tt, J = 7.1, 6.0 Hz, 4H), 7.20 - 7.12 (m, 2H), 3.42 (d, J = 6.5 Hz, 5.5H), 3.14 (s, 0.5H), 2.44 - 2.31 (m, 3H).

### Example 13 Preparation of compound 13

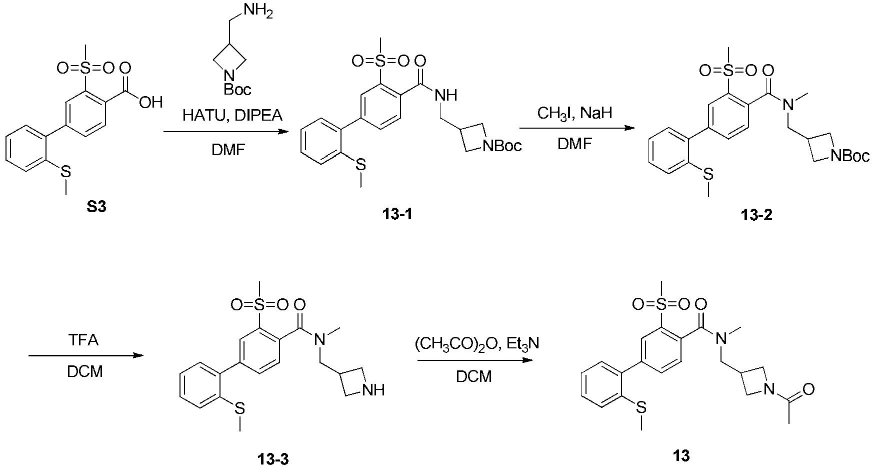

The substrate **S3** (30 mg, 0.093 mmol) and 1.5 mL DMF were added into a dried single-neck flask, followed by stirring for dissolution. The reaction mixture was added with DIPEA (117 mg, 0.9 mmol), cooled in an ice water bath, reacted for 5 min, added with HATU (28.3 mg, 0.074 mmol) and reacted for 10 min. The reaction mixture was added with N-methylaniline (8 mg, 0.074 mmol) and reacted for 0.5 h, added with HATU (42.1 mg, 0.111 mmol), stirred at 0 °C for 5 min, added with 1-Boc-3-(aminomethyl)azetidine (20.7 mg, 0.111 mmol), and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **13-1** (11.0 mg, 0.023 mmol, 24% yield). **LCMS** (ESI⁺) m/z: 491.2 [M+H]⁺.

A dried single-neck flask was added with NaH (2.8 mg, 0.115 mmol) was added into a dried single-neck flask, sealed, replaced with nitrogen three times, added with 1 mL of DMF and cooled in an ice water bath. The compound **13-1** (11 mg, 0.023 mmol) was dissolved in 0.5 mL DMF, dropwise added into the single-neck flask, stirred for 20 min, added with CH₃I (9.8 mg, 0.069 mmol) and reacted at 0 °C for 1 h. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated and purified through medium-pressure liquid chromatography to obtain a compound **13-2** (10.45 mg, 0.002 mmol, 90% yield). **LCMS** (ESI⁺) m/z: 505.2 [M+H]⁺.

The compound **13-2** was dissolved in 2 mL DCM, slowly dropwise added with 1 mL of TFA, stirred for 20 min, and directly subjected to rotary evaporation in a high-temperature water bath to obtain a crude compound **13-3.** The crude compound **13-3** can be subjected to subsequent reaction without purification.

The crude compound **13-3** and 2 mL of DCM were added into a dried single-neck flask, followed by stirring for dissolution. The reaction mixture was added with an excess amount of acetic anhydride (7.2 mg, 0.071 mmol) and triethylamine (14.3 mg, 0.141 mmol) and reacted at a room temperature overnight, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a light yellow solid as compound **13** (4.15 mg, 9.3 µmol, 20% yield). **LCMS** (ESI⁺) m/z: 447.1 [M+H]⁺.

**¹H NMR** (400 MHz, DMSO-d6) δ 7.98 - 7.92 (m, 1H), 7.81 (dd, J = 7.8, 1.8 Hz, 1H), 7.63 - 7.53 (m, 1H), 7.50 - 7.39 (m, 2H), 7.31 (q, J = 3.4, 2.9 Hz, 2H), 4.23 (t, J = 8.5 Hz, 1H), 3.95 (d, J = 11.3 Hz, 2H), 3.64 (m, 1H), 3.28 (d, J = 3.0 Hz, 4H), 2.98 (s, 2H), 2.79 (s, 3H), 2.40 (d, J = 4.4 Hz, 3H), 1.77 (s, 3H).

### Example 14 Preparation of compound 14

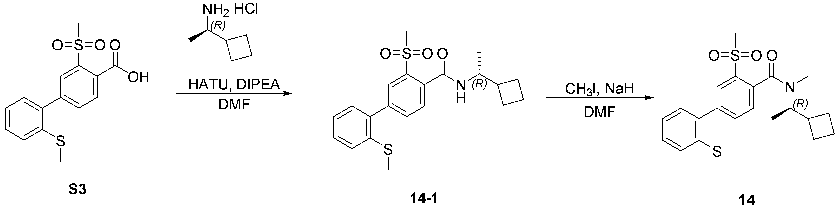

The substrate **S3** (50 mg, 155 µmol), 1mL DMF and DIPEA (100 mg, 776 µmol) were added into a dried single-neck flask, and then placed in an ice water bath. The reaction mixture was added with HATU (64.9 mg, 170 µmol), stirred in an ice water bath for 5 min, added with (R)-α-methyl-cyclobutanemethylamine hydrochloride (21 mg, 155 µmol), and stirred in an ice water bath for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and filtered, a filtrate was collected, concentrated, and purified through medium-pressure liquid chromatography to obtain a white solid as compound **14-1** (54 mg, 133.9 µmol, 86.7% yield). **LCMS** (ESI⁺) m/z: 404.1 [M+H]⁺.

**¹H NMR** (400 MHz, DMSO-d6) δ 8.48 (d, J = 8.5 Hz, 1H), 7.94 (d, J = 1.8 Hz, 1H), 7.79 (dd, J = 7.7, 1.8 Hz, 1H), 7.54 (d, J = 7.7 Hz, 1H), 7.50 - 7.38 (m, 2H), 7.35 - 7.23 (m, 2H), 3.96 (td, J = 8.7, 6.6 Hz, 1H), 3.42 (s, 3H), 2.43 (s, 3H), 2.40 - 2.32 (m, 1H), 1.98 (dq, J = 9.9, 3.5 Hz, 2H), 1.92 - 1.73 (m, 4H), 1.04 (d, J = 6.6 Hz, 3H).

A dried single-neck flask was added with NaH (2.16 mg, 90.2 µmol) was added into a dried single-neck flask, sealed, replaced with nitrogen three times, added with 0.3 mL DMF, cooled in an ice water bath. The compound **14-1** (28 mg, 69.4 µmol) was dissolved in 1 mL DMF, and then slowly added into the single-neck flask, stirred in an ice water bath for 20 min. CH₃I (10.84 mg,74.6 µmol) was dissolved in 0.5 mL DMF, and then slowly added into the single-neck flask, and stirred at the room temperature for 30 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate and filtered, and a filtrate was collected, concentrated, and purified through medium-pressure liquid chromatography to obtain a white solid as compound **14** (13.16 mg, 31.5 µmol, 45.4 yield). **LCMS** (ESI⁺) m/z: 418.1 [M+H]⁺.

**¹H NMR** (400 MHz, DMSO-d6) δ 8.04 - 7.92 (m, 1H), 7.89 - 7.77 (m, 1H), 7.65 - 7.40 (m, 3H), 7.40 - 7.25 (m, 2H), 4.58 (dq, J = 10.4, 6.7 Hz, 1H), 3.29 (d, J = 12.4 Hz, 3H), 2.82 (s, 0.75H), 2.58 (d, J = 14.6 Hz, 3H), 2.48 (s, 0.25H), 2.43 (d, J = 4.1 Hz, 3H), 2.16 - 1.52 (m, 6H), 1.19 - 0.96 (m, 3H).

### Example 15 Preparation of compound 15

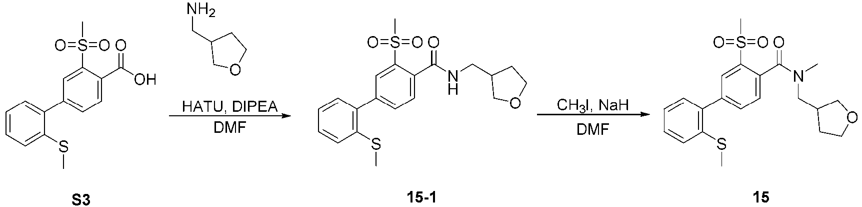

The substrate **S3** (30 mg, 93.1 µmol) was dissolved in 1 mL DMF, dropwise added into DIPEA (60 mg, 465 µmol) and placed in an ice water bath, added with HATU (38.9 mg, 102.4 µmol), stirred in an ice water bath for 5 min. The reaction mixture was added with 3-(aminomethyl)tetrahydrofuran (10.4 mg, 102.4 µmol) and stirred in an ice water bath for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and filtered, a filtrate was collected, concentrated, and purified through medium-pressure liquid chromatography to obtain a white solid as compound **15-1** (35 mg, 86.4 µmol, 92.7% yield). **LCMS** (ESI⁺) m/z: 406.2 [M+H]⁺.

A dried single-neck flask was added with NaH (2.1 mg, 86.6 µmol), sealed, replaced with nitrogen three times, added with 0.3 mL DMF, and cooled in an ice water bath. The compound **15-1** (27 mg, 66.6 µmol) was dissolved in 1 mL DMF, and slowly added into the single-neck flask, and stirred at the room temperature for 20 min. CH₃I (11.3 mg,80 µmol) was dissolved 0.5 mL DMF, and then slowly added into the single-neck flask, and stirred at the room temperature for 30 min, where the reaction completion was monitored by LC-MS. The reaction mixture was quenched with water, followed by extraction with ethyl acetate three times. The resultant organic phases were combined, washed with a saturated NaCl aqueous solution, dried over anhydrous sodium sulfate, and filtered, a filtrate was collected, concentrated, and purified through medium-pressure liquid chromatography to obtain a white solid as compound **15** (7.12 mg,16.9 µmol, 25.4% yield). **LCMS** (ESI⁺) m/z: 420.0 [M+H]⁺.

**¹H NMR** (400 MHz, DMSO-d6) δ 7.95 (t, J = 1.7 Hz, 1H), 7.81 (dd, J = 7.8, 1.8 Hz, 1H), 7.63 - 7.53 (m, 1H), 7.50 - 7.40 (m, 2H), 7.31 (pd, J = 7.5, 6.9, 2.6 Hz, 2H), 3.88 - 3.76 (m, 1.61H), 3.73 - 3.63 (m, 1H), 3.61 - 3.42 (m, 3H), 3.30 (d, J = 7.8 Hz, 3H), 3.10 (dd, J = 10.2, 7.5 Hz, 0.52H), 3.02 (d, J = 3.3 Hz, 0.75H), 2.82 (s, 2.31H), 2.73 - 2.55 (m, 1.27H),2.42 (d, J = 4.0 Hz, 3H), 1.96 (ddt, J = 56.0, 13.6, 6.2 Hz, 1H), 1.78 - 1.44 (m, 1H).

### Example 16 Preparation of compound 16

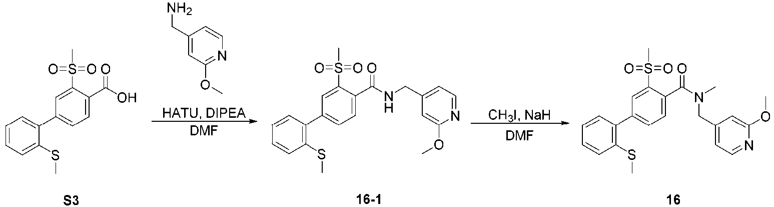

The substrate **S3** (50 mg, 155 µmol) and 1mL DMF were added into a dried single-neck flask, slowly added with DIPEA (100 mg, 776 µmol), and then placed in an ice water bath. The reaction mixture was added with HATU (64.9 mg, 170 µmol), stirred in an ice water bath for 5 min, added with (2-methoxypyridin-4-yl)methanamine (23.6 mg, 170 µmol) and stirred in an ice water bath for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and filtered, a filtrate was collected, concentrated, and purified through medium-pressure liquid chromatography to obtain a white solid as compound **16-1** (50 mg, 113 µmol, 72.8% yield). **LCMS** (ESI⁺) m/z: 443.1 [M+H]⁺.

NaH (3.5 mg, 147 µmol) was added into a dried single-neck flask, sealed, replaced with nitrogen three times. 1 mL DMF was added into the reactant, followed by cooling in an ice water bath. The compound **16-1** (50 mg, 113 µmol) was dissolved in 1 mL DMF, and then slowly added into the single-neck flask, and stirred in an ice water bath for 20 min. CH₃I (19.3 mg, 135 µmol) was dissolved in 0.5 mL DMF, and then slowly added into the single-neck flask, and stirred at the room temperature for 30 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate and filtered, and a filtrate was collected, concentrated, and purified through medium-pressure liquid chromatography to obtain a white solid as compound **16** (51 mg, 111 µmol, 98.9% yield). **LCMS** (ESI⁺) m/z: 457.1 [M+H]⁺.

**¹H NMR** (400 MHz, DMSO-d6) δ 8.21 - 8.11 (m, 1H), 7.98 (dd, J = 9.7, 1.8 Hz, 1H), 7.86 (dd, J = 7.8, 1.8 Hz, 1H), 7.66 (dd, J = 30.1, 7.8 Hz, 1H), 7.51 - 7.38 (m, 2H), 7.37 - 7.24 (m, 2H), 7.03 (ddd, J = 29.0, 5.3, 1.4 Hz, 1H), 6.94 - 6.78 (m, 1H), 4.34 (d, J = 2.5 Hz, 2H), 3.86 (d, J = 9.9 Hz, 3H), 3.35 (d, J = 4.0 Hz, 3H), 2.86 (d, J = 59.8 Hz, 3H), 2.42 (d, J = 11.6 Hz, 3H).

### Synthesis of compound S4

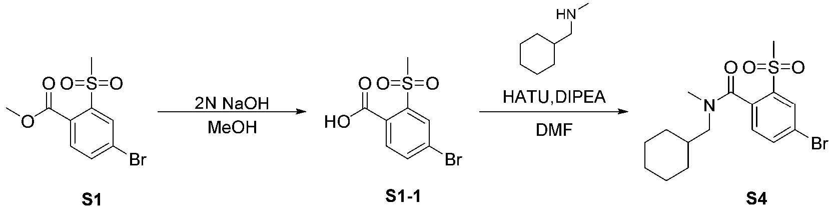

### Synthesis of compound S1-1

The substrate **S1** (500 mg, 1.71 mmol) and 5 mL MeOH were added into a dried single-neck flask, followed by stirring for dissolution. The reaction mixture was added with a 2N NaOH solution (2 M, 8.53 mL), and stirred at 50 °C for 1 h, where the reaction was monitored by LC-MS. The reaction mixture was subjected to rotary evaporation, pH adjustment to 5-6 with a 3N HCl solution, and extraction with ethyl acetate three times. The resultant organic phases were combined, washed with a saturated NaCl aqueous solution, dried over anhydrous sodium sulfate, and subjected to decompression concentration, and the remnant was purified through column chromatography to obtain a compound **S1-1** (463 mg, 1.66 mmol, 97.25% yield). **LCMS** (ESI⁺) m/z: 280.9 [M+H]⁺.

### Synthesis of compound 4

The compound **S1-1** (430 mg, 1.54 mmol) and 8 mL DMF were added into a dried single-neck flask, followed by stirring for dissolution. The reaction mixture was added with DIPEA (597.33 mg, 4.62 mmol), cooled in an ice water bath, added with HATU (702.95 mg, 1.85 mmol) and stirred at 0 °C for 5 min. The reaction mixture was added with N-methyl-1-cyclohexylmethanamine (235.21 mg, 1.85 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate and subjected to decompression concentration, and the remnant was purified through medium-pressure liquid chromatography to obtain a compound **S4** (380 mg, 978.58 µmol, 63.52% yield). **LCMS** (ESI⁺) m/z: 389.2 [M+H]⁺.

### Example 17 Preparation of compound 17

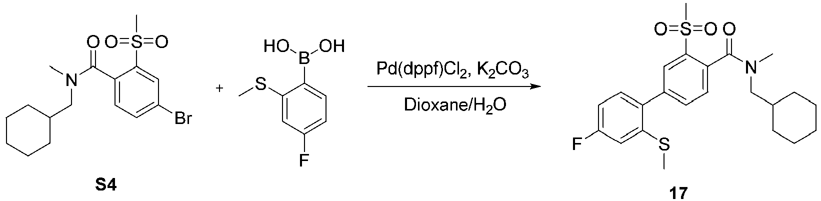

The compound **S4** (15 mg, 38.7 µmol), 4-fluoro-2-(methylthio)phenylboronic acid (8.6 mg, 46.5 µmol), Pd(dppf)Cl₂ (1.4 mg, 1.93 µmol) and K₂CO₃ (10.69 mg, 77.5 µmol) were added into a dried single-neck flask, sealed, replaced with nitrogen three times. 1 mL dioxane and 0.25 mL H₂O were added into the single-neck flask, and stirred at 100 °C for 2 h, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and filtered, a filtrate was collected, concentrated, and purified through medium-pressure liquid chromatography to obtain a white solid as compound **17** (12.37 mg, 27.5 µmol, 71% yield). **LCMS** (ESI⁺) m/z: 450.5 [M+H]⁺.

**¹H NMR** (600 MHz, DMSO-d6) δ 7.92 (d, J = 1.8 Hz, 1H), 7.79 (dt, J = 7.8, 1.5 Hz, 1H), 7.52 (dd, J = 10.5, 7.8 Hz, 1H), 7.37 (ddd, J = 11.5, 8.4, 6.0 Hz, 1H), 7.24 (dd, J = 10.2, 2.6 Hz, 1H), 7.12 (td, J = 8.4, 2.5 Hz, 1H), 3.54 (d, J = 10.1 Hz, 1H), 3.28 (d, J = 21.6 Hz, 3H), 3.09 (d, J = 13.0 Hz, 1H), 2.99 (s, 0.86H), 2.95 - 2.82 (m, 1H), 2.79 (s, 2.2H), 2.46 (d, J = 4.0 Hz, 3H), 1.86 - 1.54 (m, 6H), 1.29 - 1.16 (m, 3H), 1.08 - 0.65 (m, 2H).

### Example 18 Preparation of compound 18

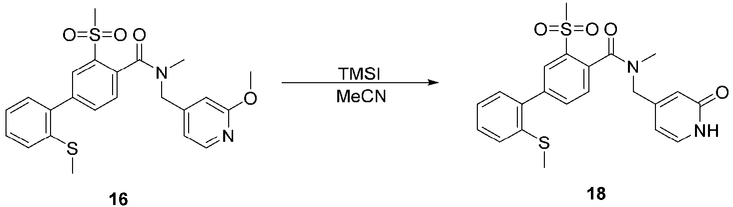

The compound **16** (25 mg, 54.8 µmol), 2 mL MeCN and TMSI (43.8 mg, 219 µmol) were added into a dried single-neck flask, followed by backflow stirring at 50 °C for 12 h, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and filtered, a filtrate was collected, concentrated, and purified through medium-pressure liquid chromatography to obtain a yellow solid as compound **18** (2.66 mg, 6 µmol, 10.9% yield). **LCMS** (ESI⁺) m/z: 443.1 [M+H]⁺.

**¹H NMR** (400 MHz, DMSO-d6) δ 11.50 (s, 1H), 7.97 (dd, J = 7.0, 1.8 Hz, 1H), 7.82 (ddd, J = 30.3, 7.8, 1.8 Hz, 1H), 7.61 (dd, J = 31.3, 7.8 Hz, 1H), 7.51 - 7.39 (m, 2H), 7.38 - 7.25 (m, 3H), 6.42 - 6.10 (m, 2H), 4.59 - 4.12 (m, 2H), 3.42 (d, J = 3.7 Hz, 3H), 2.86 (d, J = 65.2 Hz, 3H), 2.42 (d, J = 8.6 Hz, 3H).

### Example 19 Preparation of compound 19

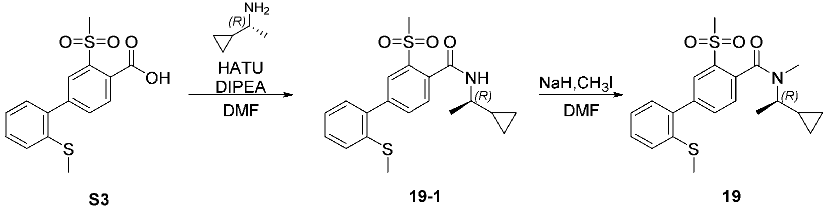

The substrate **S3** (30 mg, 0.093 mmol) and 1 mL DMF were added into a dried single-neck flask, followed by stirring for dissolution. The reaction mixture was added with DIPEA (24.05 mg, 186.10 mmol), cooled in an ice water bath, added with HATU (28.31 mg, 74.44 µmol), stirred at 0 °C for 5 min, added with (R)-1-cyclopropylethylamine hydrochloride (7.54 mg, 62.03 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and subjected to decompression concentration, and the remnant was purified through medium-pressure liquid chromatography to obtain a compound **19-1** (24 mg, 61.61 µmol, 99.32% yield). **LCMS** (ESI⁺) m/z: 390.1 [M+H]⁺.

Under the protection of N₂, NaH (2.96 mg, 73.94 µmol, 60% purity) was added into a dried single-neck flask, dispersed in 1 mL DMF, cooled in an ice water bath. The compound **19-1** (24 mg, 61.61 µmol) was slowly dropwise added into the reaction system, and reacted at 0 °C for 30 min. The reaction mixture was added with iodomethane (10.49 mg, 73.94 µmol), heated to the room temperature, and stirred for 2 h, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and subjected to decompression concentration, and the remnant was purified through column chromatography to obtain a white solid as compound **19** (14.8 mg, 36.16 µmol, 58.69% yield, 98.6% purity). **LCMS** (ESI⁺) m/z: 404.1 [M+H]⁺.

**¹H NMR** (400 MHz, DMSO-*d*₆) δ 7.98 - 7.92 (m, 1H), 7.85 - 7.76 (m, 1H), 7.60 - 7.39 (m, 3H), 7.35 - 7.26 (m, 2H), 4.03 - 3.78 (m, 1H), 3.32 - 3.26 (m, 3H), 2.99 (d, *J* = 9.3 Hz, 1H), 2.76 (d, *J* = 9.0 Hz, 2H), 2.42 (s, 3H), 1.29 - 1.19 (m, 2H), 1.13 (dd, *J* = 9.9, 6.7 Hz, 1H), 1.11 - 0.99 (m, 1H), 0.64 - 0.16 (m, 4H).

### Example 20 Preparation of compound 20

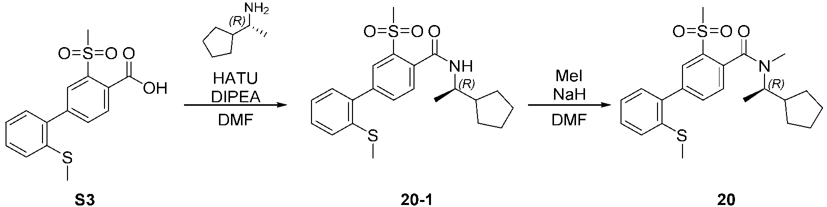

The substrate **S3** (20 mg, 62.03 mmol) and 1 mL DMF were added into a dried single-neck flask, followed by stirring for dissolution. The reaction mixture was added with DIPEA (24.05 mg, 186.10 mmol), cooled in an ice water bath, added with HATU (28.31 mg, 74.44 µmol), stirred at 0 °C for 5 min, added with (R)-1-cyclopentylethylamine hydrochloride (11.14 mg, 74.44 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and subjected to decompression concentration, and the remnant was purified through medium-pressure liquid chromatography to obtain a compound **20-1** (25.5 mg, 61.07 µmol, 98.44% yield). **LCMS** (ESI⁺) m/z: 418.2 [M+H]⁺.

Under the protection of N₂, NaH (2.93 mg, 73.28 µmol, 60% purity) was added into a dried single-neck flask, dispersed in 1 mL DMF, and cooled in an ice water bath. The compound **20-1** (25.5 mg, 61.07 µmol) was slowly dropwise added into the reaction system to react at 0 °C for 30 min. The reaction mixture was added with iodomethane (10.40 mg, 73.28 µmol), heated to the room temperature, and stirred for 2 h, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and subjected to decompression concentration, and the remnant was purified through column chromatography to obtain a white solid as compound **20** (16.7 mg, 38.69 µmol, 63.36% yield, 100% purity). **LCMS** (ESI⁺) m/z: 432.1 [M+H]⁺.

**¹H NMR** (400 MHz, DMSO-*d*₆) δ 8.00 - 7.91 (m, 1H), 7.81 (d, *J* = 6.9 Hz, 1H), 7.54 - 7.39 (m, 3H), 7.36 - 7.26 (m, 2H), 4.47 - 4.26 (m, 1H), 3.29 (d, *J* = 12.0 Hz, 3H), 2.94 - 2.61 (m, 3H), 2.42 (s, 3H), 2.10 - 1.99 (m, 1H), 1.86 - 1.69 (m, 2H), 1.68 - 1.47 (m, 4H), 1.45 - 1.20 (m, 3H), 1.14 (d, *J* = 6.9 Hz, 2H).

### Example 21 Preparation of compound 21

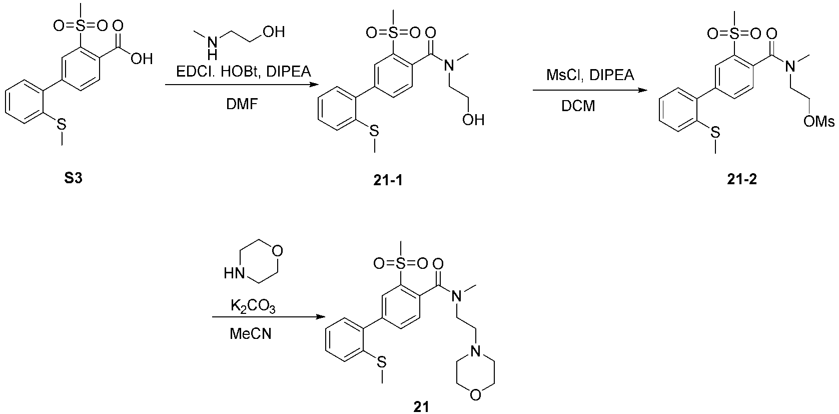

The substrate **S3** (280 mg, 0.87mmol) and 3.0 mL DMF were added into a dried single-neck flask, followed by stirring for dissolution. The reaction mixture was added with EDCI (176 mg, 1.3 mmol), HOBt (249 mg, 1.3 mmol), DIPEA (0.45 mL, 2.61 mmol) and 2-(methylamino)ethanol (78.3 mg, 1.04 mmol), and stirred at the room temperature for 16 h, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **21-1** (250 mg, 0.66 mmol, 75% yield).

The compound **21-1** (100 mg, 0.26 mmol), 2 mL CH₂Cl₂ and DIPEA (0.14 mL, 0.78 mmol) were added into a dried single-neck flask, cooled in an ice water bath, added with MsCl (90 mg, 0.78 mmol), heated to the room temperature and stirred for 30 min, where the reaction was monitored by TLC (thin-layer chromatography). The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated, and purified through medium-pressure liquid chromatography to obtain a compound **21-2** (80 mg, 0.17 mmol, 66% yield).

The compound **21-2** (33 mg, 0.07 mmol) was added into a 25 mL single-neck flask, dissolved in 3 mL acetonitrile, added with K₂CO₃ (30 mg, 0.21 mmol) and morpholine (7.5 mg, 0.08 mmol), heated to 60 °C and reacted for 2 h, where the completion was monitored by LCMS (liquid chromatography-mass spectrometry). The reaction mixture was quenched with water, followed by extraction with ethyl acetate. The resultant organic phase was washed with a saturated NaCl aqueous solution, dried over anhydrous sodium sulfate, and filtered, and a filtrate was collected, concentrated, and purified through medium-pressure liquid chromatography to obtain a compound **21** (18 mg, 0.04 mmol, 55% yield). **LCMS** (ESI⁺) m/z: 449.5 [M+H]⁺.

**¹H NMR** (400 MHz, DMSO-*d*₆) δ 7.94 (d, *J* = 1.8 Hz, 0.6H), 7.92 (d, *J* = 1.8 Hz, 0.4H), 7.81 (ddd, *J* = 8.6, 7.8, 1.8 Hz, 1H), 7.61 (d, *J* = 7.8 Hz, 0.4H), 7.53 (d, *J* = 7.8 Hz, 0.6H), 7.50 - 7.38 (m, 2H), 7.35 - 7.25 (m, 2H), 3.82 - 3.38 (m, 5.2H), 3.30 (s, 1.8H), 3.28 (s, 1.2H), 3.26 - 3.05 (m, 0.8H), 3.03 (s, 1.2H), 2.83 (s, 1.8H), 2.71 - 2.57 (m, 0.5H), 2.50 - 2.30 (m, 7H), 2.22 (t, *J* = 4.7 Hz, 1.5H).

### Example 22 Preparation of compound 22

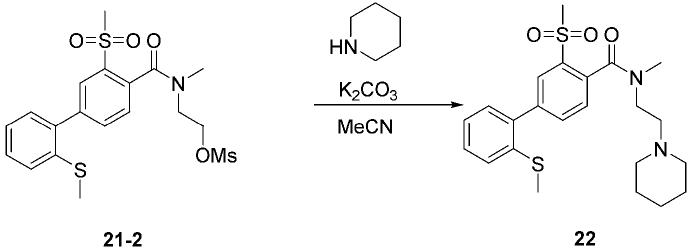

The compound **21-2** (40 mg, 0.087 mmol) was added into the 25 mL single-neck flask, dissolved in 3 mL acetonitrile, added with K₂CO₃ (36 mg, 0.26mmol) and piperidine (9 mg, 0.10 mmol), heated to 60 °C and reacted for 2 h, where the reaction was monitored by LCMS. The reaction mixture was quenched with water, followed by extraction followed by extraction with ethyl acetate. The resultant organic phase was washed with a saturated NaCl aqueous solution, dried over anhydrous sodium sulfate and filtered, and a filtrate was collected, concentrated, and purified through medium-pressure liquid chromatography to obtain a compound **22** (32.2 mg, 0.072 mmol, 82% yield). **LCMS** (ESI⁺) m/z: 447.1 [M+H]⁺. **¹H NMR** (400 MHz, DMSO-*d*₆) δ 7.94 (d, *J* = 1.8 Hz, 0.6H), 7.92 (d, *J* = 1.8 Hz, 0.4H), 7.81 (ddd, *J* = 11.3, 7.7, 1.8 Hz, 1H), 7.63 - 7.50 (m, 1H), 7.49 - 7.36 (m, 2H), 7.36 - 7.25 (m, 2H), 3.76 - 3.39 (m, 1.2H), 3.31 (s, 1.8H), 3.28 (s, 1.2H), 3.22 - 3.04 (m, 0.8H), 3.02 (s, 1.2H), 2.83 (s, 1.8H), 2.65 - 2.35 (m, 7.5H), 2.26 - 2.09 (m, 1.5H), 1.61 - 1.28 (m, 6H).

### Example 23 Preparation of compound 23

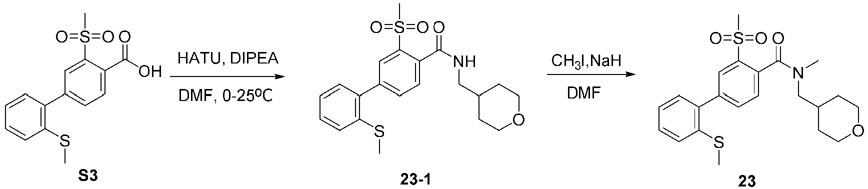

The substrate **S3** (30 mg, 0.093 mmol) and 1 mL DMF were added into a dried single-neck flask, followed by stirring for dissolution. The reaction mixture was added with DIPEA (60.13 mg, 0.465 mmol), cooled in an ice water bath, added with HATU (42.43 mg, 0.112 mmol) and stirred at 0 °C for 5 min. The reaction mixture was added with N-methyltetrahydropyran (12.86 mg, 0.111 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **23-1** (35 mg, 0.0083 mmol, 89.65% yield). **LCMS** (ESI⁺) m/z: 420.2 [M+H]⁺.

A dried single-neck flask was added with NaH (2.86 mg, 0.07 mmol, 60% purity) was added into a dried single-neck flask, sealed, replaced with nitrogen three times, added with 1 mL of DMF was added into the reaction system, and cooled in an ice water bath. The compound **23-1** (15 mg, 0.036 mmol) was dissolved in 0.5 mL DMF, and dropwise added into the single-neck flask. The temperature was kept and the reaction mixture was stirred for 20 min, added with CH₃I (15.22 mg, 0.107 mmol) and reacted at 0 °C for 1 h. The reaction mixture was quenched with water, followed by extraction with ethyl acetate three times. The resultant organic phases were combined, washed with a saturated NaCl aqueous solution, dried over anhydrous sodium sulfate, and subjected to decompression concentration, and the remnant was purified through column chromatography to obtain a white solid as compound **23** (6 mg, 0.014 mmol, 38.51% yield, 99.5% purity). **LCMS** (ESI⁺) m/z: 434.2 [M+H]⁺.

**¹H NMR** (400 MHz, DMSO-d6) δ 7.94 (d, J = 1.7 Hz, 1H), 7.80 (ddd, J = 7.8, 5.0, 1.8 Hz, 1H), 7.55 (dd, J = 7.8, 6.7 Hz, 1H), 7.50 - 7.39 (m, 2H), 7.37 - 7.25 (m, 2H), 3.88 (ddd, J = 11.3, 4.5, 2.0 Hz, 2H), 3.78 (s, 1H), 3.48 (d, J = 9.0 Hz, 1H), 3.32 - 3.30 (m, 1H), 3.30 (s, 2H), 3.26 (s, 1H), 3.22 (dd, J = 11.4, 2.3 Hz, 1H), 3.00 (s, 1H), 2.81 (s, 2H), 2.42 (d, J = 3.1 Hz, 3H), 2.03 (s, 1H), 1.69 (s, 2H), 1.26 (d, J = 13.4 Hz, 2H).

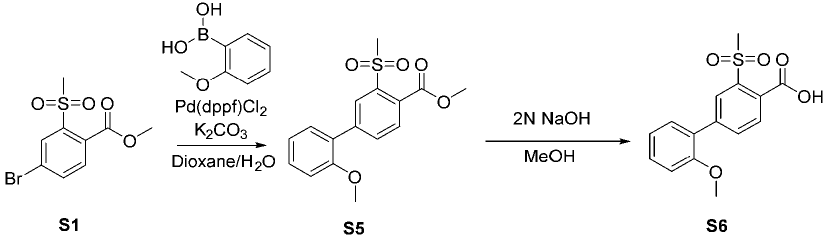

### Synthesis of compound S5

Under the protection of N₂, the substrate **S1** (1 g, 3.41 mmol), 2-methoxyphenylboronic acid (605.90 mg, 4.09 mmol), Pd(dppf)Cl₂ (248.92 mg, 0.341 mmol), K₂CO₃ (1.41 g, 10.23 mmol) and 2 mL of 1, dioxane/ H₂O (v:v=3:1) were added into a microwave tube, and stirred at 100°C for 2h, where the reaction was monitored through LC-MS. The reaction mixture was subjected to rotary evaporation, and extraction with ethyl acetate three times. The resultant organic phases were combined, washed with a saturated NaCl aqueous solution, dried over anhydrous sodium sulfate, and purified through medium-pressure liquid chromatography to obtain a brown solid as compound **S5** (1.2 g, crude). **LCMS** (ESI⁺) m/z: 321.1 [M+H]⁺.

### Synthesis of compound S6

The compound **S5** and 5 mL MeOH were added into a dried single-neck flask, followed by stirring for dissolution. The reaction mixture was added with a 2N NaOH solution (449.49 mg, 11.24 mmol) and reacted under stirring at 60 °C for 2 h, where the reaction was monitored by LC-MS. The reaction mixture was subjected to rotary evaporation, pH adjustment to 5-6 with a 3N HCl solution, freeze-drying and purification through medium-pressure liquid chromatography to obtain a light reddish-brown oily solid as compound **S6** (730 mg, 2.38 mmol, 63.62% yield). **LCMS** (ESI⁻) m/z: 305.2 [M-H]⁻.

### Example 24 Preparation of compound 24

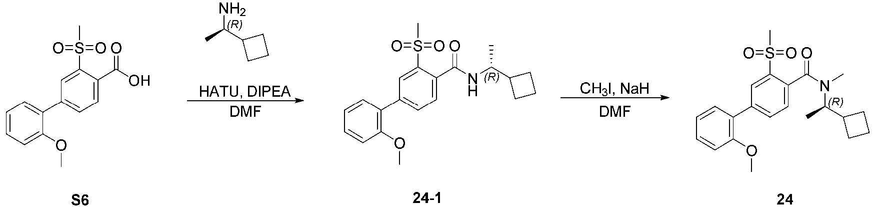

The compound **S6** (30 mg, 98 µmol), 1 mL DMF and DIPEA (63.2 mg, 490 µmol) were added into a dried single-neck flask, and placed in an ice water bath. The reaction mixture was added with HATU (40.9 mg, 107.8 µmol), stirred in an ice water bath for 5 min, added with (R)- ALPHA-cyclobutylethylamine hydrochloride (14.6 mg, 107.8 µmol), and stirred in an ice water bath for 20 min, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **24-1** (31 mg, 80 µmol, 81.6% yield). **LCMS** (ESI⁺) m/z: 388.3 [M+H]⁺.

A dried single-neck flask was added with NaH (2.5 mg, 104 µmol), sealed, replaced with nitrogen three times, added with 0.3 mL DMF, and cooled in an ice water bath. The compound **24-1** (31 mg, 80 µmol) was dissolved in 1 mL DMF, and slowly added into the reaction system, and stirred in an ice water bath for 20 min. CH₃I (12.5 mg, 88 µmol) was dissolved in 0.5 mL DMF, and slowly added into the single-neck flask, and stirred at the room temperature for 30 min, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **24** (10.18 mg, 25.3 µmol, 31.6% yield). **LCMS** (ESI⁺) m/z: 402.0 [M+H]⁺.

**¹H NMR** (400 MHz, DMSO-d6) δ 8.16 - 8.01 (m, 1H), 8.00 - 7.85 (m, 1H), 7.62 - 7.34 (m, 3H), 7.26 - 7.15 (m, 1H), 7.14 - 7.04 (m, 1H), 4.58 (dq, J = 10.4, 6.7 Hz, 0.73H), 3.82 (d, J = 6.1 Hz, 3H), 3.51 - 3.39 (m, 0.31H), 3.33 - 3.25 (m, 3H), 2.82 (s, 0.85H), 2.58 (d, J = 14.7 Hz, 2.75H), 2.15 - 1.49 (m, 6H), 1.16 - 0.97 (m, 3H).

### Example 25 Preparation of compound 25

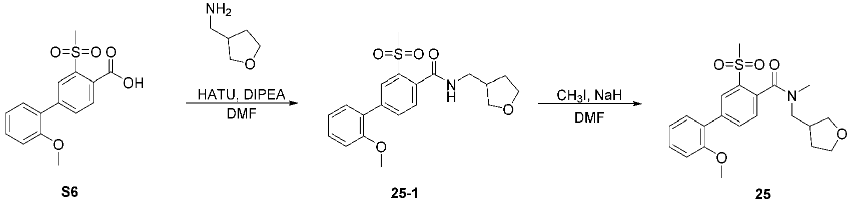

The compound S6 (30 mg, 98 µmol), 1 mL DMF and DIPEA (63.2 mg, 490 µmol) were added into a dried single-neck flask, and placed in an ice water bath. The reaction mixture was added with HATU (40.9 mg, 107.8 µmol), stirred in an ice water bath for 5 min, added with (tetrahydrofuran-3-yl)methanamine (10.9 mg, 107.8 µmol) and stirred in an ice water bath for 20 min, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **25-1** (36.8 mg, 94.5 µmol, 96.4% yield). **LCMS** (ESI⁺) m/z: 390.2 [M+H]⁺.

A dried single-neck flask was added with NaH (1.9 mg, 80.1 µmol) was added into a dried single-neck flask, sealed, replaced with nitrogen three times, added with 0.3 mL DMF was added into the reaction system, and cooled in an ice water bath. The compound **25-1** (24 mg, 61.6 µmol) was dissolved in 1 mL DMF, and slowly added into the single-neck flask, and stirred in an ice water bath for 20 min. CH₃I (12.5 mg, 88 µmol) was dissolved in 0.5 mL DMF, slowly added into the single-neck flask, and stirred at the room temperature for 30 min, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **25** (15.9 mg, 39.4 µmol, 63.9% yield). **LCMS** (ESI⁺) m/z: 404.0 [M+H]⁺.

**¹H NMR** (400 MHz, DMSO-d6) δ 8.06 (dd, J = 6.7, 1.7 Hz, 1H), 7.91 (dd, J = 7.9, 1.8 Hz, 1H), 7.57 - 7.49 (m, 1H), 7.44 (t, J = 7.6 Hz, 2H), 7.23 - 7.16 (m, 1H), 7.10 (td, J = 7.5, 1.0 Hz, 1H), 3.87 - 3.76 (m, 4.75H), 3.74 - 3.62 (m, 1H), 3.53 (dt, J = 19.2, 8.6 Hz, 3H), 3.31 (s, 3H), 3.16 - 3.06 (m, 0.52H), 3.01 (d, J = 3.3 Hz, 0.76H), 2.83 (s, 2.4H), 2.73 - 2.55 (m, 1H), 2.12 - 1.84 (m, 1H), 1.79 - 1.43 (m, 1H).

### Example 26 Preparation of compound 26

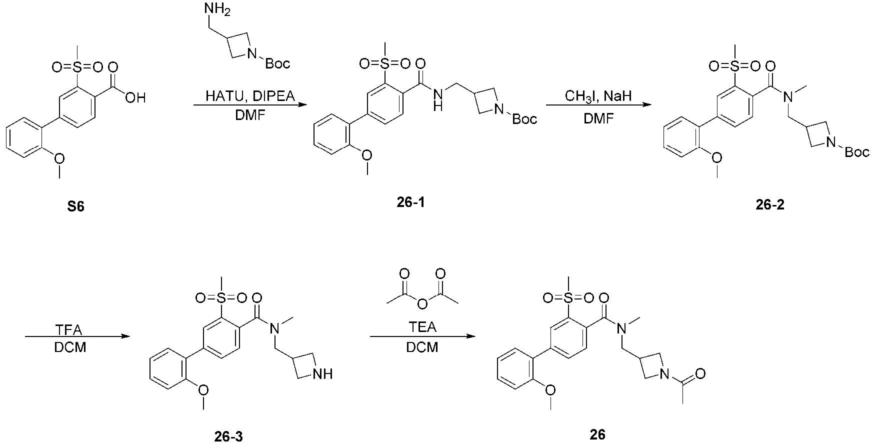

The compound **S6** (35 mg, 114 µmol), 2 mL DMF and DIPEA (73.7 mg, 571 µmol) were added into a dried single-neck flask, and placed in an ice water bath. The reaction mixture was added with HATU (47.8 mg, 125 µmol), stirred in an ice water bath for 5 min, added with 1-Boc-3-(aminomethyl)azetidine (21.2 mg, 114 µmol) and stirred in an ice water bath for 20 min, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **26-1** (50 mg, 105 µmol, 92.2% yield). **LCMS** (ESI⁺) m/z: 473.2 [M+H]⁺.

A dried single-neck flask was added with NaH (3.3mg, 137 µmol) was added into a dried single-neck flask, sealed, replaced with nitrogen three times, added with 0.5 mL DMF and cooled in an ice water bath. The compound **26-1** (50 mg, 105 µmol) was dissolved in 1 mL DMF, and slowly added into the single-neck flask, and stirred in an ice water bath for 20 min. CH₃I (17.9 mg,126 µmol) was dissolved in 0.5 mL DMF, and slowly added into the single-neck flask, and stirred at the room temperature for 30 min, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **26-2** (40 mg, 81.9 µmol, 77.7% yield). **LCMS** (ESI) m/z: 487.2 [M-H]⁻.

The compound **26-2** (40 mg, 81.9 µmol), 1 mL DCM and 1 mL TFA were added into a dried single-neck flask, and stirred at the room temperature for 30 min, where the reaction was monitored by LC-MS. The reaction mixture was subjected to rotary evaporation, added with water and extraction with ethyl acetate three times. The resultant organic phases were combined, washed with a saturated NaCl aqueous solution, and filtered to obtain a crude compound **26-3** (60 mg). The crude compound **26-3** can be directly subjected to the subsequent reaction without purification. **LCMS** (ESI⁺) m/z: 389.2 [M+H]⁺.

The crude compound **26-3** (60 mg), 2 mL DCM, TEA (24.8 mg, 245 µmol) and acetic anhydride (12.5 mg, 122 µmol) were added into a dried single-neck flask, and stirred at the room temperature for 30 min, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **26** ((25.7 mg, 59.7 µmol, 72.9% yield). **LCMS** (ESI⁺) m/z: 431.1 [M+H]⁺.

**¹H NMR** (600 MHz, DMSO-d6) δ 8.12 (dd, J = 18.7, 1.7 Hz, 1H), 7.97 (dd, J = 7.8, 1.9 Hz, 1H), 7.64 - 7.54 (m, 1H), 7.50 (td, J = 7.9, 3.6 Hz, 2H), 7.24 (d, J = 8.2 Hz, 1H), 7.16 (t, J = 7.4 Hz, 1H), 4.34 - 4.13 (m, 1H), 4.01 (ddt, J = 25.5, 14.3, 7.0 Hz, 2H), 3.87 (s, 3H), 3.85 - 3.58 (m, 2H), 3.53 - 3.45 (m, 0.75H), 3.35 (d, J = 3.7 Hz, 3H), 3.09 - 3.04 (m, 0.58H), 3.01 - 2.94 (m, 0.27H), 2.85 (s, 2.48H), 1.81 (s, 3H), 1.44 - 0.79 (m, 1H).

### Example 27 Preparation of compound 27

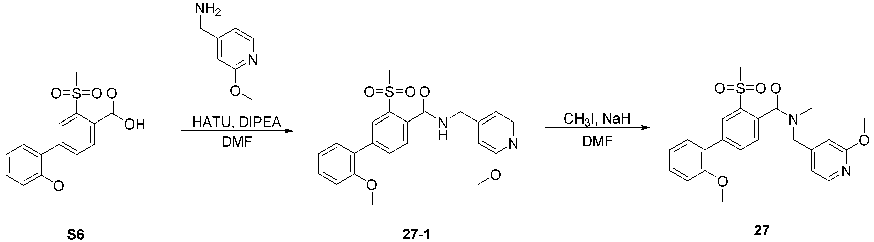

The compound **S6** (30 mg, 98 µmol), 1 mL DMF and DIPEA (63.2 mg, 490µmol) were added into a dried single-neck flask, and placed in an ice water bath. The reaction mixture was added with HATU (40.9 mg, 107 µmol) was added into the reaction system, stirred in an ice water bath for 5 min, added with (2-methoxypyridin-4-yl)methanamine (14.9 mg, 107µmol) and stirred in an ice water bath for 20 min, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **27-1** (40 mg, 93.8 µmol, 95.7% yield). **LCMS** (ESI⁺) m/z: 427.1 [M+H]⁺.

A dried single-neck flask was added with NaH (2.9 mg, 122 µmol) was added into a dried single-neck flask, sealed, replaced with nitrogen three times, added with 0.5 mL DMF and cooled in an ice water bath. The compound **27-1** (40 mg, 93.8 µmol) was dissolved in 1 mL DMF, slowly added into the single-neck flask, and stirred in an ice water bath for 20 min. CH₃I (15.9 mg, 112 µmol) was dissolved in 0.5 mL DMF, slowly added into the single-neck flask, and stirred at the room temperature for 30 min, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **27** (39 mg, 88.6 µmol, 94.3% yield). **LCMS** (ESI⁺) m/z: 441.4 [M+H]⁺.

**¹H NMR** (400 MHz, DMSO-d6) δ 8.19 - 8.06 (m, 2H), 7.90 (ddd, J = 36.9, 7.9, 1.8 Hz, 1H), 7.61 (dd, J = 26.8, 7.9 Hz, 1H), 7.43 (tdd, J = 10.9, 5.1, 2.3 Hz, 2H), 7.33 - 7.14 (m, 2H), 7.14 - 6.95 (m, 2H), 6.86 (d, J = 45.6 Hz, 1H), 4.35 (d, J = 7.8 Hz, 2H), 3.83 (dd, J = 20.9, 12.2 Hz,6H), 3.35 (d, J = 2.9 Hz, 3H), 2.85 (d, J = 54.8 Hz, 3H).

### Example 28 Preparation of compound 28

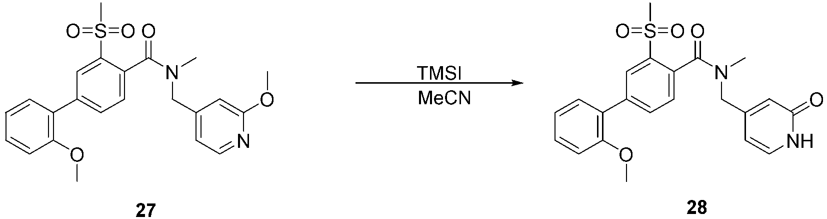

The compound **27** (25mg, 56.8 µmol), 2 mL MeCN and TMSI (45.4 mg, 227 µmol) were added into a dried single-neck flask, and subjected to backflow stirring at 50 °C for 12 h, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a yellow solid as compound **28** (18.3 mg, 42.9 µmol, 75.6% yield). **LCMS** (ESI⁺) m/z: 427.4 [M+H]⁺.

**¹H NMR** (400 MHz, DMSO-d6) δ 8.14 (dd, J = 4.8, 1.7 Hz, 1H), 7.97 (ddd, J = 30.2, 7.8, 1.7 Hz, 1H), 7.63 (dd, J = 35.2, 7.9 Hz, 1H), 7.55 - 7.38 (m, 3H), 7.27 - 7.11 (m, 2H), 6.53 - 6.24 (m, 2H), 4.68 - 4.18 (m, 2H), 3.87 (d, J = 9.5 Hz, 3H), 3.39 (s, 3H), 2.92 (d, J = 59.0 Hz, 3H).

### Example 29 Preparation of compound 29

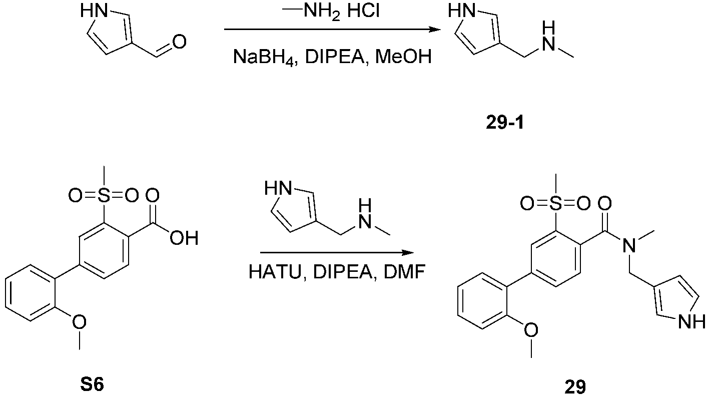

Pyrrole-3-carbaldehyde (190.2 mg, 2 mmol) and methylamine hydrochloride (162mg, 2.4 mmol) were dissolved in 5 mL methyl alcohol, added with DIPEA (1.25 mL, 7.2 mmol), reacted at the room temperature for 2 h, cooled to 0 °C, added with sodium borohydride (379.5 mg, 10 mmol) and reacted at 0 °C for 1 h, where the reaction was monitored by LC-MS. The reaction mixture was added with water, extracted with dichloromethane, dried over anhydrous sodium sulfate, filtered and subjected to decompression concentration to obtain a compound **29-1** (150 mg, crude product), and the compound **29-1** can be directly used in subsequent reaction. **LCMS** (ESI⁺) m/z: 111.2 [M+H]⁺.

The compound **S6** (15 mg, 0.048 mmol) was added into a dried single-neck flask, dissolved in 1 mL DMF, cooled to 0 °C, added with DIPEA (41 µL, 0.24 mmol), reacted for 5 min, added with HATU (21.66 mg, 0.057 mmol) and reacted for 10 min. The compound **29-1** (10.44 mg, 0.095 mmol) was added into the single-neck flask and reacted for 0.5 h, where the reaction was monitored by LC-MS. The reaction mixture was subjected to decompression concentration, and the remnant was purified through high performance liquid chromatography (basic) to obtain a white solid as compound **29** (10.1 mg, 0.025 mmol, 52.0% yield). **LCMS** (ESI⁺) m/z: 399.2 [M+H]⁺. HPLC method B: RT = 7.14 min, purity > 99.9%. **¹H NMR** (600 MHz, DMSO-d6) δ 10.69 (d, J = 26.4 Hz, 1H), 8.06 - 8.05 (m, 1H), 7.91 - 7.88 (m, 1H), 7.59 - 7.45 (m, 1H), 7.44 - 7.41 (m, 2H), 7.20 - 7.16 (m, 1H), 7.11 - 7.07(m, 1H), 6.83 - 6.73 (m, 1H), 6.73 - 6.69 (m, 1H), 6.11 - 6.04 (m, 1H), 4.66 - 4.38 (m, 1H), 4.05 - 3.80 (m, 1H), 3.81 (d, J = 3.0 Hz, 3H), 3.34 (s, 3H), 2.87 - 2.68 (m, 3H).

### Example 30 Preparation of compound 30

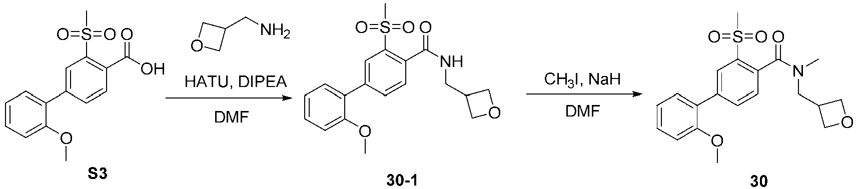

The substrate **S3** (20 mg, 0.065 mmol) and 1 mL DMF were added into a dried single-neck flask, followed by stirring for dissolution. The reaction mixture was added with DIPEA (0.11 mL, 0.65 mmol), cooled in an ice water bath, added with HATU (30 mg, 0.078 mmol), stirred at 0 °C for 5 min, added with 3-(aminomethyl)oxetane (6.8 mg, 0.078 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **30-1** (24 mg, 0.064 mmol, 99% yield). **LCMS** (ESI⁺) m/z: 376.1 [M+H]⁺.

A dried single-neck flask was added with NaH (8 mg, 0.2 mmol), sealed, replaced with nitrogen three times, added with 1 mL of DMF and cooled in an ice water bath. The compound **30-1** (24 mg, 0.064 mmol) was dissolved in 0.5 mL DMF, and dropwise added into the. The temperature was kept and the reaction mixture was stirred for 20 min. CH₃I (28 mg, 0.2 mmol) was added into the single-neck flask and reacted at 0 °C for 1 h. The reaction mixture was quenched with water, followed by extraction with ethyl acetate three times. The resultant organic phases were combined, washed with a saturated NaCl aqueous solution, dried over anhydrous sodium sulfate, and filtered, a filtrate was collected, concentrated, and purified through medium-pressure liquid chromatography to obtain a compound **30** (14 mg, 36 µmol, 56% yield). **LCMS** (ESI⁺) m/z: 390.1 [M+H]⁺.

**¹H NMR** (400 MHz, DMSO-*d*₆) δ 8.07 (d, *J =* 1.8 Hz, 0.24H), 8.02 (d, *J =* 1.8 Hz, 0.71H), 7.92 - 7.85 (m, 1H), 7.57 - 7.33 (m, 3H), 7.21 - 7.12 (m, 1H), 7.13 - 7.04 (m, 1H), 4.68 (dd, *J* = 7.9*,* 6.1 Hz, 1.58H), 4.62 - 4.51 (m, 0.57H), 4.53 - 4.35 (m, 1.56H), 4.23 - 4.12 (m, 0.64H), 4.08 - 3.95 (m, 0.72H), 3.78 (s, 0.79H), 3.77 (s, 2.21H), 3.66 - 3.52 (m, 0.72H), 3.46 - 3.26 (m, 1.64H), 3.23 (s, 3H), 2.89 (s, 0.76H), 2.72 (s, 2.24H).

### Example 31 Preparation of compound 31

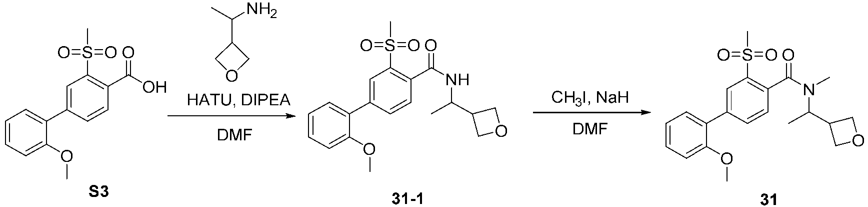

The substrate **S3** (20 mg, 0.062 mmol) and 1.5 mL DMF were added into a dried single-neck flask, followed by stirring for dissolution. The reaction mixture was added with DIPEA (117 mg, 0.9 mmol), cooled in an ice water bath, added with HATU (28.3 mg, 0.074 mmol), stirred at 0 °C for 5 min, added with 1-(oxetan-3-yl)ethan-1-amine (7.5 mg, 0.074 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **31-1** (15.6 mg, 0.04 mmol, 65% yield). **LCMS** (ESI⁺) m/z: 390.5 [M+H]⁺.

A dried single-neck flask was added with NaH (1.8 mg, 0.077 mmol), sealed, replaced with nitrogen three times, added with 1 mL of DMF and cooled in an ice water bath. The compound **31-1** (6 mg, 0.015 mmol) was dissolved in 0.5 mL DMF, and dropwise added into the single-neck flask. The reaction mixture was subjected to temperature keeping, stirred for 20 min, added with CH₃I (6.6 mg, 0.046 mmol) and reacted at 0 °C for 1 h. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated, and purified through medium-pressure liquid chromatography to obtain a compound **31** (2.34 mg, 5.8 µmol, 39% yield). **LCMS** (ESI⁺) m/z: 404.4 [M+H]⁺.

**¹H NMR** (400 MHz, DMSO-d6) δ 8.02 (d, J = 1.8 Hz, 1H), 8.00 - 7.84 (m, 1H), 7.62 - 7.35 (m, 3H), 7.16 (t, J = 7.1 Hz, 1H), 7.13 - 7.05 (m, 1H), 4.94 (t, J = 8.3 Hz, 1H), 4.78 - 4.14 (m, 4H), 3.78 (d, J = 8.9 Hz, 3H), 3.29 - 3.12 (m, 4H), 2.75 (s, 1H), 1.23 - 1.01 (m, 4H).

### Example 32 Preparation of compound 32

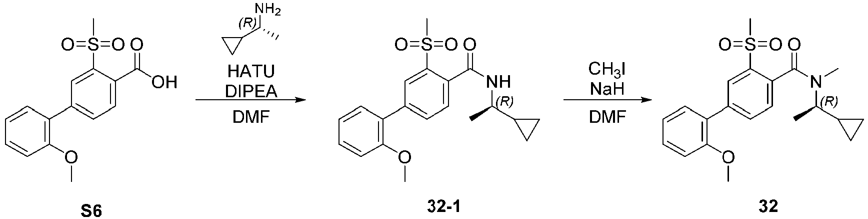

The compound **S6** (20 mg, 65.29 mmol) and 1 mL DMF were added into a dried single-neck flask, followed by stirring for dissolution. The reaction mixture was added with DIPEA (25.31 mg, 195.87 mmol), cooled in an ice water bath, added with HATU (29.79 mg, 78.35 µmol), stirred at 0 °C for 5 min, added with (R)-1-cyclopropylethylamine hydrochloride (9.53 mg, 78.35 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and subjected to decompression concentration, and the remnant was purified through column chromatography to obtain a compound **32-1** (21 mg, 56.23 µmol, 86.13% yield). **LCMS** (ESI⁺) m/z: 374.2 [M+H]⁺.

Under the protection of N₂, NaH (1.93 mg, 48.20 µmol, 60% purity) was dispersed in 1 mL DMF, and cooled in an ice water bath. The compound **32-1** (15 mg, 40.16 µmol) was slowly dropwise added into the single-neck flask and reacted at 0 °C for 30 min. The reaction mixture was added with iodomethane (6.84 mg, 48.20 µmol), heated to the room temperature and stirred for 2 h, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and subjected to decompression concentration, and the remnant was purified through column chromatography to obtain a white solid as compound 32 (5.03 mg, 12.98 µmol, 32.32% yield, 100% purity). **LCMS** (ESI⁺) m/z: 388.1 [M+H]⁺.

**¹H NMR** (400 MHz, DMSO-*d*₆) δ 7.87 - 7.80 (m, 1H), 7.70 - 7.62 (m, 1H), 7.32 - 7.17 (m, 3H), 7.01 - 6.93 (m, 1H), 6.91 - 6.84 (m, 1H), 3.78 - 3.61 (m, 1H), 3.59 (s, 3H), 3.09 - 3.03 (m, 3H), 2.75 (d, *J* = 9.2 Hz, 1H), 2.54 (d, *J* = 9.6 Hz, 2H), 1.07 - 0.95 (m, 3H), 0.93 - 0.86 (m, 1H), 0.41 - -0.05 (m, 4H).

### Example 33 Preparation of compound 33

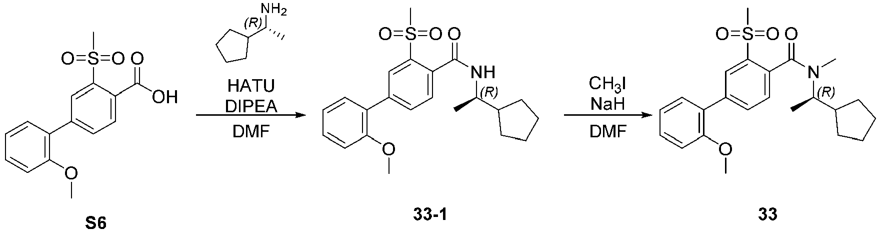

The compound **S6** (20 mg, 65.29 mmol) and 1 mL DMF were added into a dried single-neck flask, followed by stirring for dissolution. The reaction mixture was added with DIPEA (25.31 mg, 195.87 mmol), cooled in an ice water bath, added with HATU (29.79 mg, 78.35 µmol), stirred at 0 °C for 5 min, added with (R)-1-cyclopentylethylamine hydrochloride (11.73 mg, 78.35 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and subjected to decompression concentration, and the remnant was purified through column chromatography to obtain a compound **33-1** (23 mg, 57.28 µmol, 87.74% yield). **LCMS** (ESI⁺) m/z: 402.2 [M+H]⁺.

Under the protection of N₂, NaH (1.79 mg, 44.83 µmol, 60% purity) was dispersed in 1 mL DMF and cooled in an ice water bath. The compound **33-1** (15 mg, 37.36 µmol) was slowly dropwise added into the reaction mixture and reacted at 0 °C for 30 min. The reaction mixture was added with iodomethane (6.36 mg, 44.83 µmol), heated to the room temperature and stirred for 2 h, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and subjected to decompression concentration, and the remnant was purified through column chromatography to obtain a white solid as compound 33 (6.3 mg, 15.16 µmol, 40.58% yield, 100% purity). **LCMS** (ESI⁺) m/z: 416.1 [M+H]⁺.

**¹H NMR** (400 MHz, DMSO-*d*₆) δ 8.18 - 8.08 (m, 1H), 8.01 - 7.92 (m, 1H), 7.53 - 7.44 (m, 3H), 7.25 (d, *J* = 7.9 Hz, 1H), 7.20 - 7.12 (m, 1H), 4.50 - 4.33 (m, 1H), 3.88 (s, 3H), 3.36 (s, 3H), 2.94 - 2.69 (m, 3H), 2.23 - 2.02 (m, 1H), 1.91 - 1.53 (m, 6H), 1.38 - 1.17 (m, 5H).

### Example 34 Preparation of compound 34

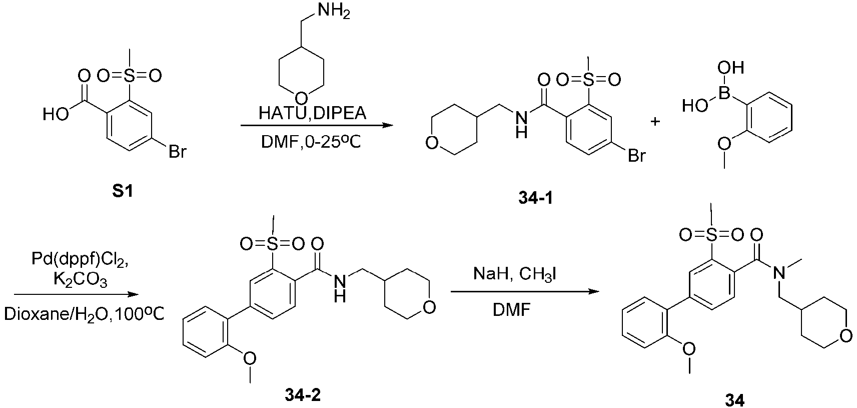

The substrate **S1** (50 mg, 0.179 mmol) and 1 mL DMF were added into a dried single-neck flask, followed by stirring for dissolution. The reaction mixture was added with DIPEA (115.76 mg, 0.896 mmol), cooled in an ice water bath, added with HATU (70.92 mg, 0.215 mmol), stirred at 0 °C for 5 min, added with 4-(aminomethyl)tetrahydropyran (20.63 mg, 0.179 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, purified through medium-pressure liquid chromatography to obtain a white solid as compound **34-1** (50 mg, 0.134 mmol, 74.18% yield). **LCMS** (ESI⁺) m/z: 377.9 [M+H]⁺.

Under the protection of N₂, the compound **34-1** (50 mg, 0.134 mmol), 2-methoxyphenylboronic acid (24.36 mg, 0.160 mmol), Pd(dppf)Cl₂ (9.75 mg, 0.013 mmol), K₂CO₃ (55.39 mg, 0.400 mmol) and 2 mL of 1, dioxane/ H₂O (v:v=3:1) were added into a microwave tube, and stirred at 100°C for 2h, where the reaction was monitored through LC-MS. The reaction mixture was subjected to rotary evaporation, and extraction with ethyl acetate three times. The resultant organic phases were combined, washed with a saturated NaCl aqueous solution, dried over anhydrous sodium sulfate, and purified through medium-pressure liquid chromatography to obtain a brown solid as compound **34-2** (50 mg, 0.124 mmol, 92.76% yield). **LCMS** (ESI⁺) m/z: 404.1 [M+H]⁺.

A dried single-neck flask was added with NaH (5.98 mg, 0.149 mmol, 60% purity), sealed, replaced with nitrogen three times, added with 1 mL DMF and cooled in an ice water bath. The compound **34-2** (20.10 mg, 0.050 mmol) was dissolved in 0.5 mL DMF, dropwise added into the single-neck flask, subjected to temperature keeping, stirred for 20 min, added with CH₃I (14.14 mg, 0.100 mmol) and reacted at 0 °C for 1 h. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated, and purified through medium-pressure liquid chromatography to obtain a compound **34** (10 mg, 0.023 mmol, 46.50% yield, 96.7% purity). **LCMS** (ESI⁺) m/z: 418.3 [M+H]⁺.

**¹H NMR** (400 MHz, DMSO-d6) δ 8.05 (dd, J = 6.0, 1.8 Hz, 1H), 7.89 (dd, J = 7.9, 1.8 Hz, 1H), 7.50 (t, J = 7.8 Hz, 1H), 7.43 (dd, J = 9.0, 7.0 Hz, 2H), 7.18 (d, J = 8.2 Hz, 1H), 7.14 - 7.05 (m, 1H), 3.88 (ddd, J = 11.3, 4.5, 2.0 Hz, 2H), 3.81 (d, J = 2.0 Hz, 3H), 3.48 (d, J = 9.3 Hz, 1H), 3.32 - 3.18 (m, 6H), 3.00 (s, 1H), 2.82 (s, 2H), 2.02 (s, 1H), 1.69 (s, 2H), 1.39 - 1.16 (m, 2H).

### Example 35 Preparation of compound 35

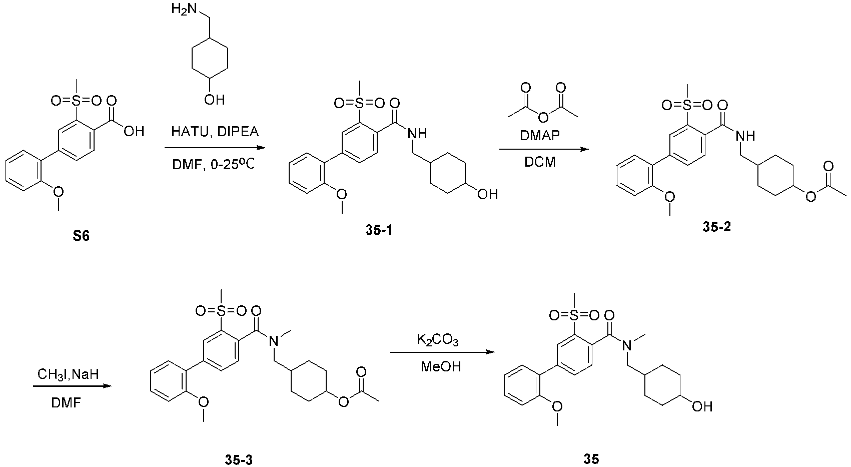

The compound **S6** (40 mg, 0.131 mmol) and 1 mL DMF were added into a dried single-neck flask, followed by stirring for dissolution. The reaction mixture was added with DIPEA (84.38 mg, 0.653 mmol), cooled in an ice water bath, added with HATU (59.54 mg, 0.157 mmol), stirred at 0 °C for 5 min, added with 4-(aminomethyl)cyclohexanol (20.24 mg, 0.157 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **35-1** (40 mg, 0.096 mmol, 73.37% yield). **LCMS** (ESI⁺) m/z: 418.1 [M+H]⁺.

The compound **35-1** (20 mg, 0.048 mmol) and 3 mL DCM were added into a dried single-neck flask, followed by stirring for dissolution. The reaction mixture was added with acetic anhydride (24.45 mg, 0.240 mmol) and DMAP (5.85 mg, 0.048 mmol) and stirred at 25 °C for 0.5 h, where the reaction was monitored by LC-MS. The reaction mixture was subjected to rotary evaporation, and purified through medium-pressure liquid chromatography to obtain a white solid as compound **35-2** (22 mg, 0.048 mmol, 99.94% yield). **LCMS** (ESI⁺) m/z: 460.1 [M+H]⁺.

A dried single-neck flask was added with NaH (3.83 mg, 0.096 mmol, 60% purity), sealed, replaced with nitrogen three times, added with 1 mL DMF and cooled in an ice water bath.

The compound **35-2** (22 mg, 0.048 mmol) was dissolved in 0.5 mL DMF dropwise added into the single-neck flask, subjected to temperature keeping, stirred for 20 min, added with CH₃I (20.38 mg, 0.144 mmol) and reacted at 0 °C for 1 h. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated, and purified through medium-pressure liquid chromatography to obtain a white solid as compound **35-3** (20 mg, 0.042 mmol, 88.22% yield). **LCMS** (ESI⁺) m/z: 474.1 [M+H]⁺.

The compound **35-3** (20 mg, 0.042 mmol) and 3 mL MeOH were added into a dried single-neck flask, followed by stirring for dissolution. The reaction mixture was added with potassium carbonate anhydrous (17.51 mg, 0.127 mmol) and stirred at 25 °C for 1 h, where the reaction was monitored by LC-MS. The reaction mixture was filtered, subjected to rotary evaporation, and purified through medium-pressure liquid chromatography to obtain a white solid as compound 35 (10 mg, 0.023 mmol, 54.32% yield, 99.0% purity). **LCMS** (ESI⁺) m/z: 432.1 [M+H]⁺.

**¹H NMR** (400 MHz, DMSO-d6) δ 8.05 (d, J = 4.8 Hz, 1H), 7.93 - 7.85 (m, 1H), 7.53 - 7.35 (m, 3H), 7.17 (d, J = 8.3 Hz, 1H), 7.09 (t, J = 7.4 Hz, 1H), 4.52 (s, 1H), 3.81 (s, 3H), 3.30 - 3.24 (m, 3H), 2.97 (d, J = 2.4 Hz, 1H), 2.79 (d, J = 2.3 Hz, 2H), 1.91 - 1.74 (m, 3H), 1.66 (dtd, J = 17.8, 9.4, 8.2, 3.7 Hz, 1H), 1.46 (s, 1H), 1.22 - 1.06 (m, 2H), 1.01 (s, 1H).

### Example 36 Preparation of compound 36

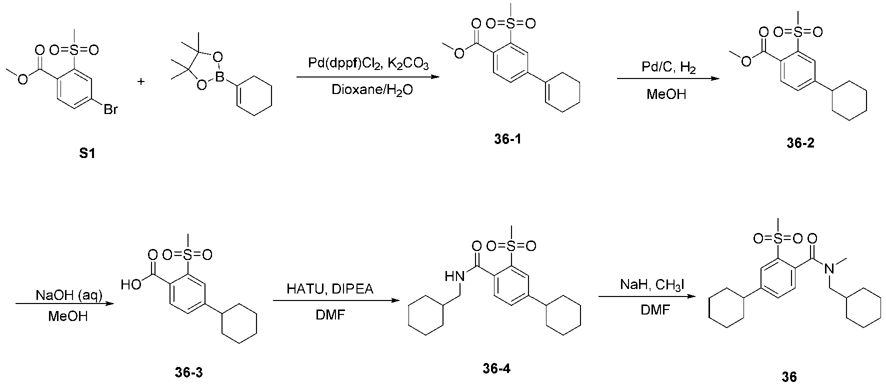

The substrate **S1** (147 mg, 0.5 mmol), cyclohex-1-ene-1-boronic acid pinacol ester (125 mg, 0.6 mmol), Pd(dppf)Cl₂ (36 mg, 0.05 mmol), K₂CO₃ (276 mg, 2 mmol), 4 mL 1,4-dioxane and 1 mL water were added into a dried single-neck flask, sealed, replaced with nitrogen three times, heated to 100 °C and stirred overnight. The reaction mixture was cooled to the room temperature, added with EtOAc and water, and subjected to liquid separation. The resultant organic phase was washed with a saturated NaCl aqueous solution, dried, filtered and concentrated to obtain a crude product. The crude product was purified through column chromatography to obtain a compound **36-1** (102 mg, 0.35 mmol, 70% yield). **LCMS** (ESI⁺) m/z: 295.0 [M+H]⁺.

A dried single-neck flask was added with the compound **36-1** (60 mg, 0.20 mmol), 4 mL MeOH and Pd/C (10%, 22 mg), replaced with hydrogen three times through a hydrogen balloon, and stirred at the room temperature for 16 h, where the reaction was monitored by LC-MS. The reaction mixture was filtered, and the filtrate was subjected to decompression concentration to obtain a compound **36-2** (62 mg, crude). The compound **36-2** can be directly used in the subsequent reaction. **LCMS** (ESI⁺) m/z: 297.0 [M+H]⁺.

The compound **36-2** (62 mg, 0.20 mml) was dissolved in a MeOH (4 mL) and H₂O (1 mL) mixed solution, and added with NaOH (80 mg, 2 mmol), followed by reaction at 70 °C for 2 h, where the reaction was monitored by LC-MS. The reaction mixture was cooled to the room temperature, subjected to pH adjustment to around 5 with a 2N HCl solution, extracted with ethyl acetate and subjected to liquid separation. The resultant organic phase was washed with a saturated NaCl aqueous solution, dried and concentrated to obtain a compound **36-3** (60 mg, crude), and the compound **36-3** can be directly used in the subsequent reaction.

The compound **36-3** (60 mg, 0.2 mmol) and 1.5 mL DMF were added into a dried single-neck flask, followed by stirring for dissolution. The reaction mixture was added with DIPEA (0.35 mL, 2 mmol), cooled in an ice water bath, added with HATU (91 mg, 0.24 mmol), stirred at 0 °C for 5 min, added with cyclohexylmethylamine (34 mg, 0.3 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **36-4** (58 mg, 0.15 mmol, 77% yield). **LCMS** (ESI⁺) m/z: 378.2 [M+H]⁺.

A dried single-neck flask was added with NaH (6 mg, 0.16 mmol), sealed, replaced with nitrogen three times, added with 1 mL DMF and cooled in an ice water bath. The compound **36-4** (20 mg, 0.053 mmol) was dissolved in 0.5 mL DMF, and dropwise added into the single-neck flask. The reaction mixture was held at 0 °C, stirred for 20 min, added with CH₃I (38 mg, 0.159 mmol) and reacted at 0 °C for 1 h. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated, and purified through medium-pressure liquid chromatography to obtain a compound **36** (15 mg, 38 µmol, 72% yield). **LCMS** (ESI⁺) m/z: 392.2 [M+H]⁺.

**¹H NMR** (400 MHz, DMSO-*d*₆) δ 7.78 (s, 1H), 7.70 - 7.62 (m, 1H), 7.39 - 7.28 (m, 1H), 3.55 - 3.40 (m, 0.7H), 3.23 (s, 2.1H), 3.20 (s, 0.9H), 3.12 - 3.01 (m, 0.7H), 2.95 (s, 0.9H), 2.92 - 2.74 (m, 0.6H), 2.72 (s, 2.1H), 2.72 - 2.60 (m, 1H), 1.89 - 1.51 (m, 11H), 1.50 - 1.33 (m, 4H), 1.31 - 0.88 (m, 5.4H), 0.76 - 0.54 (m, 0.6H).

### Example 37 Preparation of compound 37

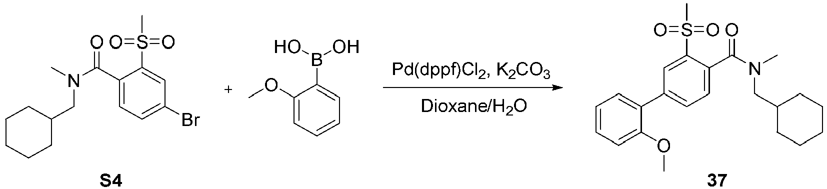

The compound **S4** (30 mg, 77.5 µmol), 2-methoxyphenylboronic acid (14.14 mg, 93 µmol), Pd(dppf)Cl₂ (2.81 mg, 3.87 µmol) and K₂CO₃ (21.39 mg, 155 µmol) were added into a dried single-neck flask, sealed, replaced with nitrogen three times, added with 1 mL dioxane and 0.25 mL H₂O and stirred at 100 °C for 2 h, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **37** (10.96 mg, 26.3 µmol, 30% yield). **LCMS** (ESI⁺) m/z: 416.2 [M+H]⁺.

**¹H NMR** (400 MHz, DMSO-d6) δ 8.06 (dd, J = 5.1, 1.8 Hz, 1H), 7.90 (ddd, J = 7.9, 4.2, 1.8 Hz, 1H), 7.53 - 7.39 (m, 3H), 7.18 (d, J = 8.3 Hz, 1H), 7.10 (t, J = 7.3 Hz, 1H), 3.81 (s, 3H), 3.54 (s, 0.75H), 3.28 (d, J = 14.1 Hz, 3H), 3.09 (s, 0.79H), 2.98 (s, 0.82H), 2.90 (t, J = 7.2 Hz, 0.53H), 2.80 (s, 2.2H), 1.84 - 1.54 (m, 6H), 1.32 - 0.64 (m, 5H).

### Example 38 Preparation of compound 38

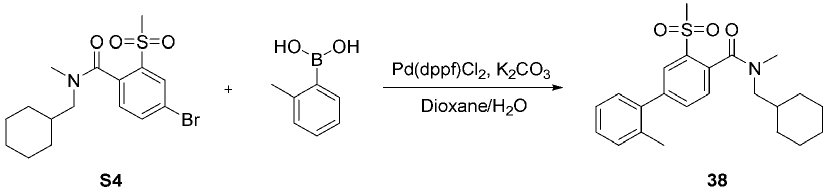

The compound **S4** (30 mg, 77.5 µmol), 2-methylphenylboronic acid (12.65 mg, 93 µmol), Pd(dppf)Cl₂ (2.81 mg, 3.87 µmol) and K₂CO₃ (21.39 mg, 155 µmol) were added into a dried single-neck flask, sealed, replaced with nitrogen three times, added with 1 mL dioxane and 0.25 mL H₂O and stirred at 100 °C for 2 h, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **38** (8.12 mg, 23.9 µmol, 26.2% yield). **LCMS** (ESI⁺) m/z: 400.1 [M+H]⁺.

**¹H NMR** (400 MHz, DMSO-d6) δ 7.86 (t, J = 1.8 Hz, 1H), 7.79 (ddd, J = 7.7, 4.0, 1.8 Hz, 1H), 7.50 (dd, J = 7.8, 5.7 Hz, 1H), 7.34 (ddt, J = 15.7, 4.7, 2.5 Hz, 4H), 3.55 (s, 0.75H), 3.30 (d, J = 13.5 Hz, 3H), 3.11 (s, 0.75H), 2.98 (s, 0.87H), 2.89 (dd, J = 11.4, 7.1 Hz, 0.52H), 2.80 (s, 2.22H), 2.27 (d, J = 2.7 Hz, 3H), 1.81 - 1.53 (m, 6H), 1.30 - 0.65 (m, 5H).

### Example 39 Preparation of compound 39

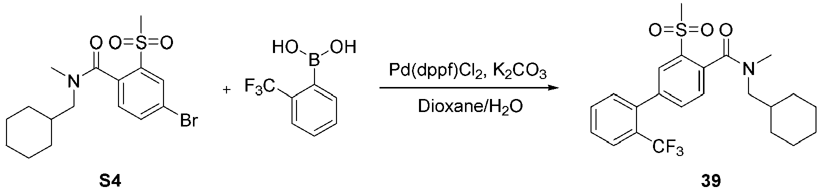

The compound **S4** (15 mg, 38.7 µmol), 2-(trifluoromethyl)phenylboronic acid (8.1 mg, 42.6 µmol), Pd(dppf)Cl₂ (1.4 mg, 1.9 µmol) and K₂CO₃ (10.69 mg, 77.5 µmol) were added into a dried single-neck flask, sealed, replaced with nitrogen three times, added with 1 mL dioxane and 0.25 mL H₂O and stirred at 100 °C for 2 h, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **39** (13.35 mg, 29.4 µmol, 76% yield). **LCMS** (ESI⁺) m/z: 454.1 [M+H]⁺.

**¹H NMR** (400 MHz, DMSO-d6) δ 7.94 - 7.85 (m, 2H), 7.84 - 7.74 (m, 2H), 7.71 (t, J = 7.7 Hz, 1H), 7.61 - 7.51 (m, 2H), 3.55 (s, 0.64H), 3.28 (d, J = 13.6 Hz, 3H), 3.16 - 3.04 (m, 0.67H), 2.99 (s, 1H), 2.95 - 2.82 (m, 0.5H), 2.78 (s, 2.19H), 1.87 - 1.53 (m, 6H), 1.33 - 0.61 (m, 5H).

### Example 40 Preparation of compound 40

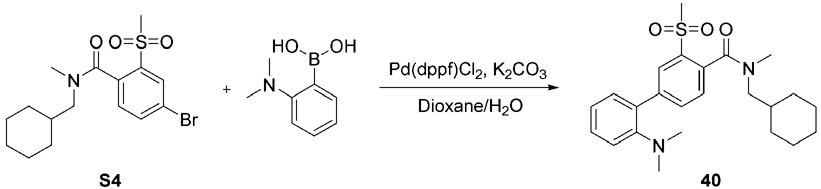

The compound **S4** (15 mg, 38.7 µmol), 2-(dimethylamino)phenylboronic acid (6.39mg, 42.6 µmol), Pd(dppf)Cl₂ (1.4 mg, 1.9 µmol) and K₂CO₃ (10.69 mg, 77.5 µmol) were added into a dried single-neck flask, sealed, replaced with nitrogen three times, added with 1 mL dioxane and 0.25 mL H₂O and stirred at 100 °C for 2 h, where the reaction was monitored by LC-MS. The reaction mixture was quenched with water, followed by extraction with ethyl acetate three times. The resultant organic phases were combined, washed with a saturated NaCl aqueous solution, filtered, concentrated, and purified through medium-pressure liquid chromatography to obtain a white solid as compound **40** (11.83 mg, 27.6 µmol, 71.2% yield). **LCMS** (ESI⁺) m/z: 429.1 [M+H]⁺.

**¹H NMR** (400 MHz, DMSO-d6) δ 8.12 (dd, J = 10.3, 1.7 Hz, 1H), 7.96 (ddd, J = 7.6, 5.4, 1.7 Hz, 1H), 7.48 (d, J = 7.8 Hz, 1H), 7.38 - 7.26 (m, 2H), 7.13 (d, J = 8.1 Hz, 1H), 7.08 (t, J = 7.4 Hz, 1H), 3.54 (d, J = 11.0 Hz, 0.69H), 3.27 (d, J = 13.8 Hz, 3H), 3.07 (d, J = 13.2 Hz, 0.68H), 2.98 (s, 0.94H), 2.95 - 2.83 (m, 0.65H), 2.79 (s, 2.10H), 2.50 (d, J = 3.7 Hz, 3H), 1.85 - 1.52 (m, 6H), 1.31 - 0.62 (m, 5H).

### Example 41 Preparation of compound 41

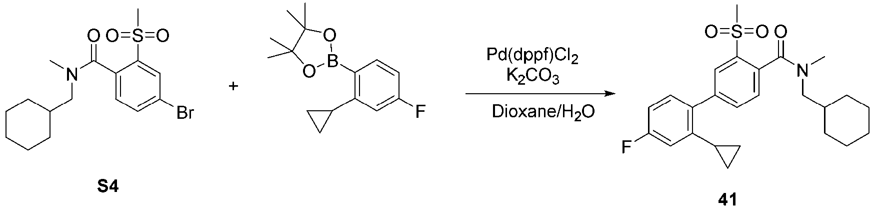

The compound **S4** (20 mg, 0.051 mmol), 2-(2-cyclopropyl-4-fluorophenyl)-4,4,5,5-tetramethyl-1,3,2-dioxaborolane (16 mg, 0.06 mmol), Pd(dppf)Cl₂ (7.3 mg, 0.01 mmol) and K₂CO₃ (14 mg, 0.1 mmol) were added into a dried single-neck flask, sealed, replaced with nitrogen three times, added with 1 mL dioxane and 0.25 mL H₂O and stirred at 100 °C overnight. The reaction mixture was cooled to the room temperature, added with EtOAc and water, and subjected to liquid separation. The resultant organic phase was washed with a saturated NaCl aqueous solution, dried, filtered and concentrated to obtain a crude product. The crude product was purified through medium-pressure liquid chromatography to obtain a white solid as compound **41** (7.6 mg, 17.07 µmol, 33% yield). **LCMS** (ESI⁺) m/z: 444.0 [M+H]⁺.

**¹H NMR** (400 MHz, DMSO-*d*₆) δ 7.98 (d, *J* = 1.9 Hz, 2H), 7.91 (d, *J* = 49.0 Hz, 0H), 7.86 - 7.83 (m, 0H), 7.83 (dd, *J =* 7.8, 1.8 Hz, 1H), 7.53 (d, *J* = 7.8 Hz, 1H), 7.51 (d, *J* = 7.8 Hz, 1H), 7.42 - 7.31 (m, 1H), 7.18 - 7.06 (m, 1H), 6.96 - 6.82 (m, 1H), 3.54 (d, *J* = 10.1 Hz, 1H), 3.31 (s, 4H), 3.27 (s, 1H), 3.11 (d, *J* = 17.1 Hz, 2H), 2.98 (s, 2H), 2.95 - 2.83 (m, 1H), 2.80 (s, 4H), 1.87 - 1.48 (m, 13H), 1.33 - 1.10 (m, 4H), 1.07 - 0.60 (m, 13H).

### Example 42 Preparation of compound 42

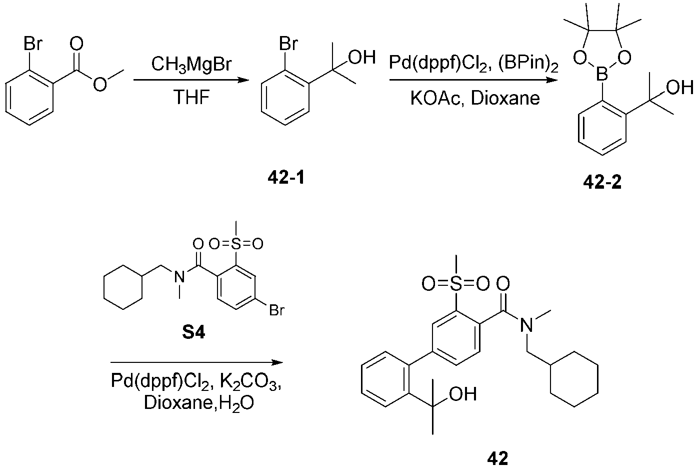

A dried single-neck flask was added with methyl 2-bromobenzoate (500 mg, 2.32 mmol), 10 mL of tetrahydrofuran, cooled to 0 °C under the protection of N₂, added with methylmagnesium bromide (11.6 mL, 11.6 mmol, 1M in THF) and reacted at the room temperature for 16, where the reaction was monitored by TLC. The reaction mixture was added with a saturated ammonium chloride solution and extracted with ethyl acetate three times. The resultant organic phases were combined, dried over anhydrous sodium sulfate, and filtered, and a filtrate was collected, concentrated, and purified through column chromatography to obtain a compound **42-1** (200 mg, 0.93 mmol). **LCMS** (ESI⁺) m/z: 215.1 [M+H]⁺.

The compound **42-1** (50 mg, 0.23 mmol), bis(pinacolato)diboron (118 mg, 0.46 mmol), Pd(dppf)Cl₂ (17 mg, 0.023 mmol) and potassium acetate (68 mg, 0.69 mmol) were added into a dried single-neck flask, and dissolved in 1,4-dioxane, and reacted at 100 °C under the protection of N₂ for 2 h, where the reaction completion was monitored by LC-MS. The reaction mixture was cooled to the room temperature, filtered, and subjected to decompression concentration to obtain a compound **42-2** (80 mg, crude). The compound **42-2** can be directly used in the subsequent reaction. **LCMS** (ESI⁺) m/z: 263.2 [M+H]⁺.

The compound **42-2** (80 mg, 0.23 mmol), the compound **S4** (50 mg, 0.13 mmol), Pd(dppf)Cl₂ (9.4 mg, 0.013 mmol) and K₂CO₃ (53.3 mg, 0.39 mmol) were added into a dried single-neck flask, dissolved in 1.5 mL 1,4-dioxane and 0.5 mL H₂O, and reacted at 100 °C under the protection of N₂ for 2 h, where the reaction was monitored by LC-MS. The reaction mixture was cooled to the room temperature, filtered, and subjected to decompression concentration, and the remnant was purified through high performance liquid chromatography (basic) to obtain a white solid as compound **42** (12.9 mg, 0.029 mmol, 22.3% yield). **LCMS** (ESI⁺) m/z: 444.1 [M+H]⁺. **HPLC** method B: R_{T} = 8.37 min, purity > 99.1%. **¹H NMR** (400 MHz, DMSO-*d*₆) δ 7.84 - 7.76 (m, 1H), 7.75 - 7.62 (m, 2H), 7.62 - 7.44 (m, 1.2H), 7.44 - 7.36 (m, 1.5H), 7.34 - 7.24 (m, 0.8H), 7.11 - 7.01 (m, 0.7H), 4.91 - 4.86 (m, 1H), 3.56 (s, 0.7H), 3.30 - 3.20 (m, 3.2H), 3.09 (s, 0.7H), 3.01 - 2.72 (m, 3.7H), 2.48 - 2.22 (m, 1H), 1.87 - 1.51 (m, 6H), 1.40 - 1.15 (m, 7H), 1.15 - 0.53 (m, 3H).

### Example 43 Preparation of compound 43

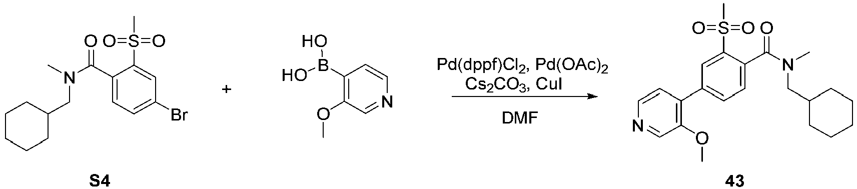

The compound **S4** (31 mg, 0.08 mmol), 3-methoxy-4-pyridineboronic acid (24.4 mg, 0.16 mmol), Pd(dppf)Cl₂ (5.8 mg, 8 µmol), Cs₂CO₃ (52 mg, 0.16 mmol), and CuI (15.2 mg, 0.08 mmol) were added into a dried single-neck flask. The reaction mixture was added with 1.5 mL DMF, sealed, replaced with nitrogen three times, stirred at 100 °C for 2 h, cooled to the room temperature, added with EtOAc and water, and subjected to liquid separation. The resultant organic phase was washed with a saturated NaCl aqueous solution, dried, filtered and concentrated to obtain a crude product. The crude product was purified through medium-pressure liquid chromatography to obtain a white solid as compound 43 (9.3 mg, 22.3 µmol, 28% yield). **LCMS** (ESI⁺) m/z: 417.1 [M+H]⁺.

**¹H NMR** (400 MHz, DMSO-*d₆*) δ 8.92 - 8.21 (m, 2H), 8.14 (d, *J =* 1.7 Hz, 1H), 8.04 - 7.92 (m, 1H), 7.62 - 7.39 (m, 2H), 3.95 (s, 3H), 3.58 -3.47 (m, 0.7H), 3.30 (s, 2.1H), 3.27 (s, 0.9H) 3.14 - 3.04 (m, 0.7H), 2.97 (s, 0.9H), 2.95 - 2.81 (m, 0.6H), 2.79 (s, 2.1H), 1.91 - 1.48 (m, 6.3H), 1.31 - 1.10(m, 2.4H), 1.10 - 0.87 (m, 1.7H), 0.77 - 0.61 (m, 0.6H).

### Example 44 Preparation of compound 44

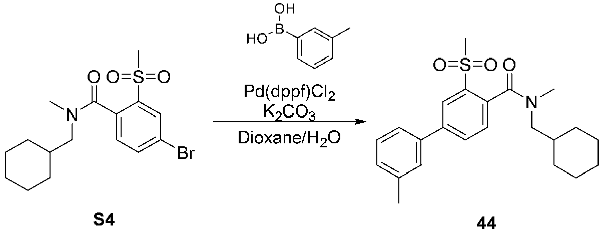

Under the protection of N₂, the compound **S4** (25 mg, 64.38 µmol), 3-methylphenylboronic acid (10.50 mg, 77.26 µmol), Pd(dppf)Cl₂ (2.34 mg, 3.22 µmol), K₂CO₃ (17.80 mg, 128.76 µmol), and 1mL dioxane/H₂O (v:v=4:1) were added into a microwave tube, and stirred at 100 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and subjected to decompression concentration, and the remnant was purified through medium-pressure liquid chromatography to obtain a white solid as compound **44** (13.3 mg, 33.29 µmol, 51.71% yield, 100% purity). **LCMS** (ESI⁺) m/z: 400.2 [M+H]⁺.

**¹H NMR** (400 MHz, DMSO-*d*₆) δ 8.17 - 8.12 (m, 1H), 8.10 - 8.03 (m, 1H), 7.62 - 7.49 (m, 3H), 7.45 - 7.40 (m, 1H), 7.28 (d, *J =* 7.6 Hz, 1H), 3.60 - 3.50 (m, 1H), 3.30 (d, *J =* 12.6 Hz, 3H), 3.15 - 3.04 (m, 1H), 2.97 (s, 1H), 2.94 - 2.84 (m, 1H), 2.78 (s, 2H), 2.41 (s, 3H), 1.85 - 1.70 (m, 4H), 1.67 - 1.55 (m, 2H), 1.29 - 1.06 (m, 4H).

### Example 45 Preparation of compound 45

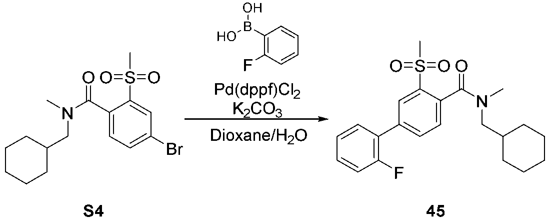

Under the protection of N₂, the substrate **S3** (25 mg, 64.38 µmol), 2-fluorophenylboronic acid (10.81 mg, 77.26 µmol), Pd(dppf)Cl₂ (2.34 mg, 3.22 µmol), K₂CO₃ (17.80 mg, 128.76 µmol), and 1mL dioxane/H2O (v:v=4:1) were added into a microwave tube, and stirred at 100 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and subjected to decompression concentration, and the remnant was purified through medium-pressure liquid chromatography to obtain a white solid as compound **45** (17 mg, 41.58 µmol, 64.59% yield, 98.7% purity). **LCMS** (ESI⁺) m/z: 404.2 [M+H]⁺.

**¹H NMR** (400 MHz, DMSO-*d*₆) δ 8.10 (s, 1H), 8.02 - 7.95 (m, 1H), 7.74 - 7.64 (m, 1H), 7.59 - 7.48 (m, 2H), 7.44 - 7.35 (m, 2H), 3.62 - 3.45 (m, 1H), 3.30 (d, *J* = 14.0 Hz, 3H), 3.20 - 3.00 (m, 1H), 2.98 (s, 1H), 2.96 - 2.82 (m, 1H), 2.79 (s, 2H), 1.91 - 1.69 (m, 4H), 1.67 - 1.50 (m, 2H), 1.29 - 0.99 (m, 4H).

### Example 46 Preparation of compound 46

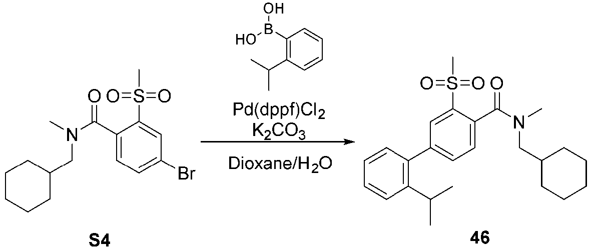

Under the protection of N₂, the compound **S4** (25 mg, 64.38 µmol), 2-isopropylphenylboronic acid (12.67 mg, 77.26 µmol), Pd(dppf)Cl₂ (2.34 mg, 3.22 µmol), K₂CO₃ (17.80 mg, 128.76 µmol), and 1 mL dioxane/H2O (v:v=4:1) were added into a microwave tube, and stirred at 100 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and subjected to decompression concentration, and the remnant was purified through medium-pressure liquid chromatography to obtain a white solid as compound **46** (17.6 mg, 41.16 µmol, 63.93% yield, 100% purity). **LCMS** (ESI⁺) m/z: 428.2 [M+H]⁺.

**¹H NMR** (400 MHz, DMSO-*d*₆) δ 7.89 - 7.78 (m, 1H), 7.79 - 7.68 (m, 1H), 7.62 - 7.47 (m, 2H), 7.46 - 7.36 (m, 1H), 7.36 - 7.17 (m, 2H), 3.56 - 3.52 (m, 1H), 3.29 (d, *J =* 14.1 Hz, 3H), 3.14 - 3.04 (m, 1H), 2.99 (s, 1H), 2.96 - 2.84 (m, 2H), 2.81 (s, 2H), 1.85 - 1.69 (m, 4H), 1.68 - 1.57 (m, 2H), 1.27 - 1.12 (m, 10H).

### Example 47 Preparation of compound 47

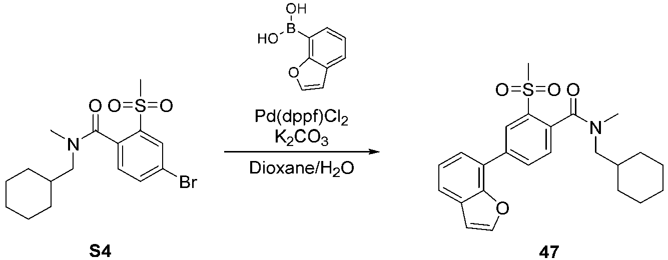

Under the protection of N₂, the compound **S4** (15 mg, 38.63 µmol), benzofuran-4-ylboronic acid (7.51 mg, 46.35 µmol), Pd(dppf)Cl₂ (1.40 mg, 1.93 µmol), K₂CO₃ (10.68 mg, 77.26 µmol), and 1 mL dioxane/H₂O (v:v=4:1) were added into a microwave tube, and stirred at 100 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and subjected to decompression concentration, and the remnant was purified through medium-pressure liquid chromatography to obtain a white solid as compound **47** (10.0 mg, 23.50 µmol, 60.84% yield, 100% purity). **LCMS** (ESI⁺) m/z: 426.3 [M+H]⁺.

### Example 48 Preparation of compound 48

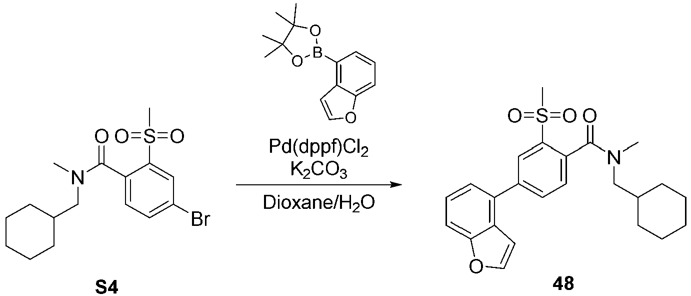

Under the protection of N₂, the compound **S4** (15 mg, 38.63 µmol), benzofuran-4-boronic acid pinacol ester (11.31 mg, 46.35 µmol), Pd(dppf)Cl₂ (1.40 mg, 1.93 µmol), K₂CO₃ (10.68 mg, 77.26 µmol), and 1mL dioxane/H₂O (v:v=4:1) were added into a microwave tube, and stirred at 100 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and subjected to decompression concentration, and the remnant was purified through medium-pressure liquid chromatography to obtain a white solid as compound **48** (9.26 mg, 21.76 µmol, 56.33% yield, 94.5% purity). **LCMS** (ESI⁺) m/z: 426.3 [M+H]⁺.

**¹H NMR** (400 MHz, DMSO-*d*₆) δ 8.23 - 8.13 (m, 2H), 8.10 - 8.04 (m, 1H), 7.72 (d, *J =* 7.9 Hz, 1H), 7.63 - 7.56 (m, 1H), 7.55 - 7.42 (m, 2H), 7.20 - 7.14 (m, 1H), 3.65 - 3.48 (m, 1H), 3.37 (s, 3H), 3.17 - 3.02 (m, 1H), 2.99 (s, 1H), 2.82 (s, 2H), 1.86 - 1.70 (m, 4H), 1.68 - 1.55 (m, 2H), 1.30 - 1.13 (m, 3H), 1.11 - 0.81 (m, 2H).

### Example 49 Preparation of compound 49

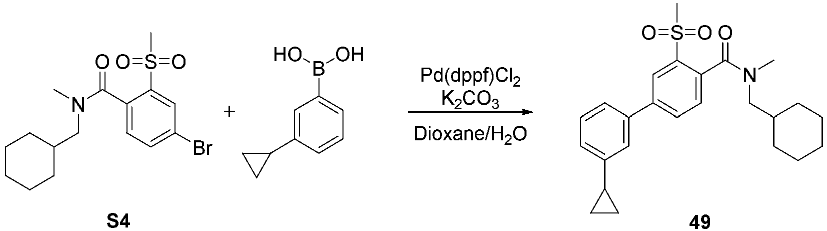

Under the protection of N₂, the compound **S4** (15 mg, 38.63 µmol), 2-isopropylphenylboronic acid (7.51 mg, 46.35 µmol), Pd(dppf)Cl₂ (1.40 mg, 1.93 µmol), K₂CO₃ (10.68 mg, 77.26 µmol), and 1 mL dioxane/H₂O (v:v=4:1) were added into a microwave tube, and stirred at 100 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and subjected to decompression concentration, and the remnant was purified through medium-pressure liquid chromatography to obtain a white solid as compound **49** (6.4 mg, 14.66 µmol, 37.96% yield, 97.5% purity). **LCMS** (ESI⁺) m/z: 426.6 [M+H]⁺.

**¹H NMR** (600 MHz, DMSO-*d*₆) δ 8.07 (d, *J* = 3.6 Hz, 1H), 8.03 - 7.96 (m, 1H), 7.48 - 7.41 (m, 2H), 7.41 - 7.36 (m, 1H), 7.36 - 7.31 (m, 1H), 7.08 (d, *J* = 7.6 Hz, 1H), 3.46 (t, *J =* 10.2 Hz, 1H), 3.24 (d, *J =* 19.1 Hz, 3H), 3.02 (t, 1H), 2.90 (s, 1H), 2.71 (s, 2H), 2.01 - 1.94 (m, 1H), 1.76 - 1.68 (m, 2H), 1.67 - 1.61 (m, 2H), 1.60 - 1.48 (m, 2H), 1.23 - 1.06 (m, 3H), 1.02 - 0.85 (m, 4H), 0.74 - 0.68 (m, 2H).

### Example 50 Preparation of compound 50

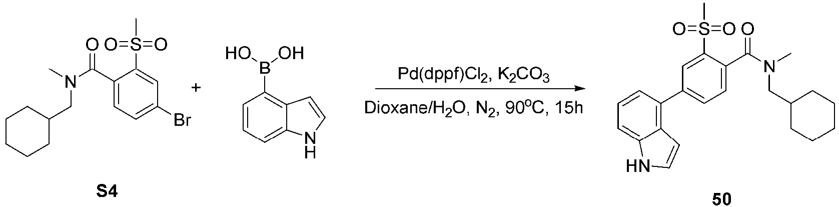

Under the protection of N₂, the compound **S4** (40 mg, 0.103 mmol), 1-indol-4-bromophenylboronic acid (19.90 mg, 0.124 mmol), Pd(dppf)Cl₂ (7.52 mg, 0.010 mmol), K₂CO₃ (42.71 mg, 0.309 mmol), and 1mL dioxane/H₂O (v:v=4:1) were added into a microwave tube, and stirred at 90 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and purified through medium-pressure liquid chromatography to obtain a white solid as compound **50** (16 mg, 0.036 mmol, 34.68% yield, 94.8% purity). **LCMS** (ESI⁺) m/z: 425.2 [M+H]⁺.

**¹H NMR** (400 MHz, DMSO-d6) δ 11.19 (s, 1H), 8.13 (d, J = 1.7 Hz, 1H), 8.01 (dt, J = 7.8, 2.1 Hz, 1H), 7.64 (dd, J = 7.7, 1.2 Hz, 1H), 7.56 (dd, J = 11.4, 7.8 Hz, 1H), 7.41 (dt, J = 5.6, 2.7 Hz, 1H), 7.26 - 7.09 (m, 2H), 6.58 (dt, J = 3.2, 1.7 Hz, 1H), 3.55 (d, J = 7.5 Hz, 1H), 3.37 (s, 2H), 3.34 (s, 1H), 3.10 (dd, J = 12.8, 6.0 Hz, 1H), 2.99 (s, 1H), 2.84 (s, 2H), 1.80 (d, J = 6.8 Hz, 2H), 1.73 (dd, J = 13.5, 3.8 Hz, 2H), 1.68 - 1.52 (m, 2H), 1.23 (d, J = 9.7 Hz, 3H), 1.01 (s, 2H).

### Example 51 Preparation of compound 51

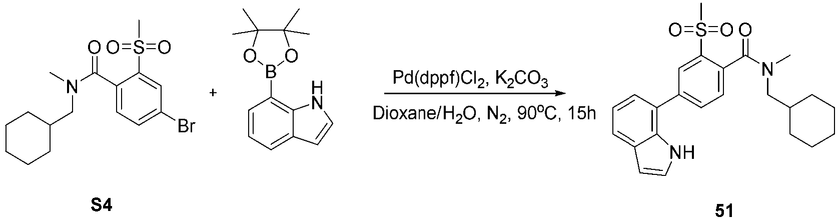

Under the protection of N₂, the compound **S4** (20 mg, 0.052 mmol), 7-indoleboronic acid pinacol ester (15.03 mg, 0.062 mmol), K₂CO₃ (21.36 mg, 0.155 mmol), Pd(dppf)Cl₂ (3.76 mg, 0.005 mmol), and 2 mL dioxane/H2O (v:v=3:1) were added into a microwave tube, and stirred at 90 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and purified through medium-pressure liquid chromatography to obtain a white solid as compound **51** (16 mg, 0.038 mmol, 73.17% yield, 100% purity). **LCMS** (ESI⁺) m/z: 425.1 [M+H]⁺.

**¹H NMR** (400 MHz, DMSO-d6) δ 11.19 (s, 1H), 8.13 (d, J = 1.7 Hz, 1H), 8.01 (dt, J = 7.8, 2.1 Hz, 1H), 7.64 (dd, J = 7.7, 1.2 Hz, 1H), 7.56 (dd, J = 11.4, 7.8 Hz, 1H), 7.41 (q, J = 2.8, 2.3 Hz, 1H), 7.26 - 7.09 (m, 2H), 6.58 (dt, J = 3.2, 1.7 Hz, 1H), 3.55 (d, J = 7.5 Hz, 1H), 3.37 (s, 3H), 3.34 (s, 1H), 3.10 (dd, J = 13.0, 5.9 Hz, 1H), 2.99 (s, 1H), 2.84 (s, 2H), 1.88 - 1.77 (m, 2H), 1.73 (dd, J = 13.5, 3.8 Hz, 2H), 1.63 (d, J = 22.6 Hz, 2H), 1.33 - 1.12 (m, 3H), 1.01 (s, 2H).

### Example 52 Preparation of compound 52

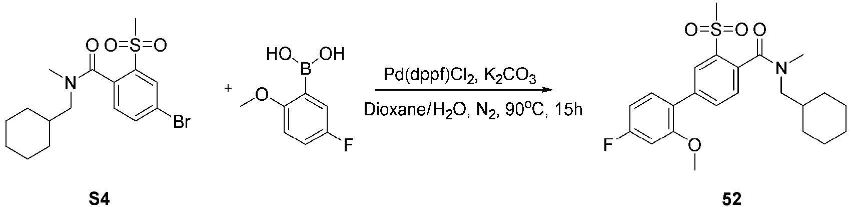

Under the protection of N₂, the compound **S4** (50 mg, 0.13 mmol), 5-fluoro-2-methoxyphenylboronic acid (24.3 mg, 0.143 mmol), K₂CO₃ (53.8 mg, 0.39 mmol), Pd(dppf)Cl₂ (11.2 mg, 0.013 mmol), and 2 mL dioxane/H₂O (v:v=3:1) were added into a microwave tube, and stirred at 90 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and purified through medium-pressure liquid chromatography to obtain a white solid as compound **52** (22.6 mg, 0.052 mmol, 40 % yield, 94.9 % purity). **LCMS** (ESI⁺) m/z: 434.1 [M+H]⁺.

**¹H NMR** (400 MHz, DMSO-d6) δ 8.00 (dd, J = 3.5, 1.8 Hz, 1H), 7.86 (dd, J = 7.9, 1.8 Hz, 1H), 7.44 (ddt, J = 8.3, 6.8, 3.7 Hz, 2H), 7.04 (dd, J = 11.4, 2.5 Hz, 1H), 6.91 (tdd, J = 8.4, 2.5, 1.1 Hz, 1H), 3.79 (d, J = 1.3 Hz, 3H), 3.47 (s, 1H), 3.21 (s, 1H), 3.08 (d, J = 9.1 Hz, 1H), 2.95 (s, 1H), 2.76 (s, 2H), 1.85 - 1.48 (m, 7H), 1.19 - 1.09 (m, 3H), 0.95 (d, J = 12.1 Hz, 2H), 0.86 - 0.59 (m, 1H).

### Example 53 Preparation of compound 53

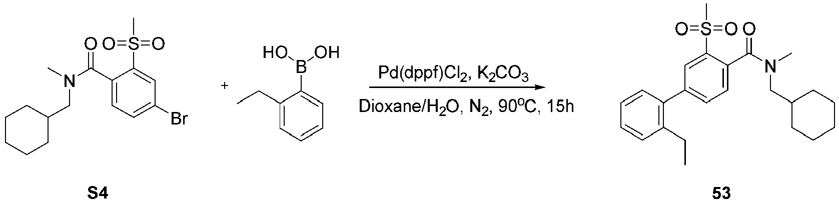

Under the protection of N₂, the compound **S4** (10 mg, 0.026 mmol), 2-ethylphenylboronic acid (4.3 mg, 0.028 mmol), K₂CO₃ (10.8 mg, 0.078 mmol), Pd(dppf)Cl₂ (2.6 mg, 0.003 mmol), and 2 mL dioxane/H₂O (v:v=3:1) were added into a microwave tube, and stirred at 90 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and purified through medium-pressure liquid chromatography to obtain a white solid as compound **53** (3.43 mg, 0.008 mmol, 31.8% yield, 97.5% purity).

**LCMS** (ESI⁺) m/z: 414.2 [M+H]⁺.

**¹H NMR** (400 MHz, DMSO-d6) δ 7.84 (d, J = 1.8 Hz, 1H), 7.76 (ddd, J = 7.6, 5.5, 1.9 Hz, 1H), 7.51 (dd, J = 7.8, 3.1 Hz, 1H), 7.43 - 7.37 (m, 2H), 7.36 - 7.24 (m, 2H), 3.55 (s, 1H), 3.29 (d, J = 8.2 Hz, 3H), 3.10 (s, 1H), 2.99 (s, 1H), 2.80 (s, 2H), 1.86 - 1.53 (m, 7H), 1.33 - 1.12 (m, 4H), 1.06 (dt, J = 17.9, 7.5 Hz, 5H).

### Example 54 Preparation of compound 54

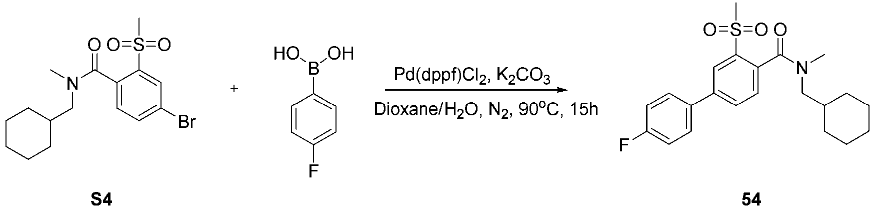

Under the protection of N₂, the compound S4 (10 mg, 0.026 mmol), 4-fluorophenylboronic acid (3.9 mg, 0.028 mmol), K₂CO₃ (10.8 mg, 0.078 mmol), Pd(dppf)Cl₂ (2.6 mg, 0.003 mmol), and 2 mL dioxane/H₂O (v:v=3:1) were added into a microwave tube, and stirred at 90 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and purified through medium-pressure liquid chromatography to obtain a white solid as compound **54** (5.12 mg, 0.012 mmol, 48.8% yield, 99.3% purity).

**LCMS** (ESI⁺) m/z: 404.1 [M+H]⁺.

**¹H NMR** (600 MHz, DMSO-d6) δ 8.14 (dd, J = 3.6, 1.8 Hz, 1H), 8.07 (ddd, J = 14.8, 7.9, 2.0 Hz, 1H), 7.84 (ddd, J = 16.5, 8.7, 5.5 Hz, 2H), 7.52 (dd, J = 12.7, 7.9 Hz, 1H), 7.38 (td, J = 8.8, 1.9 Hz, 2H), 3.53 (d, J = 10.3 Hz, 1H), 3.32 (s, 2H), 3.29 (s, 1H), 3.09 (s, 1H), 2.98 (s, 1H), 2.79 (s, 2H), 1.84 - 1.54 (m, 7H), 1.21 - 1.12 (m, 2H), 1.00 (d, J = 10.6 Hz, 2H).

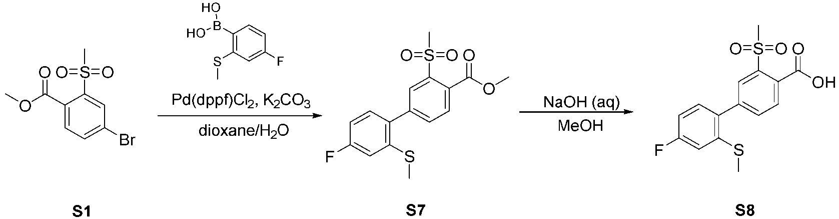

### Synthesis of compound S7

The substrate **S1** (150 mg, 0.51 mmol), 4-fluoro-2-(methylthio)phenylboronic acid (112 mg, 0.6 mmol), Pd(dppf)Cl₂ (36 mg, 0.05 mmol), K₂CO₃ (276 mg, 2 mmol), 4 mL 1,4-dioxane and 1 mL water were added into a dried single-neck flask, sealed, replaced with nitrogen three times, heated to 100 °C and stirred overnight. The reaction mixture was cooled to the room temperature, added with EtOAc and water, and subjected to liquid separation. The resultant organic phase was washed with a saturated NaCl aqueous solution, dried, filtered and concentrated to obtain a crude product. The crude product was purified through column chromatography to obtain a compound **S7** (175 mg, 0.49 mmol, 96% yield).

### Synthesis of compound S8

The compound **S7** (175 mg, 0.49 mmol) was dissolved in the MeOH/H2O mixed solution (v:v=4:1, 5mL), added with NaOH (200 mg, 5 mmol), and reacted at 70 °C for 2 h, where the reaction was monitored by LC-MS. The reaction mixture was cooled to the room temperature, subjected to pH adjustment to around 5 with a 2N HCl solution, extraction with ethyl acetate and liquid separation. The resultant organic phase was washed with a saturated NaCl aqueous solution, dried, concentrated, and purified through medium-pressure liquid chromatography to obtain a white solid as compound **S8** (160 mg, 0.47 mmol, 96% yield).

### Example 55 Preparation of compound 55

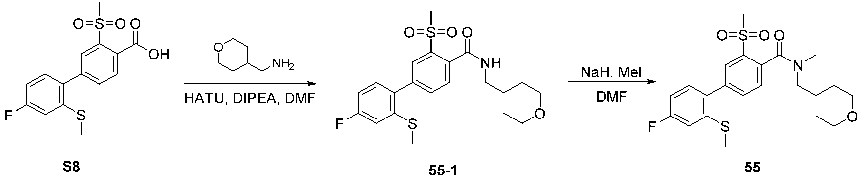

The compound **S8** (30.0 mg, 0.088 mmol) was added into a dried single-neck flask, dissolved in 1.0 mL DMF, and cooled to 0 °C. The reaction mixture was added with DIPEA (153.0 µL, 0.88 mmol), reacted for 5 min, added with HATU (40.1 mg, 0.106 mmol), reacted for 10 min, added with 4-(aminomethyl)tetrahydropyran (12.2 mg, 0.106 mmol) and reacted for 0.5 h, where the reaction was monitored by LC-MS. The reaction mixture was subjected to vacuum concentration, and the remnant was purified through high performance liquid chromatography (basic) to obtain a compound **55-1** (28.0 mg, 0.064 mmol). **LCMS** (ESI⁺) m/z: 438.1 [M+H]⁺.

A dried single-neck flask was added with NaH (5 mg, 0.128 mmol), cooled to 0 °C under the protection of N₂, added with 1 mL of DMF, and reacted for 5 min. The compound **55-1** (28.0 mg, 0.064 mmol) was dissolved in 0.5 mL DMF, added into the reaction mixture and stirred for 0.5 h. The reaction mixture was added with methyl iodide (8.2 mg, 0.128 mmol) and reacted at the room temperature for 2 h, where the reaction was monitored by LC-MS. The reaction mixture was subjected to vacuum concentration, and the remnant was purified through high performance liquid chromatography (basic) to obtain a compound **55** (8.4 mg, 0.019 mmol). **LCMS** (ESI⁺) m/z: 452.2 [M+H]⁺. HPLC method B: R_{T} = 7.53 min, purity > 98.8 %.

**¹H NMR** (600 MHz, DMSO-*d*₆) δ 7.92 (d, *J* = 1.8 Hz, 1H), 7.83 - 7.75 (m, 1H), 7.57 - 7.53 (m, 1H), 7.40 - 7.35 (m, 1H), 7.25 - 7.23 (m, 1H), 7.13 - 7.10 (m, 1H), 3.92 - 3.73 (m, 2H), 3.55 - 3.44 (m, 0.7H), 3.32 - 3.28 (m, 3.6H), 3.28 - 3.20 (m, 2H), 3.03 - 2.78 (m, 3.7H), 2.48 - 2.41 (m, 3H), 2.08 - 1.85 (m, 1H), 1.75 - 1.42 (m, 2H), 1.28 - 0.95 (m, 2H).

### Example 56 Preparation of compound 56

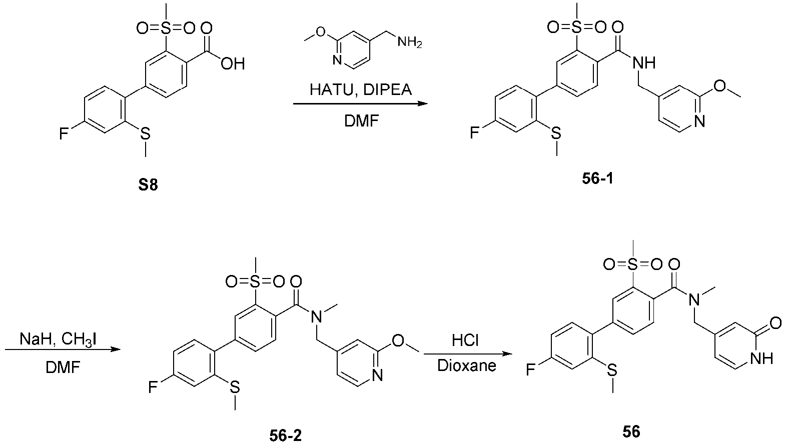

The compound **S8** (23 mg, 67.6 µmol) was added into a dried single-neck flask, and dissolved in 1.5 mL DMF under stirring. The reaction mixture was added with DIPEA (59 µL, 0.34 mmol), cooled in an ice water bath, added with HATU (31 mg, 81 µmol), reacted at 0°C under stirring for 5 min, added with 2-methoxy-4-(aminomethyl)pyridine (11.2 mg, 81 µmol) and reacted at 0°C for 20 minutes, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **56-1** (25 mg, 54.3 µmol, 80% yield).

A dried single-neck flask was added with NaH (8 mg, 0.2 mmol), sealed, replaced with nitrogen three times, added with 1 mL DMF and cooled in an ice water bath. The compound **56-1** (25 mg, 54.3 µmol) was dissolved in 0.5 mL DMF, dropwise added into the single-neck flask, subjected to temperature keeping, stirred for 20 min, added with CH₃I (28 mg, 0.2 mmol) and reacted at 0 °C for 1 h. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated, and purified through medium-pressure liquid chromatography to obtain a compound **56-2** (22 mg, 46.3 µmol, 85% yield). **LCMS** (ESI⁺) m/z: 475.0 [M+H]⁺. The compound **56-2** (17 mg, 36 µmol) was added into the 25 mL single-neck flask, dissolved in 2 mL 1,4-dioxane and stirred, added with 1 mL of concentrated hydrochloric acid, heated and stirred at 90°C for 16 h, where the reaction was monitored by LC-MS. The reaction mixture was adjusted to pH 8 with saturated NaHCO₃, extracted with ethyl acetate, washed with a saturated NaCl aqueous solution, dried over anhydrous sodium sulfate, and filtered, and a filtrate was collected, concentrated, and purified through medium-pressure liquid chromatography to obtain a white solid as compound **56** (10 mg, 21.7 µmol, 60% yield).

**LCMS** (ESI⁺) m/z: 461.1 [M+H]⁺.

**¹H NMR** (600 MHz, DMSO-*d*₆) δ 11.49 (s, 1H), 7.96 (d, *J* = 1.8 Hz, 0.7H), 7.94 (d, *J* = 1.8 Hz, 0.3H), 7.83 (dd, *J* = 7.8, 1.8 Hz, 0.7H), 7.76 (dd, *J* = 7.8, 1.8 Hz, 0.3H), 7.65 (d, *J* = 7.8 Hz, 0.7H), 7.57 (d, *J =* 7.8 Hz, 0.3H), 7.40 - 7.31 (m, 2H), 7.27 - 7.20 (m, 1H), 7.15 - 7.07 (m, 1H), 6.37 (d, *J* = 1.6 Hz, 0.7H), 6.27 - 6.21 (m, 1H), 6.15 (dd, *J* = 6.8, 1.8 Hz, 0.3H), 4.54 (s, 1.4H), 4.14 (d, *J =* 5.7 Hz, 0.6H), 3.33 (s, 3H), 2.93 (s, 0.9H), 2.77 (s, 2.1H), 2.47 (s, 2.1H), 2.45 (s, 0.9H).

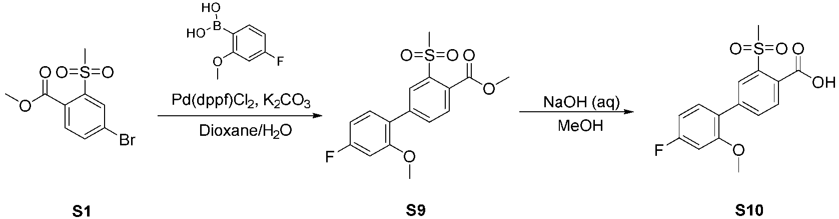

### Synthesis of compound S9

The substrate **S1** (200 mg, 0.68 mmol), 4-fluoro-2-methoxyphenylboronic acid (140 mg, 0.82 mmol), Pd(dppf)Cl₂ (58 mg, 0.068 mmol), K₂CO₃ (282 mg, 2.04 mmol) 4 mL 1,4-dioxane and 1 mL water were added into a dried single-neck flask, sealed, replaced with nitrogen three times, heated to 100 °C and stirred overnight. The reaction mixture was cooled to the room temperature, added with EtOAc and water, and subjected to liquid separation. The resultant organic phase was washed with a saturated NaCl aqueous solution, dried, filtered and concentrated to obtain a crude product. The crude product was purified through column chromatography to obtain a compound **S9** (153 mg, 0.45 mmol, 66% yield).

### Synthesis of compound S10

The compound **S9** (153 mg, 0.45 mmol) was dissolved in the MeOH/H2O mixed solution (v:v=4:1, 5 mL), added with NaOH (200 mg, 5 mmol), and reacted at 70 °C for 2 h, where the reaction was monitored by LC-MS. The reaction mixture was cooled to the room temperature, subjected to pH adjustment to around 5 with a 2N HCl solution, extraction with ethyl acetate and subjected to liquid separation. The resultant organic phase was washed with a saturated NaCl aqueous solution, dried, concentrated, and purified through medium-pressure liquid chromatography to obtain a white solid as compound **S10** (142.7 mg, 0.44 mmol, 97% yield).

### Example 57 Preparation of compound 57

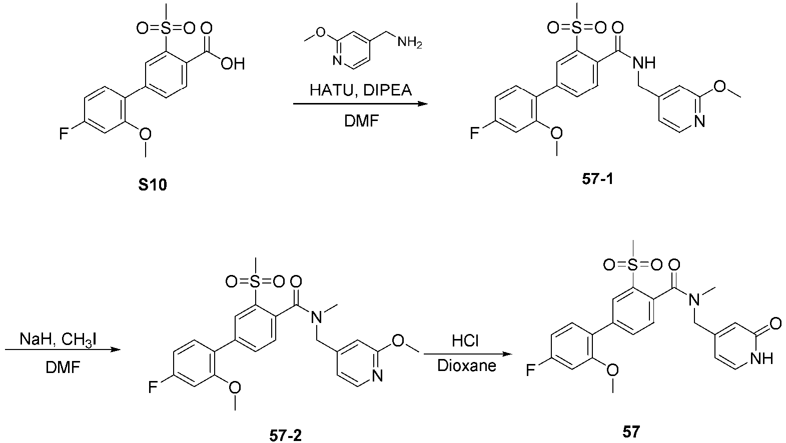

The compound **S10** (50 mg, 0.154 mmol) and 1.5 DMF were added into a dried single-neck flask, followed by stirring for dissolution. The reaction mixture was added with DIPEA (118 µL, 0.68 mmol) cooled in an ice water bath, added with HATU (70.4 mg, 0.185 mmol), stirred at 0 °C for 5 min, added with 2-methoxy-4-(aminomethyl)pyridine (25.5 mg, 0.185 mmol) and reacted at 0 °C for 20 min, where the reaction was monitored by LC-MS. The reaction mixture was quenched with water, followed by extraction with ethyl acetate three times. The resultant organic phases were combined, washed with a saturated NaCl aqueous solution, dried over anhydrous sodium sulfate, and purified through medium-pressure liquid chromatography to obtain a white solid as compound **57-1** (35 mg, 78.7 µmol, 51% yield).

**LCMS** (ESI⁺) m/z: 445.5 [M+H]⁺.

A dried single-neck flask was added with NaH (16 mg, 0.4 mmol), sealed, replaced with nitrogen three times, added with 1 mL of DMF and cooled in an ice water bath. The compound **57-1** (25 mg, 54.3 µmol) was dissolved in 0.5 mL DMF, dropwise added into the single-neck flask, subjected to temperature keeping, stirred for 20 min, added with CH₃I (56 mg, 0.4 mmol) and reacted at 0 °C for 1 h. The reaction mixture was quenched with water, followed by extraction with ethyl acetate. The resultant organic phase was washed with a saturated NaCl aqueous solution, dried over anhydrous sodium sulfate, and filtered, a filtrate was collected, concentrated, and purified through medium-pressure liquid chromatography to obtain a compound **57-2** (18.7 mg, 40.8 µmol, 45% yield). **LCMS** (ESI⁺) m/z: 459.4 [M+H]⁺.

The compound **57-2** (18.7 mg, 40.8 µmol) was added into the 25 mL single-neck flask, dissolved in 2 mL of 1,4-dioxane through stirring, added with 1 mL of concentrated hydrochloric acid, heated and stirred at 90°C for 16 h, where the reaction was monitored by LC-MS. The reaction mixture was subjected to pH adjustment to around 8 with saturated NaHCO₃, extracted with ethyl acetate, washed with a saturated NaCl aqueous solution, dried over anhydrous sodium sulfate, and purified through medium-pressure liquid chromatography to obtain a white solid as compound **57** (5.12 mg, 11.5 µmol, 28% yield).

**LCMS** (ESI⁺) m/z: 445.1 [M+H]⁺.

**¹H NMR** (400 MHz, DMSO-d6) δ 11.51 (s, 1H), 8.04 (dd, J = 5.2, 1.8 Hz, 1H), 7.88 (ddd, J = 28.9, 7.9, 1.8 Hz, 1H), 7.64 - 7.43 (m, 2H), 7.35 (dd, J = 14.4, 6.7 Hz, 1H), 7.10 (ddd, J = 10.7, 8.0, 2.5 Hz, 1H), 7.03 - 6.87 (m, 1H), 6.40 - 6.12 (m, 2H), 4.53 (s, 1H), 4.24 - 4.12 (m, 1H), 3.83 (d, J = 9.2 Hz, 3H), 3.33 (s, 3H), 2.93 (s, 1H), 2.77 (s, 2H).

### Example 58 Preparation of compound 58

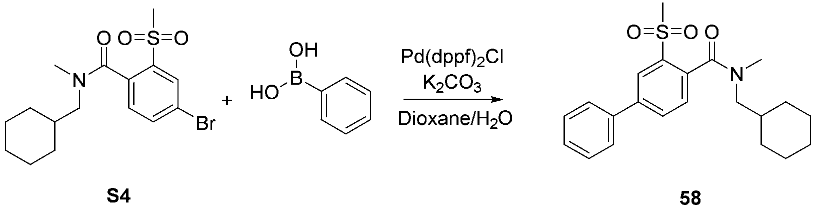

Under the protection of N₂, the compound **S4** (20 mg, 0.052 mmol), phenylboronic acid (7.54 mg, 0.062 mmol), Pd(dppf)Cl₂ (3.76 mg, 0.0052 mmol), K₂CO₃ (21.36 mg, 0.155mol), and 1 mL dioxane/H2O (v:v=3:1) were added into a microwave tube, and stirred at 100 °C for 2 h, where the reaction was monitored by LC-MS. The reaction mixture was subjected to rotary evaporation, and extraction with ethyl acetate three times. The resultant organic phases were combined, washed with a saturated NaCl aqueous solution, dried over anhydrous sodium sulfate, and purified through medium-pressure liquid chromatography to obtain a brown solid as compound **58** (7 mg, 0.018 mmol, 35.25% yield, 100% purity).

**LCMS** (ESI⁺) m/z: 386.1 [M+H]⁺.

**¹H NMR** (400 MHz, DMSO-d6) δ 8.16 (dd, J = 2.7, 1.8 Hz, 1H), 8.08 (ddd, J = 9.9, 7.9, 1.9 Hz, 1H), 7.82 - 7.72 (m, 2H), 7.54 (dd, J = 8.2, 6.7 Hz, 3H), 7.50 - 7.43 (m, 1H), 3.52 (d, J = 9.1 Hz, 1H), 3.32 (s, 2H), 3.29 (s, 1H), 3.08 (d, J = 9.7 Hz, 1H), 2.97 (s, 1H), 2.78 (s, 2H), 1.87 - 1.66 (m, 4H), 1.68 - 1.51 (m, 2H), 1.31 - 1.11 (m, 3H), 0.99 (s, 2H).

Performances of compounds of the present disclosure were shown in the following test examples.

### Test example 1 Detection of SPR Binding Assays (Surface Plasmon Resonance Binding Assay) of TRIM21 Compounds

### (1) Experimental materials and reagents

| | |
|---|---|
| Biacore 8K | Cytiva |
| S series CM5 sensor chip | Cytiva Cat#BR-1005-30 |
| Twin Strep Tag Capture Kit | IBA 2-4370-000 |
| NaOH 50 | Cytiva Cat#BR-1003-58 |
| DMSO | VETEC V900090 |
| Na₂HPO4 | Sigma 795410 |
| NaH₂PO4 | Sigma RDD007 |
| NaCl | Sangon Biotech A501218-0005 |
| Tween-20 | Sigma 93773 |
| Amine Coupling Kit | Cytiva Cat#BR100050 |
| 96-Well Plate | Greener bio-one Cat#650201 |

### (2) SPR Binding Assays

### Preparation of Running Buffer

A protein fixation buffer included 7.6 mM NaH₂OP₄, 12.4 mM Na₂HOP₄, 150.0 mM NaCl, and 0.1% of Tween 20, and adjusted to pH 7.0. A running buffer A included 7.6 mM NaH₂OP₄, 12.4 mM Na₂HOP₄, 150.0 mM NaCl, and 0.1% Tween 20, and adjusted to pH 7.0. A running buffer B included 7.6 mM NaH₂OP₄, 12.4 mM Na₂HOP₄, 150.0 mM NaCl, 0.1% of Tween 20 and 1.00% of DMSO, and adjusted to pH 7.0. The protein fixation buffer, the running buffer A and the running buffer B were prepared and filtered through a 0.22 µm membrane prior to use.

### Strep-Tactin XT Fixation

A surface of a CM5 chip was washed for three times with 50 mM NaOH at a flow rate of 60.0 µL/min and 60 seconds per wash. EDC (1-ethyl-3-(3-dimethylaminopropyl)carbodiimide) (75.00 mg/mL) and NHS (N-hydroxysuccinimide) (11.50 mg/mL) in the amine coupling kit were prepared as an activation reagent with a volume ratio of 1:1. The CM5 chip was activated for 420 s at a flow rate of 6.0 µL/min. A strep tactin XT fixation buffer fixation built in the twin strep tag capture kit was used to prepare a 50.0 µg/mL Strep tactin XT. The Strep tactin XT was injected at a flow rate of 5.0 µL/min for 600 s. After injection of His antibody (anti-polyhistidine antibody), 1 M ethanolamine from the amine coupling kit was injected at a flow rate of 6.0 µL/min for 7 min to close the surface of the CM5 chip. A final fixation amount of the Strep tactin XT was about 12000.0 RU.

### TRIM21 Protein Fixation

The running buffer A was used as the protein fixation buffer. The protein fixation buffer and the TRIM21 protein were mixed to prepare a 40.0 µg/mL mixed solution. The 40.0 µg/mL mixed solution was injected at flow rate of 5.0 µL/min for 600 s, and the TRIM21 protein was captured on the CM5 chip through Strep tactin XT. A final fixation amount of the
TRIM21 protein was about 2200.00 RU.

### Compound dilution

A compound to be tested was diluted with 100% DMSO to a concentration that was 100 times a required final concentration and mixed evenly, and 4.0 µL of such solution was added into 396 µL of the running buffer A, followed by centrifugation at 15,000 rpm for 5 min to obtain an X compound solution contained with 1% DMSO. The X compound was subjected to 8-point, 2-fold serial dilutions with the running buffer B, starting from an initial concentration. The diluted X compound was transferred into the 96-well plate for sample injection.

### Program Running

A Biacore 8K Control software was started, and tests were run in a 25 °C environment. During program running, the running buffer B was used at a flow rate of 30.0 µL/min. The running buffer B was injected for three times to complete balance. The X compound was injected from a lower concentration to a higher concentration in sequence, where a binding time and dissociation time were both 60 s. An injection needle was cleaned with 50% DMSO after each injection. A solvent difference caused by DMSO was corrected by 0.50%, 0.75%, 1.00%, 1.25% and 1.50% DMSO.

### (3) Data Analysis

A response value of binding of the X compound and the TRIM21 protein was corrected after deducting a reference channel and 0 concentration. A binding affinity Kd was matched by a steady state affinity model (1:1 binding model) through the Biacore Insight Evaluation Software.

The binding affinities between X compounds of the embodiments of the present disclosure were tested according the above method. Test results were shown in Table 1, where Kd values of the X compounds were classified according to instructions as follows:
"-" represented the Kd value > 10 µM;
"+" represented 1 µM < the Kd value ≤ 10 µM;
"++" represented 100 nM < the Kd value ≤ 1 µM;
"+++" represented 10 nM < the Kd value ≤100 nM; and
"++++" represented 1 nM < the Kd value ≤ 10 nM.

**Table 1 Binding Affinity Kd between the X Compounds and the TRIM21 Protein**

| Compound | TRIM21 Kd Test Value |
|---|---|
| **1** | ++ |
| **2** | ++ |
| **3** | +++ |
| **4** | ++ |
| **5** | + |
| **6** | ++ |
| **7** | ++ |
| **8** | ++ |
| **9** | + |
| **10** | ++ |
| **11** | +++ |
| **12** | + |
| **13** | ++ |
| **14** | ++ |
| **15** | ++ |
| **16** | ++ |
| **17** | +++ |
| **18** | + |
| **19** | ++ |
| **20** | ++ |
| **21** | - |
| **22** | - |
| **23** | +++ |
| **24** | + |
| **25** | + |
| **26** | - |
| **27** | +++ |
| **28** | +++ |
| **29** | +++ |
| **30** | + |
| **31** | + |
| **32** | + |
| **33** | + |
| **34** | ++ |
| **35** | ++ |
| **36** | - |
| **37** | ++ |
| **38** | ++ |
| **39** | ++ |
| **40** | ++ |
| **41** | +++ |
| **42** | ++ |
| **43** | + |
| **44** | - |
| **45** | + |
| **46** | ++ |
| **47** | - |
| **48** | - |
| **49** | - |
| **50** | - |
| **51** | - |
| **52** | ++++ |
| **53** | ++ |
| **54** | + |
| **55** | ++++ |
| **56** | ++++ |
| **57** | ++++ |
| **58** | - |

The experimental data indicated that the compounds of the present disclosure had a moderate to good binding affinities with the TRIM21 protein, and was considered promising in the preparation of a drug for treating TRIM21-associated diseases, such as tumors or autoimmune diseases. In addition, the compounds of the present disclosure may be also used as ligand or intermediate compounds for the development of TRIM21-based PROTACs.

## Claims

1. A compound of formula (I), or a stereoisomer, a deuterated compound or a pharmaceutically acceptable salt thereof: 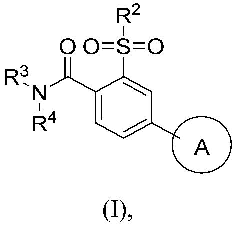 **characterized in that**:
A ring is selected from the group consisting of 6 to 10-membered aromatic ring, 5 to 10-membered heteroaromatic ring, 3 to 10-membered cycloalkyl and 3 to 10-membered heterocyclic ring, wherein aromatic ring, heteroaromatic ring, cycloalkyl and heterocyclic ring are independently unsubstituted or substituted with one, two, three or four R¹;
each R¹ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, hydroxy-substituted - C₁₋₆ alkyl, -C₀₋₄ alkylene-OR¹², -C₀₋₄ alkylene-OC(O)R¹², -C₀₋₄ alkylene-SR¹², -C₀₋₄ alkylene-S(O)₂R¹², -C₀₋₄ alkylene-S(O)R¹², -C₀₋₄ alkylene-S(O)₂NR¹²R¹³, -C₀₋₄ alkyleneS(O)NR¹²R¹³, -C₀₋₄ alkylene-C(O)R¹², -C₀₋₄ alkylene-C(O)OR¹², -C₀₋₄ alkylene-C(O)NR¹²R¹³, -C₀₋₄ alkylene-NR¹²R¹³, -C₀₋₄ alkylene-NR¹²C(O)R¹³, -C₀₋₄ alkylene-NR¹²S(O)₂R¹³, -C₀₋₄ alkylene-NR¹²S(O)R¹³, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring);
R¹² and R¹³ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R¹⁴;
each R¹⁴ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl;
each R² is independently selected from the group consisting of hydrogen, halogen, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR²², -C₀₋₄ alkylene-OC(O)R²², -C₀₋₄ alkylene-SR²², -C₀₋₄ alkylene-S(O)₂R²², -C₀₋₄ alkylene-S(O)R²², -C₀₋₄ alkyleneS(O)₂NR²²R²³, -C₀₋₄ alkylene-S(O)NR²²R²³, -C₀₋₄ alkylene-C(O)R²², -C₀₋₄ alkylene-C(O)OR²², -C₀₋₄ alkylene-C(O)NR²²R²³, -C₀₋₄ alkylene-NR²²R²³, -C₀₋₄ alkylene-NR²²C(O)R²³, -C₀₋₄ alkylene-NR²²S(O)₂R²³, -C₀₋₄ alkylene-NR²²S(O)R²³, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring);
R²² and R²³ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl,-C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R²⁴;
each R²⁴ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl;
R³ and R⁴ are each independently selected from the group consisting of hydrogen, halogen, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR³⁴, -C₀₋₄ alkylene-OC(O)R³⁴, -C₀₋₄ alkylene-SR³⁴, -C₀₋₄ alkylene-S(O)₂R³⁴, -C₀₋₄ alkylene-S(O)R³⁴, - C₀₋₄ alkylene-S(O)₂NR³⁴R³⁵, -C₀₋₄ alkylene-S(O)NR³⁴R³⁵, -C₀₋₄ alkylene-C(O)R³⁴, -C₀₋₄ alkylene-C(O)OR³⁴, -C₀₋₄ alkylene-C(O)NR³⁴R³⁵, -C₀₋₄ alkylene-NR³⁴R³⁵, -C₀₋₄ alkylene-NR³⁴C(O)R³⁵, -C₀₋₄ alkylene-NR³⁴S(O)₂R³⁵, -C₀₋₄ alkylene-NR³⁴S(O)R³⁵, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R³⁶;
each R³⁶ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR³⁴, -C₀₋₄ alkylene-OC(O)R³⁴, -C₀₋₄ alkylene-SR³⁴, -C₀₋₄ alkylene-S(O)₂R³⁴, -C₀₋₄ alkyleneS(O)R³⁴, -C₀₋₄ alkylene-S(O)₂NR³⁴R³⁵, -C₀₋₄ alkylene-S(O)NR³⁴R³⁵, -C₀₋₄ alkylene-C(O)R³⁴, -C₀₋₄ alkylene-C(O)OR³⁴, -C₀₋₄ alkylene-C(O)NR³⁴R³⁵, -C₀₋₄ alkylene-NR³⁴R³⁵, -C₀₋₄ alkylene-NR³⁴C(O)R³⁵, -C₀₋₄ alkylene-NR³⁴S(O)₂R³⁵, -C₀₋₄ alkylene-NR³⁴S(O)R³⁵, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R³⁷;
R³⁴ and R³⁵ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl and halogen-substituted -C₂₋₆ alkynyl; and
each R³⁷ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl.

2. The compound according to claim 1, **characterized in that** the compound is represented by formula (IIA): 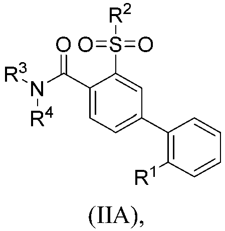 wherein R¹, R², R³ and R⁴ are defined as in claim 1.

3. The compound according to claim 1, **characterized in that** the A ring is selected from the group consisting of 6-membered aromatic ring, 6-membered cycloalkyl, 9-membered heterocycloalkyl, 6-membered heteroaromatic ring and 9-membered heteroaromatic ring, wherein aromatic ring, cycloalkyl, heterocycloalkyl and heteroaromatic ring are independently unsubstituted or substituted with one, two, three or four R¹;
each R¹ is independently selected from the group consisting of hydrogen, halogen, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, hydroxy-substituted -C₁₋₆ alkyl, -C₀₋₄ alkylene-OR¹², -C₀₋₄ alkylene-OC(O)R¹², -C₀₋₄ alkylene-SR¹², -C₀₋₄ alkylene-NR¹²R¹³, -C₀₋₄ alkylene-(3 to 6-membered cycloalkyl), -C₀₋₄ alkylene-(3 to 6-membered heterocycloalkyl), -C₀₋₄ alkylene-(6 to 10-membered aromatic ring) and -C₀₋₄ alkylene-(5 to 10-membered heteroaromatic ring); and
R¹² and R¹³ are each independently selected from the group consisting of hydrogen, - C₁₋₆ alkyl, -C₂₋₆ alkenyl, -C₂₋₆ alkynyl and halogen-substituted -C₁₋₆ alkyl.

4. The compound according to claim 3, **characterized in that** each R¹ is independently selected from the group consisting of hydrogen, methylthio, halogen, methyl, ethyl, propyl, isopropyl, methoxy, trifluoromethyl, 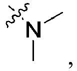 cyclopropyl and

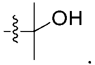

5. The compound according to claim 3, **characterized in that** the A ring is selected from the group consisting of

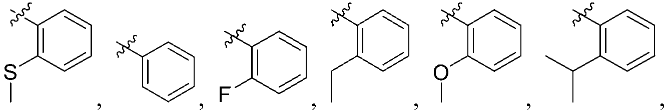

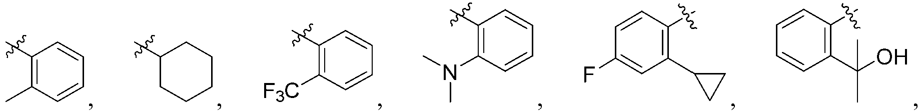

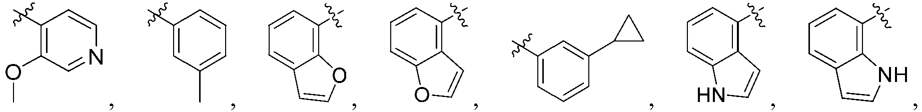

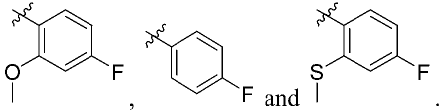

6. The compound according to claim 1 or 2, **characterized in that** R² is -C₁₋₃ alkyl.

7. The compound according to claim 1 or 2, **characterized in that** R³ and R⁴ are each independently selected from the group consisting of hydrogen, -C₁₋₆ alkyl, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R³⁶;
each R³⁶ is independently selected from the group consisting of hydrogen, halogen, cyano group, =O, -C₁₋₆ alkyl, halogen-substituted -C₁₋₆ alkyl, -C₀₋₄ alkylene-OR³⁴, -C₀₋₄ alkylene-C(O)R³⁴, -C₀₋₄ alkylene-C(O)OR³⁴, -C₀₋₄ alkylene-C(O)NR³⁴R³⁵, -C₀₋₄ alkylene-NR³⁴R³⁵, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring); and
R³⁴ and R³⁵ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl and halogen-substituted -C₂₋₆ alkynyl.

8. The compound according to claim 7, **characterized in that** R³ and R⁴ are each independently selected from the group consisting of hydrogen, methyl, ethyl, propyl, butyl, amyl, hexyl, -C₀₋₂ alkylene-(3-membered cycloalkyl), -C₀₋₂ alkylene-(4-membered cycloalkyl), -C₀₋₂ alkylene-(5-membered cycloalkyl), -C₀₋₂ alkylene-(6-membered cycloalkyl), -C₀₋₂ alkylene-(4-membered heterocycloalkyl), -C₀₋₂ alkylene-(5-membered heterocycloalkyl), -C₀₋₂ alkylene-(6-membered heterocycloalkyl), -C₀₋₂ alkylene-(9-membered heterocycloalkyl), -C₀₋₂ alkylene-(6-membered aromatic ring), -C₀₋₂ alkylene-(5-membered heteroaromatic ring), -C₀₋₂ alkylene-(6-membered heteroaromatic ring) and -C₀₋₂ alkylene-(9-membered heteroaromatic ring), wherein alkylene, cycloalkyl, heterocycloalkyl, aromatic ring and heteroaromatic ring are independently unsubstituted or substituted with one, two, three or four R³⁶;
each R³⁶ is independently selected from the group consisting of hydrogen, halogen, =O, -C₁₋₃ alkyl, halogen-substituted -C₁₋₃ alkyl, -OR³⁴, -C(O)R³⁴ and -NR³⁴R³⁵; and
R³⁴ and R³⁵ are each independently selected from the group consisting of hydrogen and -C₁₋₃ alkyl.

9. The compound according to claim 8, **characterized in that** R³ and R⁴ are each independently selected from the group consisting of hydrogen, methyl, ethyl, propyl, butyl, amyl, hexyl,

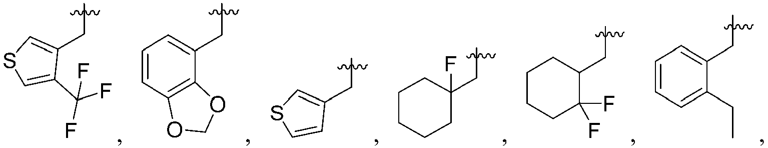

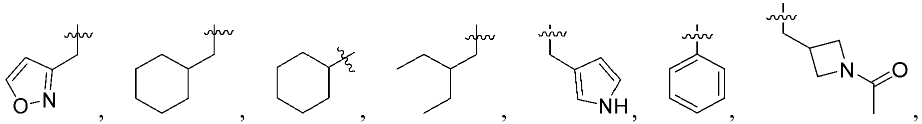

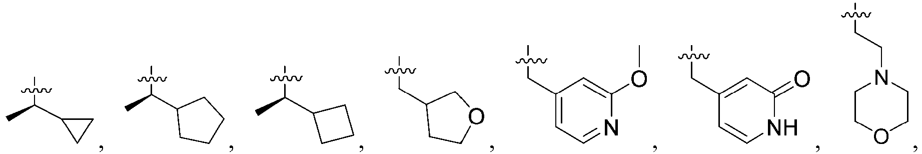

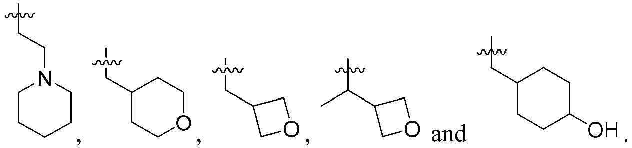

10. The compound according to any one of claims 1-9, **characterized in that** the compound is selected from the group consisting of:

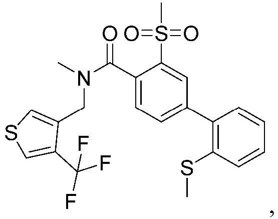

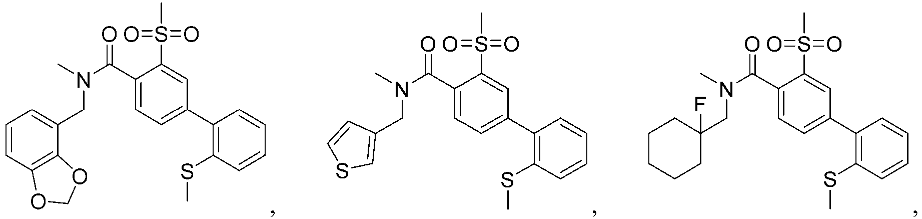

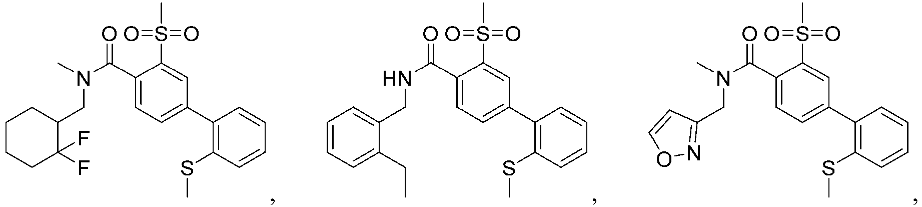

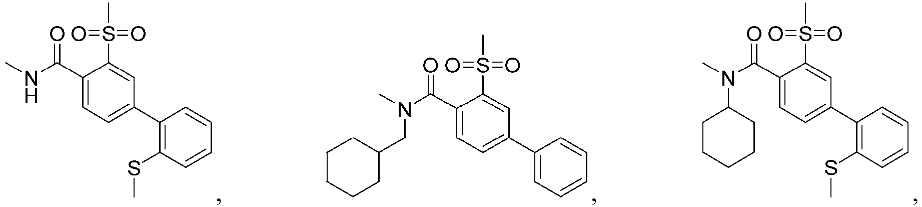

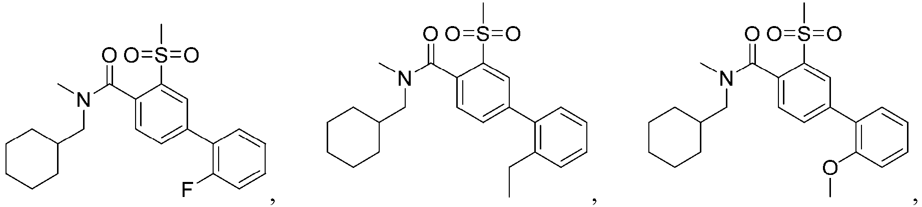

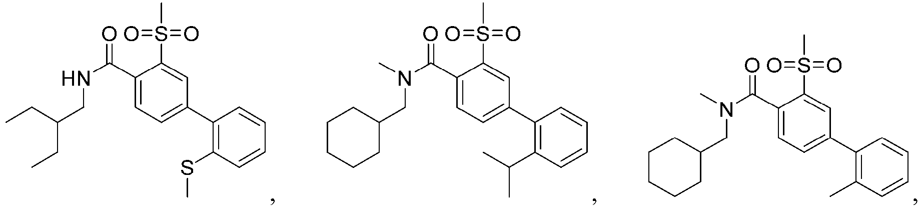

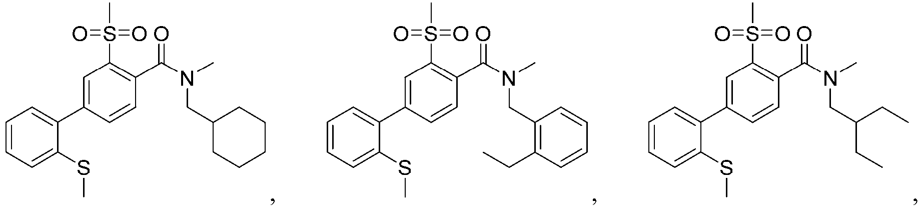

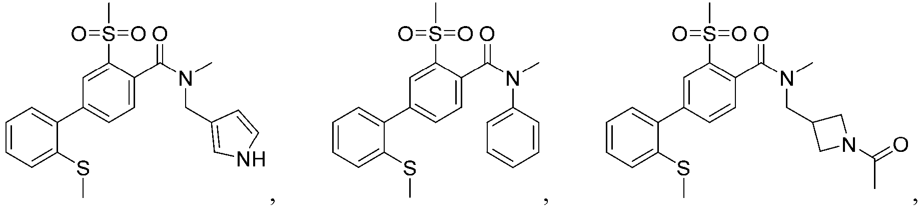

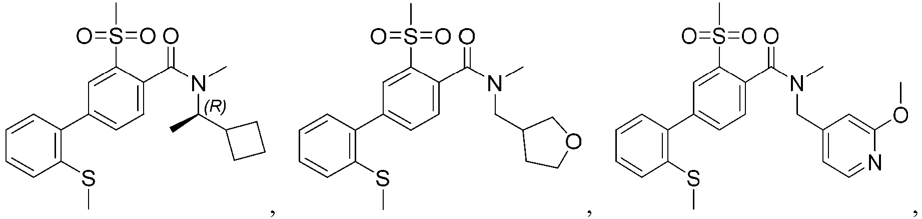

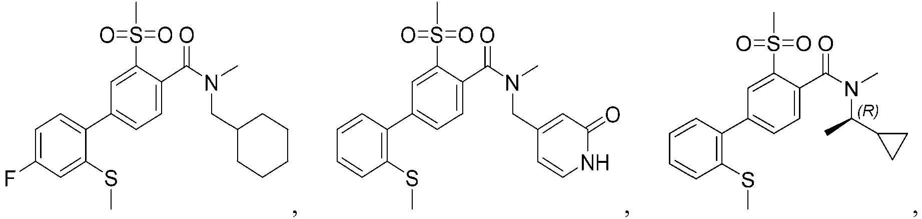

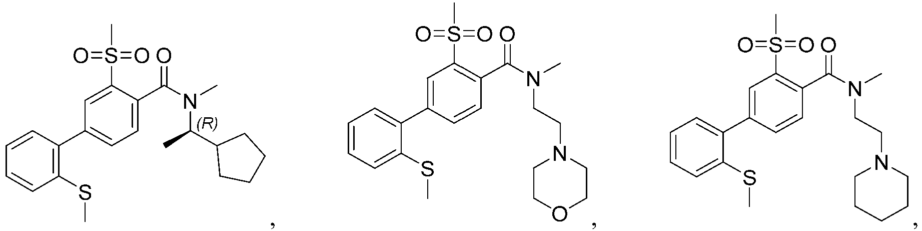

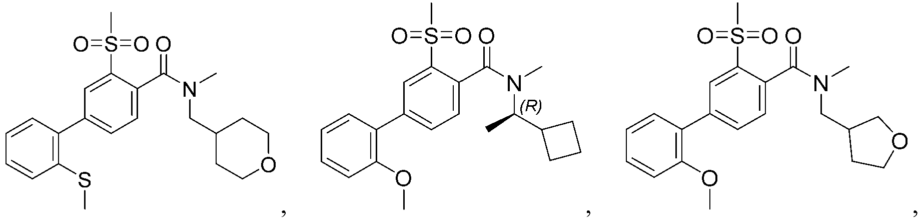

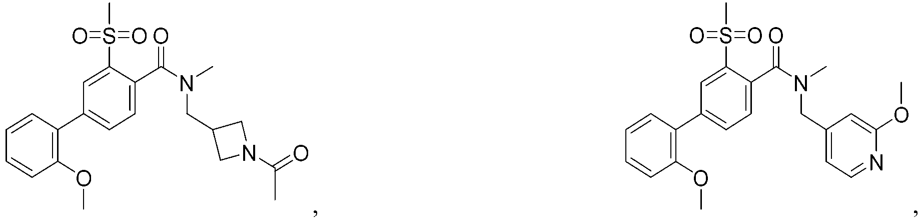

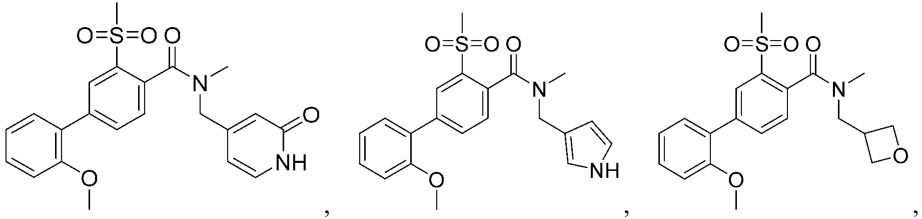

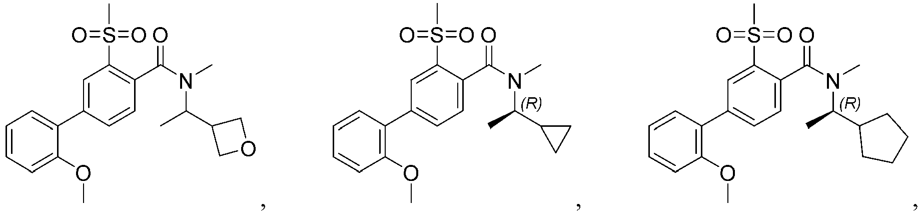

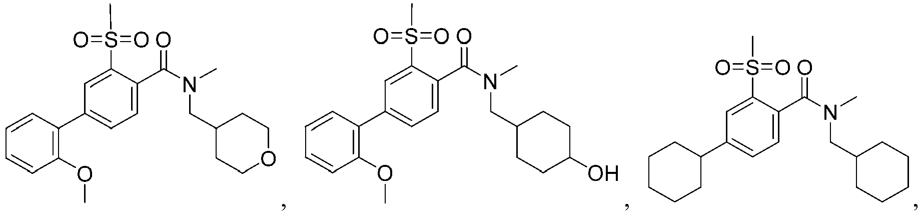

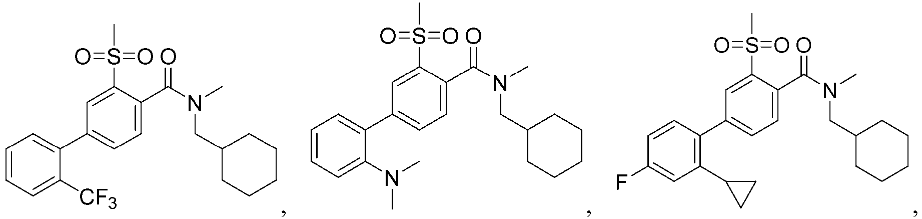

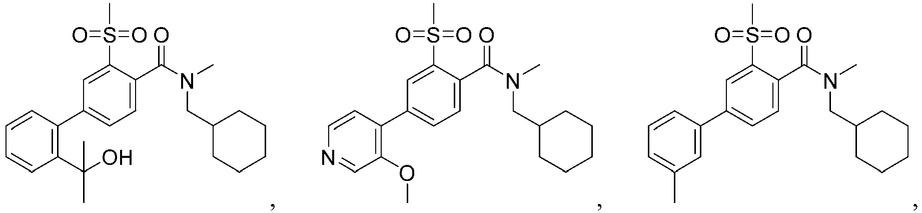

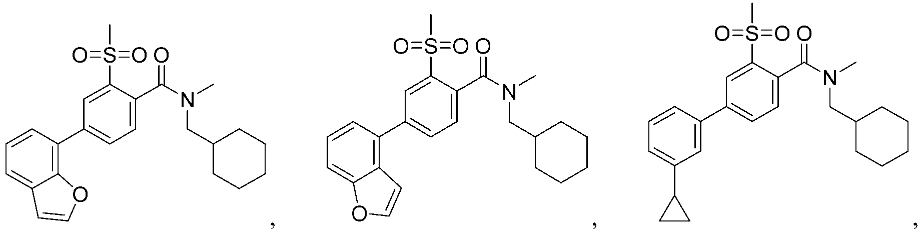

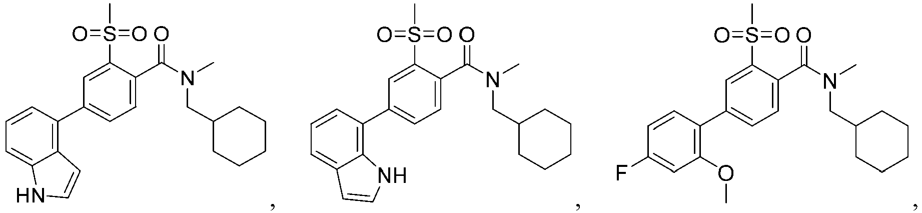

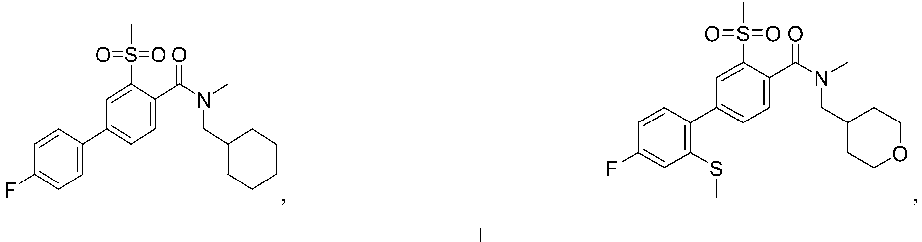

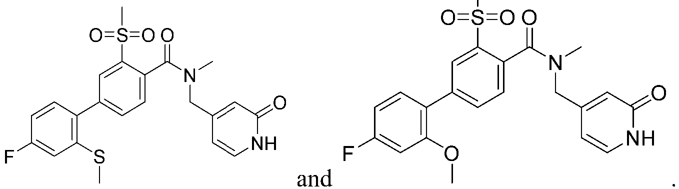

11. A compound of formula (V), or a stereoisomer, a deuterated compound or a pharmaceutically acceptable salt thereof: 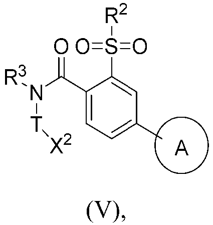 **characterized in that**:
A ring is selected from the group consisting of 6 to 10-membered aromatic ring, 5 to 10-membered heteroaromatic ring, 3 to 10-membered cycloalkyl and 3 to 10-membered heterocyclic ring, wherein aromatic ring, heteroaromatic ring, cycloalkyl and heterocyclic ring are independently unsubstituted or substituted with one, two, three or four R¹;
each R¹ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR¹², - C₀₋₄ alkylene-OC(O)R¹², -C₀₋₄ alkylene-SR¹², -C₀₋₄ alkylene-S(O)₂R¹², -C₀₋₄ alkyleneS(O)R¹², -C₀₋₄ alkylene-S(O)₂NR¹²R¹³, -C₀₋₄ alkylene-S(O)NR¹²R¹³, -C₀₋₄ alkylene-C(O)R¹², -C₀₋₄ alkylene-C(O)OR¹², -C₀₋₄ alkylene-C(O)NR¹²R¹³, -C₀₋₄ alkylene-NR¹²R¹³, -C₀₋₄ alkylene-NR¹²C(O)R¹³, -C₀₋₄ alkylene-NR¹²S(O)₂R¹³, -C₀₋₄ alkylene-NR¹²S(O)R¹³, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring);
R¹² and R¹³ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R¹⁴;
each R¹⁴ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl;
each R² is independently selected from the group consisting of hydrogen, halogen, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR²², -C₀₋₄ alkylene-OC(O)R²², -C₀₋₄ alkylene-SR²², -C₀₋₄ alkylene-S(O)₂R²², -C₀₋₄ alkylene-S(O)R²², -C₀₋₄ alkyleneS(O)₂NR²²R²³, -C₀₋₄ alkylene-S(O)NR²²R²³, -C₀₋₄ alkylene-C(O)R²², -C₀₋₄ alkylene-C(O)OR²², -C₀₋₄ alkylene-C(O)NR²²R²³, -C₀₋₄ alkylene-NR²²R²³, -C₀₋₄ alkylene-NR²²C(O)R²³, -C₀₋₄ alkylene-NR²²S(O)₂R²³, -C₀₋₄ alkylene-NR²²S(O)R²³, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring);
R²² and R²³ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl, halogen-substituted -C₂₋₆ alkynyl,-C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R²⁴;
each R²⁴ is independently selected from the group consisting of hydrogen, halogen, cyano group, nitro, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl;
R³ is independently selected from the group consisting of hydrogen, halogen, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR³⁴, -C₀₋₄ alkylene-OC(O)R³⁴, -C₀₋₄ alkylene-SR³⁴, -C₀₋₄ alkylene-S(O)₂R³⁴, -C₀₋₄ alkylene-S(O)R³⁴, -C₀₋₄ alkyleneS(O)₂NR³⁴R³⁵, -C₀₋₄ alkylene-S(O)NR³⁴R³⁵, -C₀₋₄ alkylene-C(O)R³⁴, -C₀₋₄ alkylene-C(O)OR³⁴, -C₀₋₄ alkylene-C(O)NR³⁴R³⁵, -C₀₋₄ alkylene-NR³⁴R³⁵, -C₀₋₄ alkylene-NR³⁴C(O)R³⁵, -C₀₋₄ alkylene-NR³⁴S(O)₂R³⁵, -C₀₋₄ alkylene-NR³⁴S(O)R³⁵, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R³⁶;
each R³⁶ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR³⁴, -C₀₋₄ alkylene-OC(O)R³⁴, -C₀₋₄ alkylene-SR³⁴, -C₀₋₄ alkylene-S(O)₂R³⁴, -C₀₋₄ alkyleneS(O)R³⁴, -C₀₋₄ alkylene-S(O)₂NR³⁴R³⁵, -C₀₋₄ alkylene-S(O)NR³⁴R³⁵, -C₀₋₄ alkylene-C(O)R³⁴, -C₀₋₄ alkylene-C(O)OR³⁴, -C₀₋₄ alkylene-C(O)NR³⁴R³⁵, -C₀₋₄ alkylene-NR³⁴R³⁵, -C₀₋₄ alkylene-NR³⁴C(O)R³⁵, -C₀₋₄ alkylene-NR³⁴S(O)₂R³⁵, -C₀₋₄ alkylene-NR³⁴S(O)R³⁵, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R³⁷;
R³⁴ and R³⁵ are each independently selected from the group consisting of hydrogen, - C₁₋₆ alkyl, -C₂₋₆ alkenyl, -C₂₋₆ alkynyl, halogen-substituted -C₁₋₆ alkyl, halogen-substituted - C₂₋₆ alkenyl and halogen-substituted -C₂₋₆ alkynyl;
each R³⁷ is independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl;
T is -(L^{T})_{q}-;
q is an integer selected from 1-50;
each L^{T} is independently selected from the group consisting of CR^{T2}R^{T3}, C(O), -C(S)-, O, S, S(O), S(O)₂, NR^{T2}, -CR^{T2}=CR^{T3}-, -C=C-, 3 to 12-membered cycloalkyl, 3 to 12-membered heterocycloalkyl, 6 to 10-membered aromatic ring, 5 to 10-membered heteroaromatic ring, 5 to 12-membered spiro cycle, 5 to 12-membered spiro heterocycle, 5 to 12-membered bridged ring, 5 to 12-membered bridged heterocycle and a combination thereof, wherein cycloalkyl, heterocycloalkyl, aromatic ring, heteroaromatic ring, spiro cycle, spiro heterocycle, bridged ring and bridged heterocycle are independently unsubstituted or substituted with one, two, three or four R^{T1};
each R^{T1} is independently selected from the group consisting of hydrogen, halogen, cyano group, nitro, =O, =S, =CR^{T2}R^{T3}, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-OR^{T2}, -C₀₋₄ alkylene-OC(O)R^{T2}, -C₀₋₄ alkylene-SR^{T2}, -C₀₋₄ alkylene-S(O)₂R^{T2}, -C₀₋₄ alkylene-S(O)R^{T2}, -C₀₋₄ alkylene-S(O)₂NR^{T2}R^{T3}, -C₀₋₄ alkylene-S(O)NR^{T2}R^{T3}, -C₀₋₄ alkylene-C(O)R^{T2}, -C₀₋₄ alkylene-C(O)OR^{T2}, -C₀₋₄ alkylene-C(O)NR^{T2}R^{T3}, -C₀₋₄ alkylene-NR^{T2}R^{T3}, -C₀₋₄ alkylene-NR^{T2}C(O)R^{T3}, -C₀₋₄ alkylene-NR^{T2}S(O)₂R^{T3}, -C₀₋₄ alkylene-NR^{T2}S(O)R^{T3}, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring), wherein alkylene, cycloalkyl, heterocycloalkyl, aromatic ring and heteroaromatic ring are independently unsubstituted or substituted with one, two, three or four R^{T4};
R^{T2}, R^{T3} and R^{T4} are each independently selected from the group consisting of hydrogen, halogen, cyano group, nitro, =O, =S, -C₁₋₆ alkyl, -C₂₋₆ alkenyl, -C₂₋₆ alkynyl, halogen-substituted -C₁₋₆ alkyl, halogen-substituted -C₂₋₆ alkenyl, halogen-substituted -C₂₋₆ alkynyl, -C₀₋₄ alkylene-(3 to 10-membered cycloalkyl), -C₀₋₄ alkylene-(3 to 10-membered heterocycloalkyl), -C₀₋₄ alkylene-(6 to 10-membered aromatic ring) and -C₀₋₄ alkylene-(5 to 10-membered heteroaromatic ring);
X² is selected from the group consisting of -NH₂, -NHR^{X21}, -OH, -SH, ethynyl, ethenyl, -C(O)H and -C(O)OH-; and
R^{X21} is selected from the group consisting of hydrogen, -C₁₋₆ alkyl, -C₂₋₆ alkenyl, -C₂₋₆ alkynyl, halogen-substituted -C₁₋₆ alkyl, halogen-substituted -C₂₋₆ alkenyl and halogen-substituted -C₂₋₆ alkynyl.

12. The compound according to claim 11, **characterized in that** the compound is represented by formula (VIa): 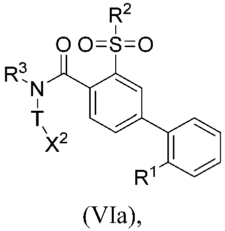 wherein R¹, R², R³, T and X² are defined as in claim 11.

13. The compound according to claim 12, **characterized in that** T is selected from the group consisting of 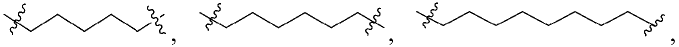 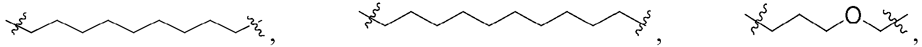 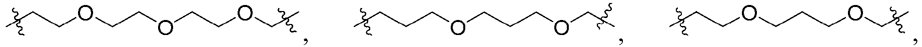 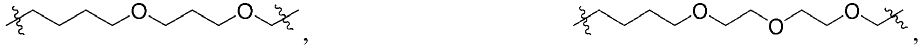 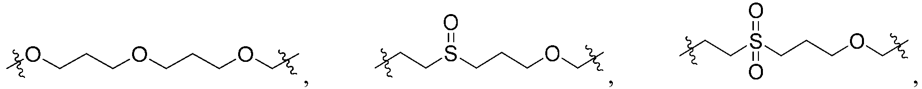 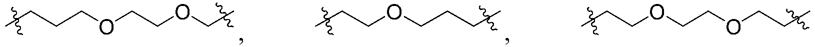 and

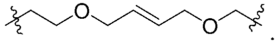

14. The compound according to any one of claims 1-13 or a stereoisomer, a deuterated compound or a pharmaceutically acceptable salt thereof for use in the treatment of a disease associated with abnormal cell proliferation.

15. The compound for use according to claim 14, wherein the disease is a cancer.

16. The compound according to any one of claims 1-13 or a stereoisomer, a deuterated compound or a pharmaceutically acceptable salt thereof for use in the preparation of a drug for targeted protein degradation.

17. The compound for use according to claim 16, **characterized in that** the compound or a stereoisomer, a deuterated compound or a pharmaceutically acceptable salt thereof acts as an intermediate in the preparation of the drug.

18. The compound for use according to claim 16, **characterized in that** the drug is dependent on a tripartite motif protein 21 E3 ubiquitin ligase (TRIM21) for protein degradation.
